(19) **Europäisches Patentamt / European Patent Office / Office européen des brevets**

(11) **EP 3 998 345 A1**

(12) **EUROPEAN PATENT APPLICATION**

(43) Date of publication:
**18.05.2022 Bulletin 2022/20**

(21) Application number: **21200635.7**

(22) Date of filing: **22.12.2016**

(51) International Patent Classification (IPC):
**C12Q 1/68** *(2018.01)* **C12Q 1/6883** *(2018.01)*
**G01N 33/68** *(2006.01)*

(52) Cooperative Patent Classification (CPC):
**C12Q 1/6883; G01N 33/6893;** C12Q 2600/158;
G01N 2800/26

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2015 AU 2015905392**

(62) Document number(s) of the earlier application(s) in accordance with Art. 76 EPC:
**16877007.1 / 3 394 291**

(71) Applicant: **ImmuneXpress Pty Ltd**
**Boonah, Queensland 4310 (AU)**

(72) Inventors:
• **BRANDON, Richard Bruce**
**Boonah, Queensland 4310 (AU)**
• **FOX, Brian Andrew**
**Seattle, WA 98103 (AU)**

• **MCHUGH, Leo Charles**
**Seattle, WA 98102 (AU)**
• **SAMSON, Dayle Lorand**
**Shoreline, WA 98133 (AU)**

(74) Representative: **Vossius & Partner**
**Patentanwälte Rechtsanwälte mbB**
**Siebertstraße 3**
**81675 München (DE)**

Remarks:
•The complete document including Reference Tables and the Sequence Listing can be downloaded from the EPO website
•This application was filed on 04-10-2021 as a divisional application to the application mentioned under INID code 62.
•Claims filed after the date of filing of the application / after the date of receipt of the divisional application (Rule 68(4) EPC).

(54) **TRIAGE BIOMARKERS AND USES THEREFOR**

(57) Disclosed are methods, apparatus, kits and compositions for determining the absence of a systemic bacterial infection (sepsis) in patients, particularly ones presenting to hospital emergency departments (ED) as outpatients, by measurement of the host immune response using peripheral blood. The methods, apparatus, kits and compositions can be used in mammals for diagnosing, making treatment decisions, determining the next procedure or diagnostic test, or management of patients suspected of having an infection, including those presenting with fever or other signs of systemic inflammation. More particularly, peripheral blood RNA and protein biomarkers are disclosed that are useful for distinguishing between the host immune response to bacteria compared to the host immune response to other causes of systemic inflammation including trauma, burns, autoimmune disease, asthma, anaphylaxis, arthritis, obesity and viral infections. As such, the biomarkers are useful for distinguishing bacterial-associated systemic inflammatory response syndrome from nonbacterial systemic inflammation to provide clinicians with strong negative predictive value (>95%) so that sepsis can be excluded as a diagnosis in patients presenting to ED with clinical signs of systemic inflammation.

Greedy addition results for all Clinical datasets
Sensitivity / Specificity
Top-3 ratios (AUC = 0.940)
Top-2 ratios (AUC = 0.920)
Top ratio (AUC = 0.863)

FIGURE 1a

FIGURE 1b

**Description**

**FIELD OF THE INVENTION**

This application claims priority to Australian Provisional Application No. 2015905392 entitled "Triage biomarkers and uses therefor" filed 24 December 2015, the contents of which are incorporated herein by reference in their entirety.

This invention relates generally to methods, apparatus, kits and compositions for determining the absence of a systemic bacterial infection (sepsis) in patients, particularly ones presenting to hospital emergency departments (ED) as outpatients, by measurement of the host immune response using peripheral blood. The invention can be used in mammals for diagnosing, making treatment decisions, determining the next procedure or diagnostic test, or management of patients suspected of having an infection, including those presenting with fever or other signs of systemic inflammation. More particularly, the present invention relates to peripheral blood RNA and protein biomarkers that are useful for distinguishing between the host immune response to bacteria compared to the host immune response to other causes of systemic inflammation including trauma, burns, autoimmune disease, asthma, anaphylaxis, arthritis, obesity and viral infections. As such, the biomarkers are useful for distinguishing bacterial-associated systemic inflammatory response syndrome from non-bacterial systemic inflammation to provide clinicians with strong negative predictive value (>95%) so that sepsis can be excluded as a diagnosis in patients presenting to ED with clinical signs of systemic inflammation.

**BACKGROUND OF THE INVENTION**

In 2010, there were over 129 million visits in the USA to emergency departments (ED). The most common principal reasons for ED visits in the USA in 2010 (all ages and in order) included; stomach and abdominal pain and spasms, chest pain, fever, headache, back symptoms, shortness of breath, cough, pain (non-specific), vomiting, and throat symptoms. The two most common principal reasons for ED visits in the USA for children under the age of 15 are fever and cough. However, the two most common primary diagnoses (as determined by a physician and by major disease category) are "injury and poisoning" and "ill-defined conditions" (Niska, R., Bhuiya, F., & Xu, J. (2010). National hospital ambulatory medical care survey: 2007 emergency department summary. Natl Health Stat Report, 26(26), 1-31). Thus, patients presenting to ED are very heterogenous and often ill-defined with respect to principal reason for presenting and primary diagnosis respectively. In a setting with limited resources and time such patients need to be triaged efficiently. That is, a clinician needs to decide on one or more of the following actions 1) admit the patient 2) observe the patient for a prescribed time period 3) send the patient home 4) take appropriate samples for diagnostic testing 5) determine the next procedure (e.g. X-ray, scan) 6) administer appropriate treatment(s). Underlying each patient's symptoms and presenting clinical signs are etiologies. It is the ED clinician's job to determine an etiology in each case and decide on an appropriate course of action to ensure the best outcomes for the patient. In many instances determining an etiology and course of action is comparatively easy - for example, an adult with a sprained ankle can be sent home after appropriate treatment and advice, a child with severe burns can be admitted immediately, an adult 70-year old male with chest pain can undergo appropriate blood tests and treatments under observation, and a trauma patient in shock can be admitted to intensive care in preparation for surgery. In other instances determining an etiology and course of action is more challenging - for example, in children or adults presenting with fever of unknown origin, or clinical signs that may indicate the presence of an infection, it can be difficult to decide on the next course of action, especially given that some patients presenting with mild clinical signs can deteriorate rapidly. Clinical signs of infection are well known and described in the literature (Bone, R., Balk, R., Cerra, F., Dellinger, R., Fein, A., Knaus, W., et al. (1992). Definitions for sepsis and organ failure and guidelines for the use of innovative therapies in sepsis. The ACCP/SCCM Consensus Conference Committee. American College of Chest Physicians/Society of Critical Care Medicine. Chest, 101(6), 1644-1655). However, identifying, managing and triaging patients with clinical signs of infection is challenging because of the medical risk of such patients progressing to sepsis, severe sepsis and septic shock (Brown, T., Ghelani-Allen, A., Yeung, D., & Nguyen, H. B. (2014). Comparative effectiveness of physician diagnosis and guideline definitions in identifying sepsis patients in the emergency department. Journal of Critical Care; Glickman, S. W., Cairns, C. B., Otero, R. M., Woods, C. W., Tsalik, E. L., Langley, R. J., et al. (2010). Disease Progression in Hemodynamically Stable Patients Presenting to the Emergency Department With Sepsis. Academic Emergency Medicine, 17(4), 383-390; Dellinger, R. P. et al. Surviving Sepsis Campaign: international guidelines for management of severe sepsis and septic shock: 2008. in Crit. Care Med. 36, 296-327 (2008)). Thus, missing a diagnosis of sepsis in patients presenting to ED carries both medical (patient) and professional (clinician) risk. With respect to correctly diagnosing sepsis, blood culture has unacceptably low negative predictive value (NPV), or unacceptably high false negative levels. Further, diagnosis based on clinical signs alone has unacceptably low positive predictive value (PPV), or unacceptably high false positive levels. In the latter instance the consequence is that many patients are unnecessarily prescribed antibiotics because of 1) the clinical risk of misdiagnosing sepsis, 2) the lack of a gold standard diagnostic test, and 3) the fact that blood culture results take too long to provide results that are clinically actionable (Braykov, N. P., Morgan, D. J., Schweizer, M. L., Uslan, D. Z., Kelesidis, T., Weisenberg, S. A., et al. (2014).

Assessment of empirical antibiotic therapy optimisation in six hospitals: an observational cohort study. The Lancet Infectious Diseases, 14(12), 1220-1227). Further, diagnosis of a viral infection is often done based on presenting clinical signs only. The reasons for this are; most viral infections are not life-threatening, there are few therapeutic interventions available, many viral infections cause the same clinical signs, and most diagnostic assays take too long and are too expensive. The consequence is that many virusinfected patients are unnecessarily prescribed antibiotics because of the clinical risk of misdiagnosing bacterial associated systemic inflammatory response syndrome (BaSIRS) or sepsis.

Therefore, in ED patients, what is needed is an assay that can distinguish patients with sepsis from those without an infection but presenting with clinical signs similar to sepsis. Such an assay needs to have high negative predictive value (that is, exclude sepsis as a diagnosis) so that a clinician can confidently either observe, or send the patient home, and/or not prescribe antibiotics. An assay with high negative predictive value for sepsis therefore provides safety for patients, surety and peace of mind for clinicians, reduced costs of care for hospitals and health care systems, reduced antibiotic use, and potentially reduced development of antibiotic resistance.

Whilst the sensitivity and specificity of an assay are independent of prevalence, negative and positive predictive value are not (Lalkhen, A. G., & McCluskey, A. (2008). Clinical tests: sensitivity and specificity. Continuing Education in Anaesthesia, Critical Care & Pain, 8(6), 221-223). In the case of diagnosing BaSIRS in the ED it is important that an assay has a low false negative rate, or is sensitive, or will not miss any cases that actually do have BaSIRS. As disease prevalence decreases in a population the negative predictive value of a sensitive assay increases. Thus, in a population with low disease prevalence an assay with high sensitivity will have high negative predictive value. The prevalence of severe sepsis in adults and children in ED patients is relatively low (6.4% and 0.34% respectively) (Rezende, E., Silva Junior, J. M., Isola, A. M., Campos, E. V., Amendola, C. P., & Almeida, S. L. (2008). Epidemiology of severe sepsis in the emergency department and difficulties in the initial assistance. Clinics, 63(4), 457-464; Singhal, S., Allen, M. W., McAnnally, J.-R., Smith, K. S., Donnelly, J. P., & Wang, H. E. (2013). National estimates of emergency department visits for pediatric severe sepsis in the United States. PeerJ, 1(Suppl 1), e79-12). The prevalence of "infection" as a primary diagnosis in ED patients is also relatively low at approximately 10% for children and 5% for adults (Niska, R., Bhuiya, F., & Xu, J. (2010). National hospital ambulatory medical care survey: 2007 emergency department summary. Natl Health Stat Report, 26(26), 1-31). Similarly, the prevalence of suspected systemic infection, as determined by the pecent of patients presenting to emergency that had a blood culture taken, is also low and estimated to be 4% (Niska et al., 2010). Thus, the prevalence of BaSIRS in adult patients presenting to ER in the USA is estimated to be between 4 and 10%.

In patients suspected of having a BaSIRS a clinical diagnosis and treatment regimen is provided by the physician(s) at the time the patient presents and often in the absence of any results from diagnostic tests. This is done in the interests of rapid treatment and positive patient outcomes. However, such an approach leads to over-prescribing of antibiotics irrespective of whether the patient has a microbial infection or not. Clinician diagnosis (diagnosis by the clinician without the aid of other diagnostic tests) of BaSIRS is reasonably reliable (0.88) in children but only with respect to differentiating between patients ultimately shown to be blood culture positive and those that were judged to be unlikely to have an infection at the time antibiotics were administered (Fischer, J. E. et al. Quantifying uncertainty: physicians' estimates of infection in critically ill neonates and children. Clin. Infect. Dis. 38, 1383-1390 (2004)). In Fischer et al. (2004), 54% of critically ill children were put on antibiotics during their hospital stay, of which only 14% and 16% had proven systemic bacterial infection or localized infection respectively. In this study, 53% of antibiotic treatment courses for critically ill children were for those that had an unlikely infection and 38% were antibiotic treatment courses for critically ill children as a rule-out treatment episode. Clearly, pediatric physicians err on the side of caution with respect to treating critically ill patients by placing all patients suspected of an infection on antibiotics - 38% of all antibiotics used in critically ill children are used on the basis of ruling out BaSIRS, that is, are used as a precaution. Antibiotics are also widely prescribed and overused in adult patients as reported in Braykov et al., 2014 (Braykov, N. P., Morgan, D. J., Schweizer, M. L., Uslan, D. Z., Kelesidis, T., Weisenberg, S. A., et al. (2014). Assessment of empirical antibiotic therapy optimisation in six hospitals: an observational cohort study. The Lancet Infectious Diseases, 14(12), 1220-1227). In this study, across six US hospitals over four days in 2009 and 2010, 60% of all patients admitted received antibiotics. Of those patients prescribed antibiotics 30% were afebrile and had a normal white blood cell count and were therefore prescribed antibiotics as a precaution. Independent surveys of clinicians, conducted by the current patent authors, their colleagues and associates, have revealed that for a clinician to withhold antibiotics from a patient a diagnostic assay for sepsis would need to have a negative predictive value of at least 95%. As such, an assay that can accurately diagnose patients without BaSIRS with negative predictive value greater than 95% would be clinically useful and may lead to more appropriate use of antibiotics.

Testing for the presence of bacteria requires that clinical samples be taken from patients. Examples of clinical samples include; blood, plasma, serum, cerebrospinal fluid (CSF), stool, urine, tissue, pus, saliva, semen, skin, other body fluids. Examples of clinical sampling methods include; venipuncture, biopsy, scrapings, aspirate, lavage, collection of body fluids and stools into sterile containers. Most clinical sampling methods are invasive (physically or on privacy), or painful, or laborious, or require multiple samplings over time, or, in some instances, dangerous (e.g. large CSF volumes in neonates). In some instances multiple samples from multiple sites may need to be taken to increase the likelihood of isolating bacteria. The taking of blood via venipuncture is perhaps the least invasive method of clinical sampling and host immune response markers circulate in peripheral blood in response to both systemic and localized infection. Therefore, what is needed is a diagnostic assay, based on the use of a peripheral blood sample, with high negative predictive value for BaSIRS in an heterogenous ED patient population.

The purported "gold standard" of diagnosis for microbial infection is culture (growth of an organism and partial or complete identification by staining or biochemical or serological assays). Thus, confirmation of a diagnosis of BaSIRS requires isolation and identification of live microbes from blood or tissue or body fluid samples using culture, but this technique has its limitations (Thierry Calandra and Jonathan Cohen, "The International Sepsis Forum Consensus Conference on Definitions of Infection in the Intensive Care Unit," Critical Care Medicine 33, no. 7 (July 2005): 1538-1548; R Phillip Dellinger et al., "Surviving Sepsis Campaign: International Guidelines for Management of Severe Sepsis and Septic Shock: 2008.," vol. 36, 2008, 296-327, doi: 10.1097/01.CCM.0000298158.12101.41). Microbial culture usually takes a number of days to obtain a positive result and over five days (up to a month) to confirm a negative result - hence blood culture has little to no negative predictive value in an ED setting. A positive result confirms bacteremia if the sample used was whole blood. However, blood culture is insufficiently reliable with respect to sensitivity, specificity and predictive value, failing to detect a clinically determined 'bacterial' cause of fever in 60-80% of patients with suspected primary or secondary bloodstream infection, and in many instances the organism grown is a contaminant (Müller, B., Schuetz, P. & Trampuz, A. Circulating biomarkers as surrogates for bloodstream infections. International Journal of Antimicrobial Agents 30, 16-23 (2007); Jean-Louis Vincent et al., "Sepsis in European Intensive Care Units: Results of the SOAP Study∗," Critical Care Medicine 34, no. 2 (February 2006): 344-353, doi:10.1097/01.CCM.0000194725.48928.3A; Brigitte Lamy et al., "What Is the Relevance of Obtaining Multiple Blood Samples for Culture? A Comprehensive Model to Optimize the Strategy for Diagnosing Bacteremia", Clinical Infectious Diseases: an Official Publication of the Infectious Diseases Society of America 35, no. 7 (October 1, 2002): 842-850, doi:10.1086/342383; M D Aronson and D H Bor, "Blood Cultures.," Annals of Internal Medicine 106, no. 2 (February 1987): 246-253); Bates, D. W., Goldman, L. & Lee, T. H. Contaminant blood cultures and resource utilization. The true consequences of false-positive results. JAMA 265, 365-369 (1991)).

In an attempt to overcome the turnaround time limitations of blood culture molecular nucleic acid-based tests have been developed to detect the major sepsis-causing microbial pathogens in whole blood from patients with suspected sepsis (e.g., SeptiFast® from Roche, Iridica® from Abbott, Sepsis Panel from Biofire (Biomerieux), Prove-it® Sepsis from Mobidiag, SepsiTest from Molzym Molecular Diagnostics). Whilst sensitive and specific, such assays also have limitations, especially with respect to clinical interpretation of assay results for suspected sepsis patients that are 1) PCR or assay positive and blood culture negative, and 2) PCR or assay negative (Bauer M, Reinhart K (2010) Molecular diagnostics of sepsis - Where are we today? International Journal of Medical Microbiology 300: 411-413; Ljungström, L., Enroth, H., Claesson, B. E., Ovemyr, I., Karlsson, J., Fröberg, B., et al. (2015). Clinical evaluation of commercial nucleic acid amplification tests in patients with suspected sepsis. BMC Infectious Diseases, 15(1), 199; Avolio, M., Diamante, P., Modolo, M. L., De Rosa, R., Stano, P., & Camporese, A. (2014). Direct Molecular Detection of Pathogens in Blood as Specific Rule-In Diagnostic Biomarker in Patients With Presumed Sepsis. Shock, 42(2), 86-92).

Alternative diagnostic approaches to BaSIRS have been investigated including determination of host response using biomarkers (Michael Bauer and Konrad Reinhart, "Molecular Diagnostics of Sepsis - Where Are We Today?" International Journal of Medical Microbiology 300, no. 6 (August 1, 2010): 411-413, doi:10.1016/j.ijmm.2010.04.006; John C Marshall and Konrad Reinhart, "Biomarkers of Sepsis," Critical Care Medicine 37, no. 7 (July 2009): 2290-2298, doi: 10.1097/CCM.0b013e3181a02afc.). A systematic literature search identified nearly 180 molecules as potential biomarkers of sepsis of which 20% have been assessed in appropriately designed sepsis studies including C-reactive protein (CRP), procalcitonin (PCT), and IL6 (Reinhart, K., Bauer, M., Riedemann, N. C. & Hartog, C. S. New Approaches to Sepsis: Molecular Diagnostics and Biomarkers. Clinical Microbiology Reviews 25, 609-634 (2012)). PCT is perhaps the best studied of these biomarkers (Wacker, C., Prkno, A., Brunkhorst, F. M., & Schlattmann, P. (2013). Procalcitonin as a diagnostic marker for sepsis: a systematic review and meta-analysis. The Lancet Infectious Diseases, 13(5), 426-435) but it also has its limitations with respect to determining the presence of BaSIRS and it is generally considered that PCT is a marker of systemic inflammation rather than a specific marker for BaSIRS (Hoenigl, M., Raggam, R. B., Wagner, J., Prueller, F., Grisold, A. J., Leitner, E., et al. (2014). Procalcitonin fails to predict bacteremia in SIRS patients: a cohort study. International Journal of Clinical Practice, 68(10), 1278-1281). A combination of biomarkers has been researched to gain better diagnostic performance in sepsis (Gibot, S., Bene, M. C., Noel, R., Massin, F., Guy, J., Cravoisy, A., et al. (2012). Combination biomarkers to diagnose sepsis in the critically ill patient. American Journal of Respiratory and Critical Care Medicine, 186(1), 65-71) as has the use of a single biomarker (PCT) on multiple days through the determination of a PCT ratio to rule out soft necrotizing tissue infections (Friederichs, J., Hutter, M., Hierholzer, C., Novotny, A., Friess, H., Buhren, V., & Hungerer, S. (2013). Procalcitonin ratio as a predictor of successful surgical treatment of severe necrotizing soft tissue infections. American Journal of Surgery, 206(3), 368-373).

Because of a current lack of suitable diagnostic tools that clinicians can use to diagnose BaSIRS in the ED they rely largely on clinical judgment, the presence or absence of pathognomonic clinical signs, clinical algorithms and standard international definitions. However, it has been shown that such an approach lacks discriminative ability such that patients with BaSIRS are missed or patients with non-bacterial SIRS are unnecessarily prescribed antibiotics (Gille-Johnson, P., Hansson, K. E., & Gardlund, B. (2013). Severe sepsis and systemic inflammatory response syndrome in emergency department patients with suspected severe infection. Scandinavian Journal of Infectious Diseases, 45(3), 186-193; Brown, T., Ghelani-Allen, A., Yeung, D., & Nguyen, H. B. (2014). Comparative effectiveness of physician diagnosis and guideline definitions in identifying sepsis patients in the emergency department. Journal of Critical Care; Craig, J. C., Williams, G. J., Jones, M., Codarini, M., Macaskill, P., Hayen, A., et al. (2010). The accuracy of clinical symptoms and signs for the diagnosis of serious bacterial infection in young febrile children: prospective cohort study of 15,781 febrile illnesses. BMJ (Clinical Research Ed.), 340, c1594).

Whilst there is a reasonable body of knowledge describing biomarkers capable of determining the presence of sepsis, or predicting likelihood of mortality in patients at risk of sepsis, the literature is silent on identifying biomarkers that have high negative predictive value for a systemic host response to infection in an heterogenous patient population with a low to medium prevalence of systemic inflammation. Biomarkers with high negative predictive value would have clinical utility in that they provide clinicians with the confidence to send patients home, or withhold antibiotics, despite the presence of clinical signs of systemic inflammation.

## SUMMARY OF THE INVENTION

The present invention arises from the discovery that certain host response peripheral blood expression products, including RNA transcripts, are specifically and differentially expressed in patients presenting to emergency departments with systemic inflammation associated with bacterial infection. Surprisingly these expression products have high negative predictive value and, as such, are useful in excluding a bacterial infection as the cause of the presenting clinical signs associated with systemic inflammation (*e.g.*, fever, increased heart rate, increased respiratory rate, increased white blood cell count).

Based on this determination, the present inventors have developed various methods, apparatus, compositions, and kits, which take advantage of these differentially expressed biomarkers (which are referred to herein as 'rule out' (RO) BaSIRS biomarkers, including ratios thereof (derived RO BaSIRS biomarkers), to exclude the presence of BaSIRS in subjects presenting to emergency departments with fever or clinical signs of systemic inflammation. In certain embodiments, these methods, apparatus, compositions, and kits represent a significant advance over prior art processes and products, which have not been able to distinguish BaSIRS from other etiologies of systemic inflammation, including viruses, trauma, autoimmune disease, allergy and cancer.

Accordingly, in one aspect, the present invention provides methods for determining an indicator used in assessing a likelihood of a subject presenting to emergency having an absence of BaSIRS. These methods generally comprise, consist or consist essentially of: (1) determining biomarker values that are measured or derived for at least two (*e.g.*, 2, 3, 4, 5, 6, 7, 8, 9, 10, or more) corresponding RO BaSIRS biomarkers in a sample taken from the subject and that is at least partially indicative of the levels of the RO BaSIRS biomarkers in the sample; and (2) determining the indicator using the biomarker values. Suitably, the methods further comprise ruling out the likelihood of BaSIRS for the subject or not, based on the indicator.

Thus, in a related aspect, the present invention provides methods for ruling out the likelihood of BaSIRS (*i.e.*, for diagnosing the absence of BaSIRS), or not, for a subject presenting to emergency having an absence of BaSIRS. These methods generally comprise, consist or consist essentially of: (1) determining biomarker values that are measured or derived for at least two (*e.g.*, 2, 3, 4, 5, 6, 7, 8, 9, 10, or more) corresponding RO BaSIRS biomarkers in a sample taken from the subject and that is at least partially indicative of the levels of the RO BaSIRS biomarkers in the sample; (2) determining the indicator using the biomarker values; and (3) ruling out the likelihood of BaSIRS for the subject or not, based on the indicator.

The subject typically has at least one clinical sign of systemic inflammatory response syndrome (SIRS). The at least two RO BaSIRS biomarkers are suitably not biomarkers of at least one other SIRS condition (*e.g.*, 1, 2, 3, 4 or 5 other SIRS conditions) selected from the group consisting of: autoimmune disease associated SIRS (ADaSIRS), cancer associated SIRS (CANaSIRS), trauma associated SIRS (TRAUMaSIRS), anaphylaxis associated SIRS (ANAPHYLaSIRS), schizophrenia associated SIRS (SCHIZaSIRS) and virus associated SIRS (VaSIRS). The sample is suitably a biological sample, representative examples of which include blood samples including peripheral blood samples, and leukocyte samples. The at least two RO BaSIRS biomarkers and their corresponding biomarkers values on which an indicator is determined that is indicative of the likelihood of the absence of BaSIRS, and on which the likelihood of BaSIRS is ruled out, or not, define a RO BaSIRS biomarker profile.

In specific embodiments, the at least two RO BaSIRS biomarkers are expression products of a gene selected from the group consisting of: *ADAM19, ADD1, ADGRE1, AIF1, AKAP7, AKT1, AKTIP, ALDOA, AMD1, ARL2BP, ATG9A, ATP13A3, ATP6VOA1, ATP8B4, BRD7, BTG2, C21orf59, C6orf48, CCND2, CD44, CD59, CDC14A, CERK, CHPT1, CLEC4E, CLU, CNBP, COMMD4, COQ10B, COX5B, CPVL, CTDSP2, CTSA, CTSC, CTSH, CYBB, CYP20A1, DERA,*

*DHX16, DIAPH2, DLST, EIF4A2, EIF4E2, EMP3, ENO1, FBX07, FCER1G, FGL2, FLVCR2, FTL, FURIN, FUT8, FXR1, GAPDH, GAS7, GBP2, GIMAP4, GLOD4, GNS, GRAP2, GSTO1, HEBP1, HIST1H2BM, HIST1H3C, HIST1H4L, HLA-DPA1, HMG20B, HMGN4, HOXB6, HSPA4, ID3, IFIT1, IFNGR2, IL7R, IMP3, IMPDH1, INPP1, ISG20, ITGAX, ITGB1, KATNA1, KLF2, KLRF1, LAMP1, LFNG, LHFPL2, LILRB3, LTA4H, LTF, MAP4K2, MAPK14, MAPK8IP3, MCTP1, MEGF9, METTL9, MFSD10, MICAL1, MMP8, MNT, MRPS18B, MUT, MX1, MYL9, MYOM2, NAGK, NMI, NUPL2, OBFC1, OSBPL9, PAFAH2, PARL, PDCD5, PDGFC, PHB, PHF3, PLAC8, PLEKHG3, PLEKHM2, POLR2C, PPP1CA, PPP1CB, PPP1R11, PROS1, PRPF40A, PRRG4, PSMB4, PSTPIP2, PTPN2, PUS3, RAB11FIP1, RAB11FIP3, RAB9A, RANBP10, RASGRP2, RASGRP3, RASSF7, RDX, RNASE6, RNF34, RPA2, RPS6K82, RPS8, S100A12, S100P, SASH3, SBF1, 5DF2L1, SDHC, SERTAD2, SH3BGRL, SH3GLB2, SLAMF7, SLC11A2, SLC12A9, SLC25A37, SLC2A3, SLC39A8, SLC9A3R1, SNAPC1, SORT1, SSBP2, ST3GAL5, ST3GAL6, STK38, SYNE2, TAX1BP1, TIMP1, TINF2, TLR5, TMEM106C, TMEM80, TOB1, TPP2, TRAF3IP2, USP3, VAV1, WDR33, YPEL5,* and *ZBTB17.* Non-limiting examples of nucleotide sequences for these RO BaSIRS biomarkers are listed in SEQ ID NOs: 1-179. Non-limiting examples of amino acid sequences for these RO BaSIRS biomarkers are listed in SEQ ID NOs: 180-358. In illustrative examples, an individual RO BaSIRS biomarker is selected from the group consisting of: (a) a polynucleotide expression product comprising a nucleotide sequence that shares at least 70% (or at least 71% to at least 99% and all integer percentages in between) sequence identity with the sequence set forth in any one of SEQ ID NO: 1-179, or a complement thereof; (b) a polynucleotide expression product comprising a nucleotide sequence that encodes a polypeptide comprising the amino acid sequence set forth in any one of SEQ ID NO: 180-358; (c) a polynucleotide expression product comprising a nucleotide sequence that encodes a polypeptide that shares at least 70% (or at least 71% to at least 99% and all integer percentages in between) sequence similarity or identity with at least a portion of the sequence set forth in SEQ ID NO: 180-358; (d) a polynucleotide expression product comprising a nucleotide sequence that hybridizes to the sequence of (a), (b), (c) or a complement thereof, under medium or high stringency conditions; (e) a polypeptide expression product comprising the amino acid sequence set forth in any one of SEQ ID NO: 180-358; and (f) a polypeptide expression product comprising an amino acid sequence that shares at least 70% (or at least 71% to at least 99% and all integer percentages in between) sequence similarity or identity with the sequence set forth in any one of SEQ ID NO: 180-358.

The RO BaSIRS biomarkers of the present invention have strong negative predictive value when combined with one or more other RO BaSIRS biomarkers. In some embodiments, pairs of biomarkers are used to determine the indicator. In illustrative examples of this type, one biomarker of a biomarker pair is selected from Group A RO BaSIRS biomarkers and the other is selected from Group B RO BaSIRS biomarkers, wherein an individual Group A RO BaSIRS biomarker is an expression product of a gene selected from the group consisting of: *DIAPH2, CYBB, SLC39A8, PRPF40A, MUT, NMI, PUS3, MNT, SLC11A2, FXR1, SNAPC1, PRRG4, SLAMF7, MAPK8IP3, GBP2, PPP1CB, TMEM80, HIST1H2BM, NAGK, HIST1H4L* and wherein an individual Group B RO BaSIRS biomarker is an expression product of a gene selected from the group consisting of: *SERTAD2, PHF3, BRD7, TOB1, MAP4K2, WDR33, BTG2, AMD1, RNASE6, RAB11FIP1, ADD1, HMG20B.*

In other illustrative examples, one biomarker of a biomarker pair is selected from Group C RO BaSIRS biomarkers and the other is selected from Group D RO BaSIRS biomarkers, wherein an individual Group C RO BaSIRS biomarker is an expression product of a gene selected from the group consisting of: *PARL, AIF1, PTPN2, COX5B, PSMB4, EIF4E2, RDX, DERA, CTSH, HSPA4, VAV1, PPP1CA, CPVL, PDCD5,* and wherein an individual Group D RO BaSIRS biomarker is an expression product of a gene selected from the group consisting of: *PAFAH2, IMP3, GLOD4, IL7R, ID3, KLRF1, SBF1, CCND2, LFNG, MRPS18B, HLA-DPA1, SLC9A3R1, HMGN4, C6orf48, ARL2BP, CDC14A, RPA2, ST3GAL5, EIF4A2, CERK, RASSF7, PHB, TRAF3IP2, KLF2, RAB11FIP3, C21orf59, SSBP2, GIMAP4, CYP20A1, RASGRP2, AKT1, HCP5, TPP2, SYNE2, FUT8, NUPL2, MYOM2, RPS8, RNF34, DLST, CTDSP2, EMP3, PLEKHG3, DHX16, RASGRP3, COMMD4, ISG20, POLR2C, SH3GLB2, SASH3, GRAP2, RPS6KB2, FGL2, AKAP7, SDF2L1, FBX07, MX1, IFIT1, TMEM106C, RANBP10.*

In other illustrative examples, one biomarker of a biomarker pair is selected from Group E RO BaSIRS biomarkers and the other is selected from Group F RO BaSIRS biomarkers, wherein an individual Group E RO BaSIRS biomarker is an expression product of a gene selected from the group consisting of: *SORT1, GAS7, FLVCR2, TLR5, FCER1G, SLC2A3, S100A12, PSTPIP2, GNS, METTL9, MMP8, MAPK14, CD59, CLEC4E, MICAL1, MCTP1, GAPDH, IMPDH1, ATP8B4, EMR1, SLC12A9, S100P, IFNGR2, PDGFC, CTSA, ALDOA, ITGAX, GST01, LHFPL2, LTF, SDHC, TIMP1, LTA4H, USP3, MEGF9, FURIN, ATP6VOA1, PROS1, ATG9A, PLAC8, LAMP1, COQ108, ST3GAL6, CTSC, ENO1, OBFC1, TAX1BP1, MYL9, HIST1H3C, ZBTB17, CHPT1, SLC25A37, PLEKHM2, LILRB3, YPEL5, FTL, SH3BGRL, HOXB6, PPP1R11, CLU, HEBP1,* and wherein an individual Group F RO BaSIRS biomarker is an expression product of a gene selected from the group consisting of: *OSBPL9, CD44, AKTIP, ATP13A3, ADAM19, KATNA1, STK38, TINF2, RAB9A, INPP1, CNBP, ITGB1, MFSD10.*

In some embodiments, biomarker values are measured or derived for a Group A RO BaSIRS biomarker and for a Group B RO BaSIRS biomarker, and the indicator is determined by combining the biomarker values. In some embodiments, biomarker values are measured or derived for a Group C RO BaSIRS biomarker and for a Group D RO BaSIRS biomarker, and the indicator is determined by combining the biomarker values. In some embodiments, biomarker

7

values are measured or derived for a Group E RO BaSIRS biomarker and for a Group F RO BaSIRS biomarker, and the indicator is determined by combining the biomarker values. In other embodiments, biomarker values are measured or derived for a Group A RO BaSIRS biomarker, for a Group B RO BaSIRS biomarker, for a Group C RO BaSIRS biomarker and for a Group D RO BaSIRS biomarker, and the indicator is determined by combining the biomarker values. In other embodiments, biomarker values are measured or derived for a Group A RO BaSIRS biomarker, for a Group B RO BaSIRS biomarker, for a Group C RO BaSIRS biomarker, for a Group D RO BaSIRS biomarker, for a Group E RO BaSIRS biomarker, for a Group F RO BaSIRS biomarker, and the indicator is determined by combining the biomarker values. Suitably, in the above embodiments, the methods comprise combining the biomarker values using a combining function, wherein the combining function is at least one of: an additive model; a linear model; a support vector machine; a neural network model; a random forest model; a regression model; a genetic algorithm; an annealing algorithm; a weighted sum; a nearest neighbor model; and a probabilistic model.

In some embodiments, the methods comprise: (a) determining a pair of biomarker values, each biomarker value being a value measured or derived for at least one corresponding RO BaSIRS biomarker; (b) determining a derived biomarker value using the pair of biomarker values, the derived biomarker value being indicative of a ratio of concentrations of the pair of RO BaSIRS biomarkers; and determining the indicator using the derived marker value. In illustrative examples of this type, biomarker values are measured or derived for a Group A RO BaSIRS biomarker and for a Group B RO BaSIRS biomarker to obtain the pair of biomarker values and the derived biomarker value is determined using the pair of biomarker values. In other illustrative examples, biomarker values are measured or derived for a Group C RO BaSIRS biomarker and for a Group D RO BaSIRS biomarker to obtain the pair of biomarker values and the derived biomarker value is determined using the pair of biomarker values. In other illustrative examples, biomarker values are measured or derived for a Group E RO BaSIRS biomarker and for a Group F RO BaSIRS biomarker to obtain the pair of biomarker values and the derived biomarker value is determined using the pair of biomarker values.

In some embodiments, the methods comprise: (a) determining a first derived biomarker value using a first pair of biomarker values, the first derived biomarker value being indicative of a ratio of concentrations of first and second RO BaSIRS biomarkers; (b) determining a second derived biomarker value using a second pair of biomarker values, the second derived biomarker value being indicative of a ratio of concentrations of third and fourth RO BaSIRS biomarkers; (c) determining a third derived biomarker value using a third pair of biomarker values, the third derived biomarker value being indicative of a ratio of concentrations of fifth and sixth RO BaSIRS biomarkers; and (d) determining the indicator by combining the first, second and third derived biomarker values. Suitably, the first RO BaSIRS biomarker is selected from Group A RO BaSIRS biomarkers, the second RO BaSIRS biomarker is selected from Group B RO BaSIRS biomarkers, the third RO BaSIRS biomarker is selected from Group C RO BaSIRS biomarkers, the fourth RO BaSIRS biomarker is selected from Group D RO BaSIRS biomarkers, the fifth RO BaSIRS biomarker is selected from Group E RO BaSIRS biomarkers, and the sixth RO BaSIRS biomarker is selected from Group F RO BaSIRS biomarkers. In illustrative examples of this type, the methods comprise combining the biomarker values using a combining function, wherein the combining function is at least one of: an additive model; a linear model; a support vector machine; a neural network model; a random forest model; a regression model; a genetic algorithm; an annealing algorithm; a weighted sum; a nearest neighbor model; and a probabilistic model.

Suitably, in embodiments that utilize pairs of RO BaSIRS biomarkers as broadly described above and elsewhere herein, an individual pair of RO BaSIRS biomarkers has a mutual correlation in respect of ruling out BaSIRS that lies within a mutual correlation range, the mutual correlation range being between $\pm0.9$ (or between $\pm0.8$, $\pm0.7$, $\pm0.6$, $\pm0.5$, $\pm0.4$, $\pm0.3$, $\pm0.2$ or $\pm0.1$) and the indicator has a performance value greater than or equal to a performance threshold representing the ability of the indicator to diagnose the absence of BaSIRS, wherein the performance threshold is indicative of an explained variance of at least 0.3. In illustrative examples of this type, an individual RO BaSIRS biomarker has a condition correlation with the absence of RO BaSIRS that lies outside a condition correlation range, wherein the condition correlation range is between $\pm0.3$. In other illustrative examples, an individual RO BaSIRS biomarker has a condition correlation with the absence of BaSIRS that lies outside a condition correlation range, wherein the condition correlation range is at least one of $\pm0.9$, $\pm0.8$, $\pm0.7$, $\pm0.6$, $\pm0.5$ or $\pm0.4$. In specific embodiments, the performance threshold is indicative of an explained variance of at least one of 0.4, 0.5, 0.6, 0.7, 0.8 and 0.9.

In certain embodiments that utilize pairs of RO BaSIRS biomarkers as broadly described above and elsewhere herein the Group A RO BaSIRS biomarker is suitably an expression product of *DIAPH2,* the Group B RO BaSIRS biomarker is suitably an expression product of *SERTAD2,* the Group C RO BaSIRS biomarker is suitably an expression product of *PARL,* the Group D RO BaSIRS biomarker is suitably an expression product of *PAFAH2,* the Group E RO BaSIRS biomarker is suitably an expression product of *SORT1,* and the Group F RO BaSIRS biomarker is suitably an expression product of *OSBPL9.*

Another aspect of the present invention provides apparatus for determining an indicator used in assessing a likelihood of a subject having an absence of BaSIRS. This apparatus generally comprises at least one electronic processing device that:

a) determines a pair of biomarker values, each biomarker value being a value measured or derived for at least one corresponding RO BaSIRS biomarker, as broadly described above and elsewhere herein, of a sample taken from the subject and being at least partially indicative of a concentration of the RO BaSIRS biomarker in the sample;

b) determines a derived biomarker value using the pair of biomarker values, the derived biomarker value being indicative of a ratio of concentrations of the pair of RO BaSIRS biomarkers; and

c) determines the indicator using the derived biomarker value.

In yet another aspect, the present invention provides compositions for determining an indicator used in assessing a likelihood of a subject having an absence of BaSIRS. These compositions generally comprise, consist or consist essentially of at least one pair of cDNAs and at least one oligonucleotide primer or probe that hybridizes to an individual one of the cDNAs, wherein the at least one pair of cDNAs is selected from pairs of cDNAs including a first pair, a second pair and a third pair of cDNAs, wherein the first pair comprises a Group A RO BaSIRS biomarker cDNA and a Group B RO BaSIRS biomarker cDNA, and wherein the second pair comprises a Group C RO BaSIRS biomarker cDNA and a Group D RO BaSIRS biomarker cDNA, and wherein the third pair comprises a Group E RO BaSIRS biomarker cDNA and a Group F RO BaSIRS biomarker cDNA. Suitably, the compositions comprise a population of cDNAs corresponding to mRNA derived from a cell or cell population. In some embodiments, the cell is a cell of the immune system, suitably a leukocyte. In some embodiments, the cell population is blood, suitably peripheral blood. In some embodiments, the at least one oligonucleotide primer or probe is hybridized to an individual one of the cDNAs. In any of the above embodiments, the composition may further comprise a labeled reagent for detecting the cDNA. In illustrative examples of this type, the labeled reagent is a labeled said at least one oligonucleotide primer or probe. In other embodiments, the labeled reagent is a labeled said cDNA. Suitably, the at least one oligonucleotide primer or probe is in a form other than a high density array. In non-limiting examples of these embodiments, the compositions comprise labeled reagents for detecting and/or quantifying no more than 4, 5, 6, 7, 8, 9, 10, 15, 20, 30, 40 or 50 different RO BaSIRS biomarker cDNAs. In specific embodiments, the compositions comprise for a respective cDNA, (1) two oligonucleotide primers (e.g., nucleic acid amplification primers) that hybridize to opposite complementary strands of the cDNA, and (2) an oligonucleotide probe that hybridizes to the cDNA. In some embodiments, one or both of the oligonucleotide primers are labeled. In some embodiments, the oligonucleotide probe is labeled. In illustrative examples, the oligonucleotide primers are not labeled and the oligonucleotide probe is labeled. Suitably, in embodiments in which the oligonucleotide probe is labeled, the labeled oligonucleotide probe comprises a fluorophore. In representative examples of this type, the labeled oligonucleotide probe further comprises a quencher. In certain embodiments, different labeled oligonucleotide probes are included in the composition for hybridizing to different cDNAs, wherein individual oligonucleotide probes comprise detectably distinct labels (e.g. different fluorophores).

Still another aspect of the present invention provides kits for determining an indicator which is indicative of the likelihood of the absence of BaSIRS, and on which the likelihood of BaSIRS is ruled out or not. The kits generally comprise, consist or consist essentially of at least one pair of reagents selected from reagent pairs including a first pair of reagents, a second pair of reagents and a third pair of reagents, wherein the first pair of reagents comprises (i) a reagent that allows quantification of a Group A RO BaSIRS biomarker; and (ii) a reagent that allows quantification of a Group B RO BaSIRS biomarker, wherein the second pair of reagents comprises: (iii) a reagent that allows quantification of a Group C RO BaSIRS biomarker; and (iv) a reagent that allows quantification of a Group D RO BaSIRS biomarker, and wherein the third pair of reagents comprises: (v) a reagent that allows quantification of a Group E RO BaSIRS biomarker; and (vi) a reagent that allows quantification of a Group F RO BaSIRS biomarker. In non-limiting examples of these embodiments, the kits comprise labeled reagents for detecting and/or quantifying no more than 4, 5, 6, 7, 8, 9, 10, 15, 20, 30, 40 or 50 different RO BaSIRS biomarker polynucleotides (*e.g.*, mRNAs, cDNAs, *etc.*).

In a further aspect, the present invention provides methods for managing a subject with at least one clinical sign of SIRS. These methods generally comprise, consist or consist essentially of: not exposing the subject to a treatment regimen for specifically treating BaSIRS based on an indicator obtained from an indicator-determining method, wherein the indicator is indicative of the absence of BaSIRS in the subject, and of ruling out the likelihood of the presence of BaSIRS in the subject, and wherein the indicator-determining method is an indicator-determining method as broadly described above and elsewhere herein. In some embodiments, when the indicator is indicative of the absence of BaSIRS in the subject, the methods further comprise exposing the subject to a non-BaSIRS treatment. In illustrative examples of this type, the non-BaSIRS treatment is a treatment for a SIRS other than BaSIRS (*e.g.*, a treatment for ADaSIRS, CANaSIRS, TRAUMaSIRS, ANAPHYLaSIRS, SCHIZaSIRS and VaSIRS). In other embodiments, when the indicator is indicative of the absence of BaSIRS in the subject, the methods further comprises not exposing the subject to a treatment. In some embodiments, the methods further comprise taking a sample from the subject and determining an indicator indicative of the likelihood of the absence of BaSIRS using the indicator-determining method. In other embodiments, the methods further comprise sending a sample taken from the subject to a laboratory at which the indicator is determined according to the indicator-determining method. In these embodiments, the methods suitably further comprise receiving the indicator from the laboratory.

**BRIEF DESCRIPTION OF THE DRAWINGS**

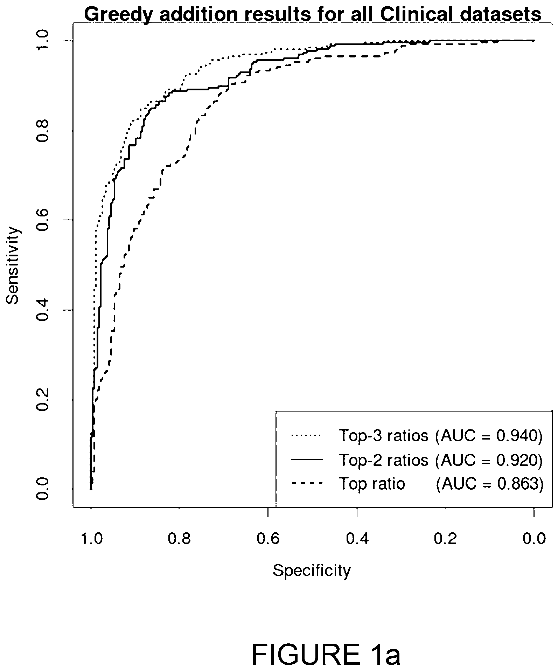

Figure 1a: ROC curves for the components of the derived biomarker signature consisting of DIAPH2 / SERTAD2; PARL/ PAFAH2; SORT1 / OSBPL9. The dashed line is the ROC curve for DIAPH2 / SERTAD2 alone (AUC = 0.863). The full line is for the combination of DIAPH2 / SERTAD2; PARL / PAFAH2 (AUC = 0.92). The dotted line is for the combination of DIAPH2 / SERTAD2; PARL / PAFAH2; SORT1 / OSBPL9 (AUC = 0.94).

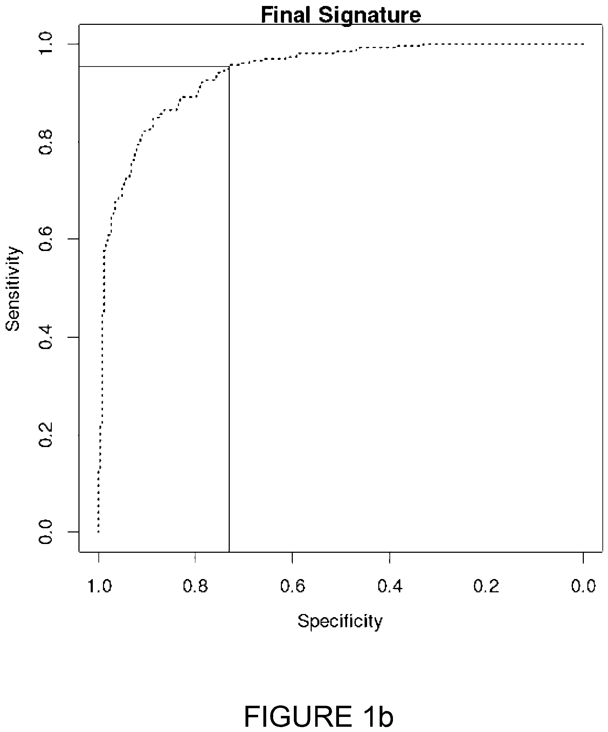

Figure 1b: ROC curve for the final triage signature consisting of DIAPH2 / SERTAD2; PARL/ PAFAH2; SORT1 / OSBPL9 indicating the chosen specificity and sensitivity used to determine AUC and NPV at set prevalences of 10% and 5% (see Table 6 and Table 7), and NPV at prevalences of 4%, 6%, 8% and 10% (see Table 8).

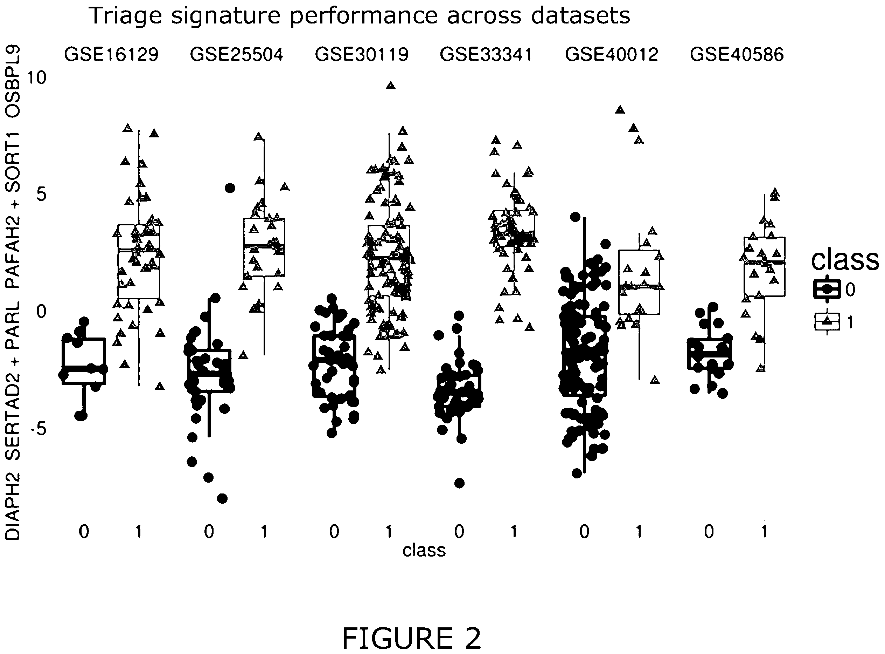

Figure 2: Box and whisker plots of the performance of the combined derived biomarker signature (DIAPH2 / SERTAD2; PARL / PAFAH2; SORT1 / OSBPL9) in the BaSIRS datasets. Dark dots represent the control samples (those subjects without BaSIRS) and lighter dots represent samples from those patients with BaSIRS.

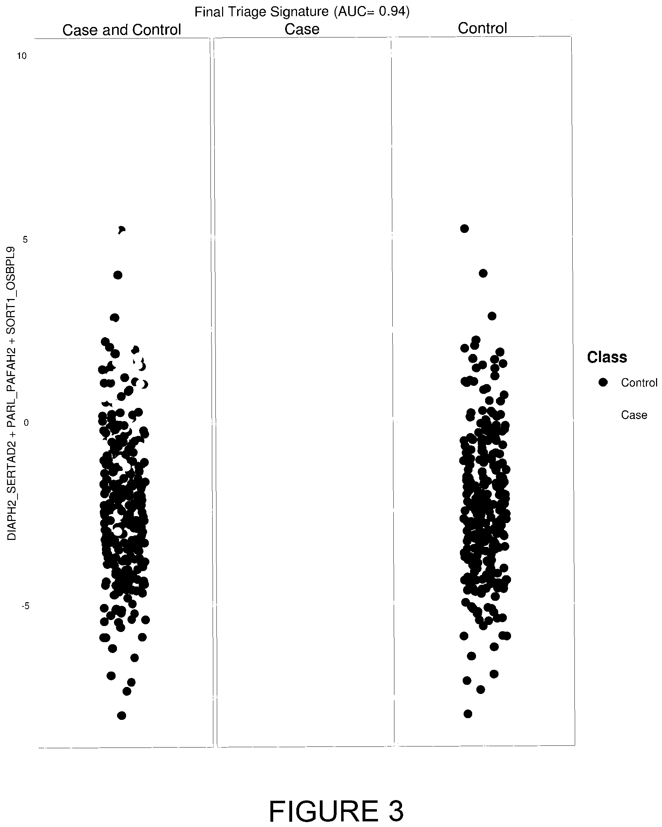

Figure 3: Scatter plot showing performance of the combined derived biomarker signature (DIAPH2 / SERTAD2; PARL / PAFAH2; SORT1 / OSBPL9) in all of the samples in the BaSIRS datasets. Dark dots represent the control samples (those subjects without BaSIRS) and lighter dots represent samples from those patients with BaSIRS (case). The AUC for the combined derived biomarker signature is 0.94.

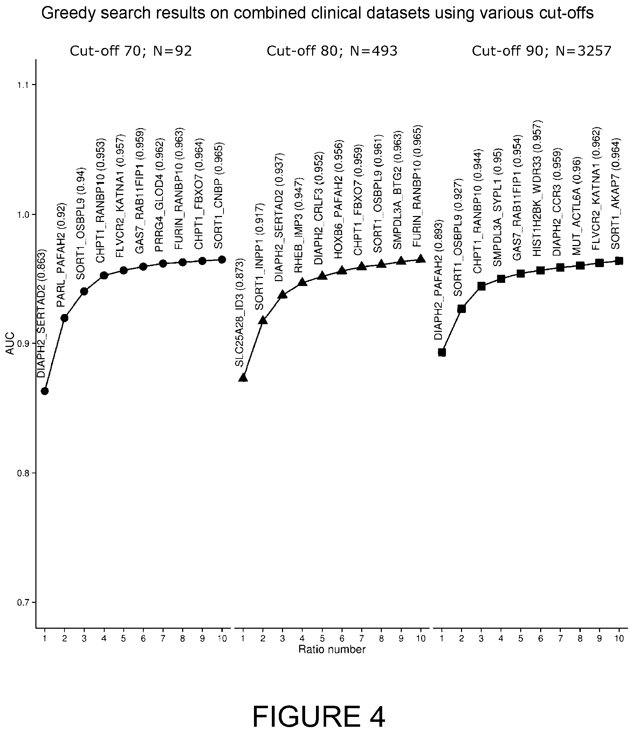

Figure 4: Plots of AUC versus the number and identity of biomarker ratios applying a correlation filter at different coefficient cut-off values. Correlation cut-off values of 70, 80 and 90 were used for selecting derived biomarkers from the non-BaSIRS datasets by removing ratios with high pair-wise correlations. As such the data was enriched to contain ratios with orthogonal information, i.e. ratios that contain biologically relevant information but have lower correlation to each other. Such derived biomarkers were then subtracted from the pool of derived biomarkers from the BaSIRS datasets. The lower the cut-off value the larger the number of derived biomarkers that were subtracted. As such, 92, 493 and 3257 derived biomarkers remained following subtraction when using cut-off values of 70, 80 and 90 respectively. Ultimately a cut-off of 70 was used to ensure specificity in the final derived biomarker signature (see curve on the left hand side). Looking at the curves it can be seen that the AUC increases with each successive addition of a derived biomarker. It was considered that a combination of three derived biomarkers provided the best AUC (0.94) with the least likelihood of introduction of noise. As such, the combination of DIAPH2 / SERTAD2; PARL / PAFAH2; SORT1 / OSBPL9 was considered to have the greatest commercial utility.

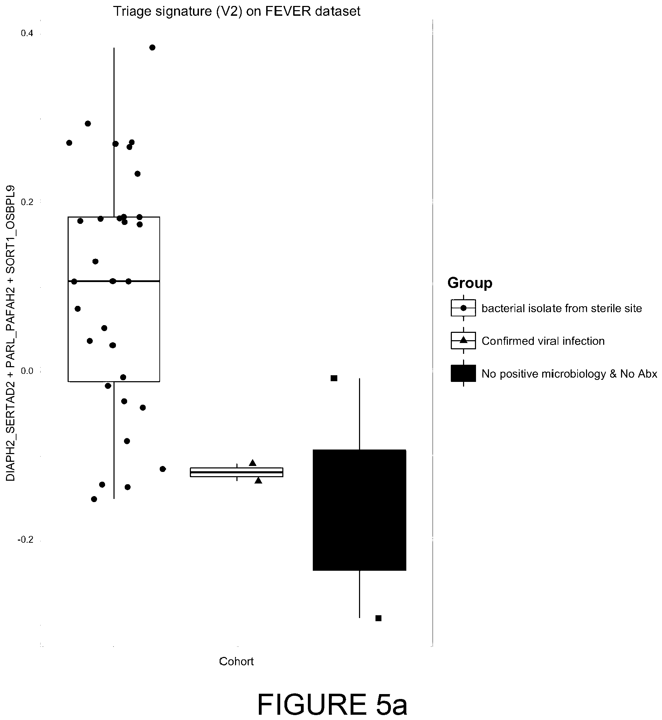

Figure 5a: Box and whisker plot of the results of validation of this six biomarker signature on an unseen validation set of ED patients presenting with fever, the AUC was 0.903 between bacterial positive patients and all others (viral positive and bacterial negative pooled). Each patient was clinically and retrospectively (note, not at the time the sample was taken) confirmed as having either a bacteria isolated from a sterile site, a confirmed viral infection or no positive microbiology result (and the patient was not on antibiotics). Each patient sample had a SeptiCyte Triage score calculated (Y axis on left hand side). In this instance, on a scale of minus 0.4 to positive 0.4, it can be seen that patients with positive clinical microbiology obtain a higher Diagnostic Score compared to those without positive microbiology. Patients with a confirmed viral infection (only) also have a low Diagnostic Score. Further, it can be seen that an arbitrary cut-off line can be drawn that more or less separates the two conditions depending upon the desired false negative or false positive rate (when using clinical microbiology as the gold standard).

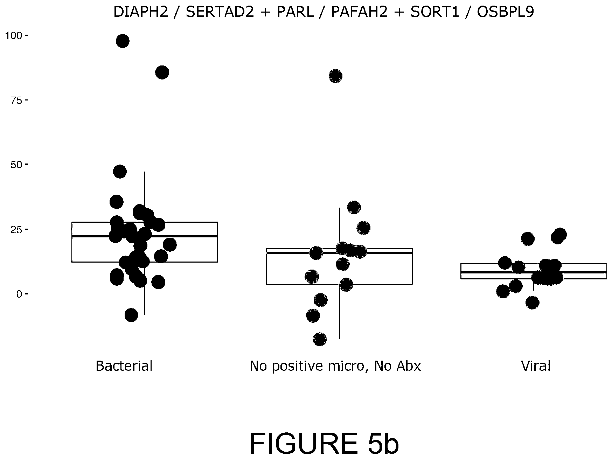

Figure 5b: Box and whisker plot of the results of validation of a six biomarker signature, DIAPH2 / SERTAD2; PARL / PAFAH2; SORT1 / OSBPL9, on an expanded cohort of 59 ED patients presenting with fever and admitted to hospital. Each patient was clinically and retrospectively (note, not at the time the sample was taken) confirmed as having either a bacteria isolated from a sterile site ("bacterial", n=32), a confirmed viral infection (virus identified = "Viral", n=14) or no positive microbiology result (and the patient was not on antibiotics and the condition resolved = "No positive micro, no Abx", n=13). Only those patients suspected of having a viral infection were tested for the presence of the suspected virus. Each patient sample had a SeptiCyte Triage score calculated (Y axis on left hand side). In this instance, it can be seen that patients with positive clinical microbiology obtain a higher Diagnostic Score compared to those without positive microbiology. Patients with a confirmed viral infection (only) also have a lower Diagnostic Score. AUCs for bacterial vs viral and bacterial vs indeterminate are 0.79 and 0.65 respectively. Negative Predictive Value (NPV) for bacterial vs other is 0.975 (at a sepsis prevalence of 4%, specificity of 0.78, sensitivity of 0.53 and threshold 25). It should be noted that patients were selected based on presenting signs of a fever, which is not a good indicator of a bacterial infection and, as such, this patient cohort is not fully representative of patients that would be tested for being at risk of sepsis. Further, not all patients received comprehensive microbial or viral testing and, as such, the final diagnosis for some patients is based on clinical impression only. The performance of individual ratios in this signature can be found in Table 6.

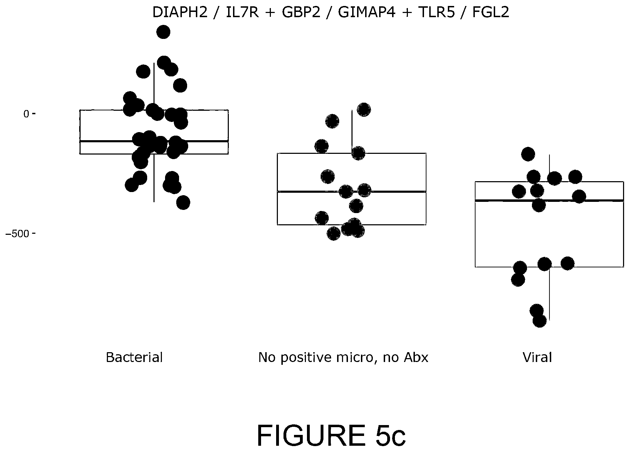

Figure 5c: Box and whisker plot of the results of validation of another six biomarker signature, DIAPH2 / IL7R + GBP2 / GIMAP4 + TLR5 / FGL2 (using biomarkers from different groups for each ratio), on an expanded cohort of 59 ED patients presenting with fever and admitted to hospital. Each patient sample had a SeptiCyte Triage score calculated (Y axis on left hand side). In this instance, it can be seen that patients with positive clinical microbiology obtain a higher Diagnostic Score compared to those without positive microbiology. Patients with a confirmed viral infection (only) also have a lower Diagnostic Score. AUCs for bacterial vs viral and bacterial vs indeterminate are 0.93 and 0.83 respectively. Negative Predictive Value (NPV) for bacterial vs other is 0.978 (at a sepsis prevalence of 4%, specificity of 0.9, sensitivity of 0.53 and threshold 0.00). The performance of individual ratios in this signature can be found in Table 6.

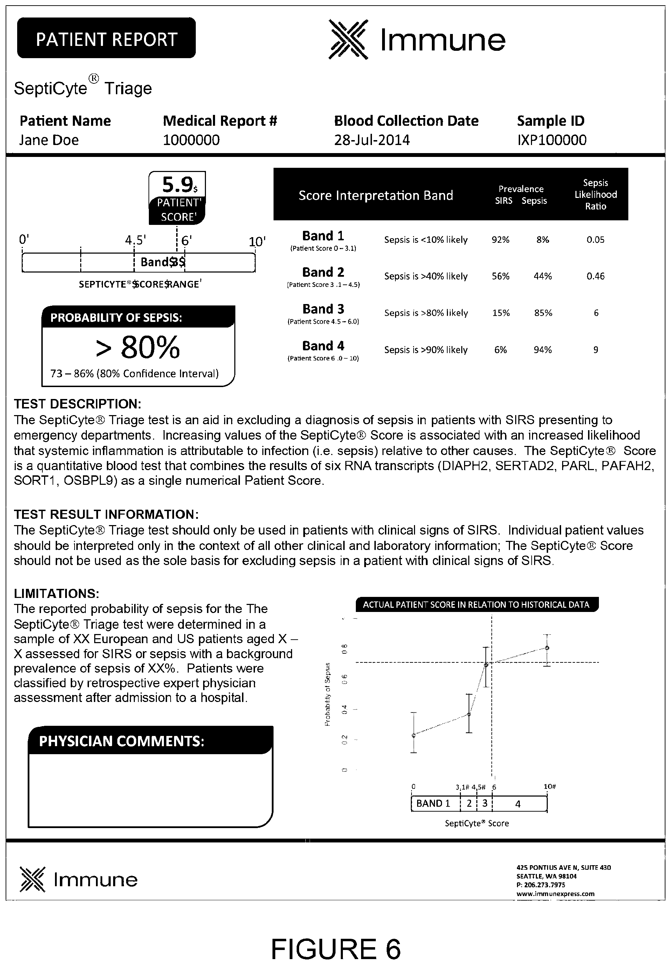

Figure 6: Example output depicting an indicator that is useful for assessing the absence of BaSIRS in a patient. In this instance the patient had a score of 5.9 indicating a >80% likelihood of BaSIRS.

## BRIEF DESCRIPTION OF THE TABLES

Table 1: List and condition description of public datasets (GEO) used to find the best performing BaSIRS derived biomarkers for use in a triage setting, including the number of subjects in each cohort (in brackets).

Table 2: List and condition description of public datasets (GEO) used to find the best performing non-bacterial SIRS derived biomarkers. These were then subtracted from the BaSIRS derived biomarkers identified from the datasets in Table 1. Note that other datasets were used to derive a set of specific viral derived biomarkers which were also subtracted from the BaSIRS derived biomarkers identified from the datasets in Table 1.

Table 3: The mean cumulative performance (AUC) in the BaSIRS datasets of the derived biomarkers (that comprise the three derived biomarker signature) when each are added sequentially.

Table 4: Results of greedy searches to find the best performing derived biomarkers (when added sequentially up to 10) using the combined bacterial datasets. Three different cut-off values were used ($r$ = 70, 80 and 90) for derived biomarkers in the non-bacterial datasets. Using a low cut-off value in the non-bacterial datasets resulted in more derived biomarkers that were taken from the pool of derived biomarkers identified using the bacterial datasets. Hence, the total numbers of derived biomarkers remaining after subtraction were 92, 493 and 3257 for cut-off values of 70, 80 and 90 respectively. The best combination of derived biomarkers with the maximum AUC, maximum specificity, minimum noise and highest commercial utility was considered to be DIAPH2 / SERTAD2; PARL / PAFAH2; SORT1 / OSBPL9 obtained after the third greedy search iteration.

Table 5 (a and b): Groups of derived biomarkers (A-F) based on their correlation to each individual biomarker in the three derived biomarker signature of DIAPH2 / SERTAD2; PARL / PAFAH2; SORT1 / OSBPL9. Groups A-C are contained in Table 5a and Groups D-F are contained in Table 5b. A DNA SEQ ID# is provided for each biomarker HUGO gene symbol.

Table 6: Performance of 200 derived biomarkers at a set sepsis prevalence of 10%. Performance measures include Area Under Curve (AUC) and Negative Predictive Value (NPV). The NPV of these derived biomarkers increases as the prevalence of sepsis decreases, so all those listed would perform well in an emergency room setting where the prevalence of sepsis is estimated to be closer to 4%.

Table 7: Performance of 200 derived biomarkers at a set sepsis prevalence of 5%.

Table 8: Table of calculated negative predictive values (NPV) for the final triage signature (DIAPH2 / SERTAD2; PARL / PAFAH2; SORT1 / OSBPL9) at sepsis prevalences of 4, 6, 8 and 10%. Based on the scientific literature, the prevalence of sepsis in the ER is approximately 4%. For these calculations the sensitivity and specificity were set at 0.9535 and 0.7303 respectively based on the ROC curve for the final triage signature (see Figure 1b).

Table 9: List of numerators and denominators that occur more than once in the top 200 derived biomarkers.

Table 10: SEQ ID numbers, HUGO gene symbol and Ensembl ID for individual biomarkers.

Table 11: SEQ ID numbers, HUGO gene symbol and Ensembl ID for individual biomarkers.

## DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (*i.e.*, to at least one) of the grammatical object of the article. By way of example, "an element" means one element or more than one element.

As used herein, "and/or" refers to and encompasses any and all possible combinations of one or more of the associated listed items, as well as the lack of combinations when interpreted in the alternative (or).

The term "biomarker" broadly refers to any detectable compound, such as a protein, a peptide, a proteoglycan, a glycoprotein, a lipoprotein, a carbohydrate, a lipid, a nucleic acid (*e.g.*, DNA, such as cDNA or amplified DNA, or RNA, such as mRNA), an organic or inorganic chemical, a natural or synthetic polymer, a small molecule (*e.g.*, a metabolite), or a discriminating molecule or discriminating fragment of any of the foregoing, that is present in or derived from a sample. "Derived from" as used in this context refers to a compound that, when detected, is indicative of a particular molecule being present in the sample. For example, detection of a particular cDNA can be indicative of the presence of a particular RNA transcript in the sample. As another example, detection of or binding to a particular antibody can be indicative of the presence of a particular antigen (*e.g.*, protein) in the sample. Here, a discriminating molecule or fragment is a molecule or fragment that, when detected, indicates presence or abundance of an aboveidentified compound. A biomarker can, for example, be isolated from a sample, directly measured in a sample, or detected in or determined to be in a sample. A biomarker can, for example, be functional, partially functional, or non-functional. In specific embodiments, the "biomarkers" include "immune system biomarkers", which are described in more detail below.

The term "biomarker value" refers to a value measured or derived for at least one corresponding biomarker of a subject and which is typically at least partially indicative of an abundance or concentration of a biomarker in a sample taken from the subject. Thus, biomarker values could be measured biomarker values, which are values of biomarkers measured for the subject, or alternatively could be derived biomarker values, which are values that have been derived from one or more measured biomarker values, for example by applying a function to the one or more measured biomarker values. Biomarker values can be of any appropriate form depending on the manner in which the values are determined. For example, the biomarker values could be determined using high-throughput technologies such as mass spectrometry, sequencing platforms, array and hybridization platforms, immunoassays, flow cytometry, or any combination of such technologies and in one preferred example, the biomarker values relate to a level of activity or abundance of an expression product or other measurable molecule, quantified using a technique such as polymerase chain reaction (PCR), sequencing or the like. In this case, the biomarker values can be in the form of amplification amounts, or cycle times, which are a logarithmic representation of the concentration of the biomarker within a sample, as will be appreciated by persons skilled in the art and as will be described in more detail below.

As used herein, the term "biomarker profile" refers to a plurality of one or more types of biomarkers (*e.g.*, an mRNA molecule, a cDNA molecule and/or a protein, *etc.),* or an indication thereof, together with a feature, such as a measurable aspect (*e.g.*, biomarker value) of the biomarker(s). A biomarker profile may comprise at least two such biomarkers or indications thereof, where the biomarkers can be in the same or different classes, such as, for example, a nucleic acid and a polypeptide. Thus, a biomarker profile may comprise at least 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, or 100 or more biomarkers or indications thereof. In some embodiments, a biomarker profile comprises hundreds, or even thousands, of biomarkers or indications thereof. A biomarker profile can further comprise one or more controls or internal standards. In certain embodiments, the biomarker profile comprises at least one biomarker, or indication thereof, that serves as an internal standard. In other embodiments, a biomarker profile comprises an indication of one or more types of biomarkers. The term "indication" as used herein in this context merely refers to a situation where the biomarker profile contains symbols, data, abbreviations or other similar indicia for a biomarker, rather than the biomarker molecular entity itself. The term "biomarker profile" is also used herein to refer to a combination of at least two biomarker values, wherein individual biomarker values correspond to values of biomarkers that can be measured or derived from one or more subjects, which combination is characteristic of a discrete condition or not, stage of condition or not, subtype of condition or not or a prognosis for a discrete condition or not, stage of condition or not, subtype of condition or not. The term "profile biomarkers" is used to refer to a subset of the biomarkers that have been identified for use in a biomarker profile that can be used in performing a clinical assessment, such as to rule in or rule out a specific condition, different stages or severity of conditions, subtypes of different conditions or different prognoses. The number of profile biomarkers will vary, but is typically of the order of 10 or less.

The terms "complementary" and "complementarity" refer to polynucleotides *(i.e.,* a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands.

Throughout this specification, unless the context requires otherwise, the words "comprise," "comprises" and "comprising" will be understood to imply the inclusion of a stated step or element or group of steps or elements but not the exclusion of any other step or element or group of steps or elements. Thus, use of the term "comprising" and the like indicates that the listed elements are required or mandatory, but that other elements are optional and may or may not be present. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

The term "correlating" refers to determining a relationship between one type of data with another or with a state.

As used herein, the terms "detectably distinct" and "detectably different" are used interchangeably herein to refer to a signal that is distinguishable or separable by a physical property either by observation or by instrumentation. For example, a fluorophore is readily distinguishable either by spectral characteristics or by fluorescence intensity, lifetime, polarization or photo-bleaching rate from another fluorophore in a sample, as well as from additional materials that are optionally present. In certain embodiments, the terms "detectably distinct" and "detectably different" refer to a set of labels (such as dyes, suitably organic dyes) that can be detected and distinguished simultaneously.

As used herein, the terms "diagnosis", "diagnosing" and the like are used interchangeably herein to encompass determining the likelihood that a subject has or a condition, or not, or will develop a condition, or not, or the existence or nature of a condition in a subject. These terms also encompass determining the severity of disease or episode of disease, as well as in the context of rational therapy, in which the diagnosis guides therapy, including initial selection of therapy, modification of therapy (e.g., adjustment of dose or dosage regimen), and the like. By "likelihood" is meant a measure of whether a subject with particular measured or derived biomarker values actually has a condition, or not, based on a given mathematical model. An increased likelihood for example may be relative or absolute and may be expressed qualitatively or quantitatively. For instance, a decreased likelihood may be determined simply by determining the subject's measured or derived biomarker values for at least two RO BaSIRS biomarkers and placing the subject in an "decreased likelihood" category, based upon previous population studies. The term "likelihood" is also used interchangeably herein with the term "probability". The term "risk" relates to the possibility or probability of a particular event occurring at some point in the future. "Risk stratification" refers to an arraying of known clinical risk factors to allow physicians to classify patients into a low, moderate, high or highest risk of having, or developing, a particular disease or condition.

As used herein, "emergency" refers to any location, including an emergency care environment, where subjects feeling unwell or subjects looking for an evaluation of their individual risk of developing certain diseases present, in order to consult a person having a medical background, preferably a physician, to obtain an analysis of their physiological status and/or the cause underlying their discomfort. Typical examples are emergency departments (ED) or emergency rooms (ER) in hospitals, ambulances, medical doctors' practices or doctors' offices and other institutions suitable for diagnosis and/or treatment of subjects.

"Fluorophore" as used herein to refer to a moiety that absorbs light energy at a defined excitation wavelength and emits light energy at a different defined wavelength. Examples of fluorescence labels include, but are not limited to: Alexa Fluor dyes (Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), AMCA, AMCA-S, BODIPY dyes (BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), Carboxyrhodamine 6G, carboxy-X-rhodamine (ROX), Cascade Blue, Cascade Yellow, Cyanine dyes (Cy3, Cy5, Cy3.5, Cy5.5), Dansyl, Dapoxyl, Dialkylaminocoumarin, 4',5'-Dichloro-2',7'-dimethoxy-fluorescein, DM-NERF, Eosin, Erythrosin, Fluorescein, FAM, Hydroxycoumarin, IRDyes (IRD40, IRD 700, IRD 800), JOE, Lissamine rhodamine B, Marina Blue, Methoxycoumarin, Naphthofluorescein, Oregon Green 488, Oregon Green 500, Oregon Green 514, Pacific Blue, PyMPO, Pyrene, Rhodamine 6G, Rhodamine Green, Rhodamine Red, Rhodol Green, 2',4',5',7'-Tetra-bromosulfone-fluorescein, Tetramethyl-rhodamine (TMR), Carboxytetramethylrhodamine (TAMRA), Texas Red and Texas Red-X.

The term "gene", as used herein, refers to a stretch of nucleic acid that codes for a polypeptide or for an RNA chain that has a function. While it is the exon region of a gene that is transcribed to form mRNA, the term "gene" also includes regulatory regions such as promoters and enhancers that govern expression of the exon region.

The term "high-density array" refers to a substrate or collection of substrates or surfaces bearing a plurality of array elements (*e.g.*, discrete regions having particular moieties, *e.g.,* proteins (*e.g.*, antibodies), nucleic acids (*e.g.*, oligonucleotide probes), *etc.,* immobilized thereto), where the array elements are present at a density of about 100 elements/ $cm^2$ or more, about 1,000 elements/ $cm^2$ or more, about 10,000 elements/ $cm^2$ or more, or about 100,000 elements/ $cm^2$ or more. In specific embodiments, a "high-density array" is one that comprises a plurality of array elements for detecting about 100 or more different biomarkers, about 1,000 or more different biomarkers, about 10,000 or more different biomarkers, or about 100,000 or more different biomarkers. In representative example of these embodiments, a "high-density array" is one that comprises a plurality of array elements for detecting biomarkers of about 100 or more different genes, of about 1,000 or more different genes, of about 10,000 or more different genes, or of about 100,000 or more different genes. Generally, the elements of a high-density array are not labeled. The term "low-density array" refers to a substrate or collection of substrates or surfaces bearing a plurality of array elements (*e.g.*, discrete regions having particular moieties, *e.g.*, proteins (*e.g.*, antibodies), nucleic acids (*e.g.*, oligonucleotide probes), *etc.,* immobilized thereto), where the array elements are present at a density of about 100 elements/ $cm^2$ or less, about 50 elements/ $cm^2$ or less, about 20 elements/ $cm^2$ or less, or about 10 elements/ $cm^2$ or less. In specific embodiments, a "low-density array" is one that comprises a plurality of array elements for detecting about 100 or less different biomarkers, about 50 or less different biomarkers, about 20 or less different biomarkers, or about 10 or less different biomarkers. In representative example of these embodiments, a "low-density array" is one that comprises a plurality of array elements for detecting biomarkers of about 100 or less different genes, of about 50 or less different genes, of about 20 or less different genes, or of about 10 or less different genes. Generally, the elements of a low-density array are not labeled. In specific embodiments, the a "high-density array" or "low-density array" is a microarray.

The term "indicator" as used herein refers to a result or representation of a result, including any information, number, ratio, signal, sign, mark, or note by which a skilled artisan can estimate and/or determine a likelihood or risk of whether or not a subject is suffering from a given disease or condition. In the case of the present invention, the "indicator" may optionally be used together with other clinical characteristics, to arrive at a diagnosis (that is, the occurrence or nonoccurrence) of an absence of BaSIRS or a prognosis for a non-BaSIRS condition in a subject. That such an indicator is "determined" is not meant to imply that the indicator is 100% accurate. The skilled clinician may use the indicator together with other clinical indicia to arrive at a diagnosis.

The term "immobilized" means that a molecular species of interest is fixed to a solid support, suitably by covalent linkage. This covalent linkage can be achieved by different means depending on the molecular nature of the molecular species. Moreover, the molecular species may be also fixed on the solid support by electrostatic forces, hydrophobic or hydrophilic interactions or Van-der-Waals forces. The above described physico-chemical interactions typically occur in interactions between molecules. In particular embodiments, all that is required is that the molecules (*e.g.*, nucleic acids or polypeptides) remain immobilized or attached to a support under conditions in which it is intended to use the support, for example in applications requiring nucleic acid amplification and/or sequencing or in in antibody-binding assays. For example, oligonucleotides or primers are immobilized such that a 3' end is available for enzymatic extension and/or at least a portion of the sequence is capable of hybridizing to a complementary sequence. In some embodiments, immobilization can occur via hybridization to a surface attached primer, in which case the immobilized primer or oligonucleotide may be in the 3'-5' orientation. In other embodiments, immobilization can occur by means other than base-pairing hybridization, such as the covalent attachment.

The term "immune system", as used herein, refers to cells, molecular components and mechanisms, including antigen-specific and non-specific categories of the adaptive and innate immune systems, respectively, that provide a defense against damage and insults resulting from a viral infection. The term "innate immune system" refers to a host's non-specific reaction to insult to include antigen-nonspecific defense cells, molecular components and mechanisms that come into action immediately or within several hours after exposure to almost any insult or antigen. Elements of the innate immunity include for example phagocytic cells (monocytes, macrophages, dendritic cells, polymorphonuclear leukocytes such as neutrophils, reticuloendothelial cells such as Küpffer cells, and microglia), cells that release inflammatory mediators (basophils, mast cells and eosinophils), natural killer cells (NK cells) and physical barriers and molecules such as keratin, mucous, secretions, complement proteins, immunoglobulin M (IgM), acute phase proteins, fibrinogen and molecules of the clotting cascade, and cytokines. Effector compounds of the innate immune system include chemicals such as lysozymes, IgM, mucous and chemoattractants (*e.g.*, cytokines or histamine), complement and clotting proteins. The term "adaptive immune system" refers to antigen-specific cells, molecular components and mechanisms that emerge over several days, and react with and remove a specific antigen. The adaptive immune system develops throughout a host's lifetime. The adaptive immune system is based on leukocytes, and is divided into two major sections: the humoral immune system, which acts mainly *via* immunoglobulins produced by B cells, and the cell-mediated immune system, which functions mainly *via* T cells.

Reference herein to "immuno-interactive" includes reference to any interaction, reaction, or other form of association between molecules and in particular where one of the molecules is, or mimics, a component of the immune system.

As used herein, the term "label" and grammatical equivalents thereof, refer to any atom or molecule that can be used to provide a detectable and/or quantifiable signal. In particular, the label can be attached, directly or indirectly, to a nucleic acid or protein. Suitable labels that can be attached include, but are not limited to, radioisotopes, fluorophores, quenchers, chromophores, mass labels, electron dense particles, magnetic particles, spin labels, molecules that emit chemiluminescence, electrochemically active molecules, enzymes, cofactors, and enzyme substrates. A label can include an atom or molecule capable of producing a visually detectable signal when reacted with an enzyme. In some embodiments, the label is a "direct" label which is capable of spontaneously producing a detectible signal without the addition of ancillary reagents and is detected by visual means without the aid of instruments. For example, colloidal gold particles can be used as the label. Many labels are well known to those skilled in the art. In specific embodiments, the label is other than a naturally-occurring nucleoside. The term "label" also refers to an agent that has been artificially added, linked or attached via chemical manipulation to a molecule.

The term "microarray" refers to an arrangement of hybridizable array elements, e.g., probes (including primers), ligands, biomarker nucleic acid sequence or protein sequences on a substrate.

The term "nucleic acid" or "polynucleotide" as used herein includes RNA, mRNA, miRNA, cRNA, cDNA mtDNA, or DNA. The term typically refers to a polymeric form of nucleotides of at least 10 bases in length, either ribonucleotides or deoxynucleotides or a modified form of either type of nucleotide. The term includes single and double stranded forms of DNA or RNA.

By "obtained" is meant to come into possession. Samples so obtained include, for example, nucleic acid extracts or polypeptide extracts isolated or derived from a particular source. For instance, the extract may be isolated directly from a biological fluid or tissue of a subject.

"Protein", "polypeptide" and "peptide" are used interchangeably herein to refer to a polymer of amino acid residues and to variants and synthetic analogues of the same.

By "primer" is meant an oligonucleotide which, when paired with a strand of DNA, is capable of initiating the synthesis of a primer extension product in the presence of a suitable polymerizing agent. The primer is preferably single-stranded for maximum efficiency in amplification but can alternatively be double-stranded. A primer must be sufficiently long to prime the synthesis of extension products in the presence of the polymerization agent. The length of the primer depends on many factors, including application, temperature to be employed, template reaction conditions, other reagents, and source of primers. For example, depending on the complexity of the target sequence, the primer may be at least about 5, 6, 7, 8, 9, 10, 11, 12, 13, 14, 15, 16, 17, 18, 19, 20, 21, 22, 23, 24, 25, 26, 27, 28, 29, 30, 35, 40, 50, 75, 100, 150, 200, 300, 400, 500, to one base shorter in length than the template sequence at the 3' end of the primer to allow extension of a nucleic acid chain, though the 5' end of the primer may extend in length beyond the 3' end of the template sequence. In certain embodiments, primers can be large polynucleotides, such as from about 35 nucleotides to several kilobases or more. Primers can be selected to be "substantially complementary" to the sequence on the template to which it is designed to hybridize and serve as a site for the initiation of synthesis. By "substantially complementary", it is meant that the primer is sufficiently complementary to hybridize with a target polynucleotide. Desirably, the primer contains no mismatches with the template to which it is designed to hybridize but this is not essential. For example, non-complementary nucleotide residues can be attached to the 5' end of the primer, with the remainder of the primer sequence being complementary to the template. Alternatively, non-complementary nucleotide residues or a stretch of non-complementary nucleotide residues can be interspersed into a primer, provided that the primer sequence has sufficient complementarity with the sequence of the template to hybridize therewith and thereby form a template for synthesis of the extension product of the primer.

As used herein, the term "probe" refers to a molecule that binds to a specific sequence or sub-sequence or other moiety of another molecule. Unless otherwise indicated, the term "probe" typically refers to a nucleic acid probe that binds to another nucleic acid, also referred to herein as a "target polynucleotide", through complementary base pairing. Probes can bind target polynucleotides lacking complete sequence complementarity with the probe, depending on the stringency of the hybridization conditions. Probes can be labeled directly or indirectly and include primers within their scope.

The term "prognosis" as used herein refers to a prediction of the probable course and outcome of a clinical condition or disease. A prognosis is usually made by evaluating factors or symptoms of a disease that are indicative of a favorable or unfavorable course or outcome of the disease. The skilled artisan will understand that the term "prognosis" refers to an increased probability that a certain course or outcome will occur; that is, that a course or outcome is more likely to occur in a subject exhibiting a given condition, when compared to those individuals not exhibiting the condition.

As used herein, the term "quencher" includes any moiety that in close proximity to a donor fluorophore, takes up emission energy generated by the donor fluorophore and either dissipates the energy as heat or emits light of a longer wavelength than the emission wavelength of the donor fluorophore. In the latter case, the quencher is considered to be an acceptor fluorophore. The quenching moiety can act via proximal (*i.e.,* collisional) quenching or by Forster or fluorescence resonance energy transfer ("FRET"). Quenching by FRET is generally used in TaqMan® probes while proximal quenching is used in molecular beacon and Scorpion® type probes. Suitable quenchers are selected based on the fluorescence spectrum of the particular fluorophore. Useful quenchers include, for example, the Black Hole™ quenchers BHQ-1, BHQ-2, and BHQ-3 (Biosearch Technologies, Inc.), and the ATTO-series of quenchers (ATTO 540Q, ATTO 580Q, and ATTO 612Q; Atto-Tec GmbH).

The term "rule-out" and its grammatical equivalents refer to a diagnostic test with high sensitivity that optionally coupled with a clinical assessment indicates a lower likelihood for BaSIRS. Accordingly, the term "ruling out" as used herein is meant that the subject is selected not to receive a BaSIRS treatment protocol or regimen.

The term "sample" as used herein includes any biological specimen that may be extracted, untreated, treated, diluted or concentrated from a subject. Samples may include, without limitation, biological fluids such as whole blood, serum, red blood cells, white blood cells, plasma, saliva, urine, stool (*i.e.*, feces), tears, sweat, sebum, nipple aspirate, ductal lavage, tumor exudates, synovial fluid, ascitic fluid, peritoneal fluid, amniotic fluid, cerebrospinal fluid, lymph, fine needle aspirate, amniotic fluid, any other bodily fluid, cell lysates, cellular secretion products, inflammation fluid, semen and vaginal secretions. Samples may include tissue samples and biopsies, tissue homogenates and the like. Advantageous samples may include ones comprising any one or more biomarkers as taught herein in detectable quantities. Suitably, the sample is readily obtainable by minimally invasive methods, allowing the removal or isolation of the sample from the subject. In certain embodiments, the sample contains blood, especially peripheral blood, or a fraction or extract thereof. Typically, the sample comprises blood cells such as mature, immature or developing leukocytes, including lymphocytes, polymorphonuclear leukocytes, neutrophils, monocytes, reticulocytes, basophils, coelomocytes, hemocytes, eosinophils, megakaryocytes, macrophages, dendritic cells natural killer cells, or fraction of such cells (*e.g.,* a nucleic acid or protein fraction). In specific embodiments, the sample comprises leukocytes including peripheral blood mononuclear cells (PBMC).

The term "solid support" as used herein refers to a solid inert surface or body to which a molecular species, such as a nucleic acid and polypeptides can be immobilized. Non-limiting examples of solid supports include glass surfaces, plastic surfaces, latex, dextran, polystyrene surfaces, polypropylene surfaces, polyacrylamide gels, gold surfaces, and silicon wafers. In some embodiments, the solid supports are in the form of membranes, chips or particles. For example, the solid support may be a glass surface (*e.g.*, a planar surface of a flow cell channel). In some embodiments, the solid support may comprise an inert substrate or matrix which has been "functionalized", such as by applying a layer or coating of an intermediate material comprising reactive groups which permit covalent attachment to molecules such as polynucleotides. By way of non-limiting example, such supports can include polyacrylamide hydrogels supported on an inert substrate such as glass. The molecules (*e.g.*, polynucleotides) can be directly covalently attached to the intermediate material (*e.g.*, a hydrogel) but the intermediate material can itself be non-covalently attached to the substrate or matrix (*e.g.*, a glass substrate). The support can include a plurality of particles or beads each having a different attached molecular species.

As used herein, the term SIRS ("systemic inflammatory response syndrome") refers to a clinical response arising from a non-specific insult with two or more of the following measureable clinical characteristics; a body temperature greater than 38° C or less than 36° C, a heart rate greater than 90 beats per minute, a respiratory rate greater than 20 per minute, a white blood cell count (total leukocytes) greater than 12,000 per $mm^3$ or less than 4,000 per $mm^3$, or a band neutrophil percentage greater than 10%. From an immunological perspective, it may be seen as representing a systemic response to insult (*e.g.*, major surgery) or systemic inflammation. As used herein, "BaSIRS" includes any one or more (*e.g.*, 1, 2, 3, 4, 5) of the clinical responses noted above but with underlying bacterial infection etiology. Confirmation of infection can be determined using any suitable procedure known in the art, illustrative examples of which include blood culture, nucleic acid detection (*e.g.*, PCR, mass spectroscopy, immunological detection (*e.g.*, ELISA), isolation of bacteria from infected cells, cell lysis and imaging techniques such as electron microscopy. From an immunological perspective, BaSIRS may be seen as a systemic response to bacterial infection, whether it is a local, peripheral or systemic infection.

The terms "subject", "individual" and "patient" are used interchangeably herein to refer to an animal subject, particularly a vertebrate subject, and even more particularly a mammalian subject. Suitable vertebrate animals that fall within the scope of the invention include, but are not restricted to, any member of the phylum Chordata, subphylum vertebrata including primates, rodents (*e.g.*, mice rats, guinea pigs), lagomorphs (*e.g.*, rabbits, hares), bovines (*e.g.*, cattle), ovines (*e.g.*, sheep), caprines (*e.g.*, goats), porcines (*e.g.*, pigs), equines (*e.g.*, horses), canines (*e.g.*, dogs), felines (*e.g.*, cats), avians (*e.g.*, chickens, turkeys, ducks, geese, companion birds such as canaries, budgerigars *etc.),* marine mammals (*e.g.*, dolphins, whales), reptiles (snakes, frogs, lizards, *etc.*), and fish. A preferred subject is a primate (*e.g.*, a human, ape, monkey, chimpanzee). The subject suitably has at least one (*e.g.*, 1, 2, 3, 4, 5 or more) clinical sign of SIRS.

As used herein, the term "treatment regimen" refers to prophylactic and/or therapeutic *(i.e., after onset of a specified condition)* treatments, unless the context specifically indicates otherwise. The term "treatment regimen" encompasses natural substances and pharmaceutical agents *(i.e., "drugs")* as well as any other treatment regimen including but not limited to dietary treatments, physical therapy or exercise regimens, surgical interventions, and combinations thereof.

It will be appreciated that the terms used herein and associated definitions are used for the purpose of explanation only and are not intended to be limiting.

## *2. Bacterial systemic inflammation biomarkers and their use for identifying subjects with and without BaSIRS*

The present invention concerns methods, apparatus, compositions and kits for identifying subjects without BaSIRS or for providing strong negative predictive value in patients presenting to emergency rooms suspected of having BaSIRS. In particular, RO BaSIRS biomarkers are disclosed for use in these modalities to assess the likelihood of the absence of BaSIRS in subjects, or for providing high negative predictive value for BaSIRS in subjects presenting to emergency with at least one clinical sign of SIRS. The methods, apparatus, compositions and kits of the invention are useful for exclusion of BaSIRS as a diagnosis, thus allowing better treatment interventions for subjects with symptoms of SIRS that do not have a bacterial infection.

The present inventors have determined that certain expression products are commonly, specifically and differentially expressed during systemic inflammations with a range of bacterial etiologies. The results presented herein provide clear evidence that a unique biologicallyrelevant biomarker profile can exclude BaSIRS with a NPV greater than 95% in emergency room patients. This rule-out "bacterial" systemic inflammation biomarker profile was validated in an independently derived external dataset consisting of subjects presenting to emergency with fever (see Figure 5), and subsequently produced an AUC of 0.903 between infection positive and control patients (infection negative or viral positive). Overall, these findings provide compelling evidence that the expression products disclosed herein can function as biomarkers for excluding BaSIRS and may potentially serve as a useful diagnostic for triaging treatment decisions for SIRS-affected subjects. In this regard, it is proposed that the methods, apparatus, compositions and kits disclosed herein that are based on these biomarkers may serve in the point-of-care diagnostics that allow for rapid and inexpensive screening for BaSIRS, which may result in significant cost savings to the medical system as subjects without BaSIRS can be either exposed, or not exposed, to appropriate management procedures and therapeutic agents, including antibiotics, that are suitable for treating a particular type of SIRS.

Thus, specific expression products are disclosed herein as RO BaSIRS biomarkers that provide a means for distinguishing BaSIRS from other SIRS conditions including ADaSIRS, CANaSIRS, TRAUMaSIRS, ANAPHYLaSIRS, SCHIZaSIRS and VaSIRS. Evaluation of these RO BaSIRS biomarkers through analysis of their levels in a subject or in a sample taken from a subject provides a measured or derived biomarker value for determining an indicator that can be used for assessing the absence of BaSIRS in a subject.

Accordingly, biomarker values can be measured derived biomarker values, which are values that have been derived from one or more measured biomarker values, for example by applying a function to the one or more measured biomarker values. As used herein, biomarkers to which a function has been applied are referred to as "derived markers".

The biomarker values may be determined in any one of a number of ways. An exemplary method of determining biomarker values is described by the present inventors in WO 2015/117204, which is incorporated herein by reference in its entirety. In one example, the process of determining biomarker values can include measuring the biomarker values, for example by performing tests on the subject or on sample(s) taken from the subject. More typically however, the step of determining the biomarker values includes having an electronic processing device receive or otherwise obtain biomarker values that have been previously measured or derived. This could include for example, retrieving the biomarker values from a data store such as a remote database, obtaining biomarker values that have been manually input, using an input device, or the like. The indicator is determined using a combination of the plurality of biomarker values, the indicator being at least partially indicative of the absence of BaSIRS. Assuming the method is performed using an electronic processing device, an indication of the indicator is optionally displayed or otherwise provided to the user. In this regard, the indication could be a graphical or alphanumeric representation of an indicator value. Alternatively however, the indication could be the result of a comparison of the indicator value to predefined thresholds or ranges, or alternatively could be an indication of the absence of a BaSIRS derived using the indicator.

In some embodiments, biomarker values are combined, for example by adding, multiplying, subtracting, or dividing biomarker values to determine an indicator value. This step is performed so that multiple biomarker values can be combined into a single indicator value, providing a more useful and straightforward mechanism for allowing the indicator to be interpreted and hence used in diagnosing the absence of BaSIRS in the subject.

In some embodiments in which a plurality of biomarkers and biomarker values are used, in order to ensure that an effective diagnosis or prognosis can be determined, at least two of the biomarkers have a mutual correlation in respect of absence of BaSIRS that lies within a mutual correlation range, the mutual correlation range being between ±0.9. This requirement means that the two biomarkers are not entirely correlated in respect of each other when considered in the context of the absence of BaSIRS being diagnosed or prognosed. In other words, at least two of the biomarkers in the combination respond differently as the condition changes, which adds significantly to their ability when combined to discriminate between at least two conditions, to diagnose the absence of BaSIRS.

Typically, the requirement that biomarkers have a low mutual correlation means that the biomarkers may relate to different biological attributes or domains such as, but not limited, to different molecular functions, different biological processes and different cellular components. Illustrative examples of molecular function include addition of, or removal of, one of more of the following moieties to, or from, a protein, polypeptide, peptide, nucleic acid (*e.g.*, DNA, RNA): linear, branched, saturated or unsaturated alkyl (*e.g.*, $C_1$-$C_{24}$ alkyl); phosphate; ubiquitin; acyl; fatty acid, lipid, phospholipid; nucleotide base; hydroxyl and the like. Molecular functions also include signaling pathways, including without limitation, receptor signaling pathways and nuclear signaling pathways. Non-limiting examples of molecular functions also include cleavage of a nucleic acid, peptide, polypeptide or protein at one or more sites; polymerization of a nucleic acid, peptide, polypeptide or protein; translocation through a cell membrane (*e.g.*, outer cell membrane; nuclear membrane); translocation into or out of a cell organelle (*e.g.*, Golgi apparatus, lysosome, endoplasmic reticulum, nucleus, mitochondria); receptor binding, receptor signaling, membrane channel binding, membrane channel influx or efflux; and the like.

Illustrative examples of biological processes include: stages of the cell cycle such as meiosis, mitosis, cell division, prophase, metaphase, anaphase, telophase and interphase, stages of cell differentiation; apoptosis; necrosis; chemotaxis; immune responses including adaptive and innate immune responses, pro-inflammatory immune responses, autoimmune responses, tolerogenic responses and the like. Other illustrative examples of biological processes include generating or breaking down adenosine triphosphate (ATP), saccharides, polysaccharides, fatty acids, lipids, phospholipids, sphingolipids, glycolipids, cholesterol, nucleotides, nucleic acids, membranes (*e.g.*, cell plasma membrane, nuclear membrane), amino acids, peptides, polypeptides, proteins and the like. Representative examples of cellular components include organelles, membranes, as for example noted above, and others.

It will be understood that the use of biomarkers that have different biological attributes or domains provides further information than if the biomarkers were related to the same or common biological attributes or domains. In this regard, it will be appreciated if the at least two biomarkers are highly correlated to each other, the use of both biomarkers would add little diagnostic/prognostic improvement compared to the use of a single one of the biomarkers. Accordingly, an indicator-determining method of the present invention in which a plurality of biomarkers and biomarker values are used preferably employ biomarkers that are not well correlated with each other, thereby ensuring that the inclusion of each biomarker in the method adds significantly to the discriminative ability of the indicator.

Despite this, in order to ensure that the indicator can accurately be used in performing the discrimination between at least two conditions (*e.g.*, BaSIRS and a SIRS other than BaSIRS), or the diagnosis of the absence of BaSIRS, the indicator has a performance value that is greater than or equal to a performance threshold. The performance threshold may be of any suitable form but is to be typically indicative of an explained variance of at least 0.3, or an equivalent value of another performance measure.

Suitably, a combination of biomarkers is employed, which biomarkers have a mutual correlation between ±0.9 and which combination provides an explained variance of at least 0.3. This typically allows an indicator to be defined that is suitable for ensuring that an accurate discrimination, diagnosis or prognosis can be obtained whilst minimizing the number of biomarkers that are required. Typically the mutual correlation range is one of ±0.8; ±0.7; ±0.6; ±0.5; ±0.4; ±0.3; ±0.2; and, ±0.1. Typically each RO BaSIRS biomarker has a condition correlation with the absence of BaSIRS that lies outside a condition correlation range, the condition correlation range being between ±0.3 and more typically ±0.9; ±0.8; ±0.7; ±0.6; ±0.5; and, ±0.4. Typically the performance threshold is indicative of an explained variance of at least one of 0.4; 0.5; 0.6; 0.7; 0.8; and 0.9.

It will be understood that in this context, the biomarkers used within the above-described method can define a biomarker profile indicative of the likelihood of an absence of BaSIRS or for ruling out BaSIRS, which includes a minimal number of biomarkers, whilst maintaining sufficient performance to allow the biomarker profile to be used in making a clinically relevant diagnosis, prognosis, or differentiation. Minimizing the number of biomarkers used minimizes the costs associated with performing diagnostic or prognostic tests and in the case of nucleic acid expression products, allows the test to be performed utilizing relatively straightforward techniques such as nucleic acid array, and PCR processes, or the like, allowing the test to be performed rapidly in a clinical environment.

Furthermore, producing a single indicator value allows the results of the test to be easily interpreted by a clinician or other medical practitioner, so that test can be used for reliable diagnosis in a clinical environment.

Processes for generating suitable biomarker profiles are described for example in WO 2015/117204, which uses the term "biomarker signature" in place of "biomarker profile" as defined herein. It will be understood, therefore, that terms "biomarker profile" and "biomarker signature" are equivalent in scope. The biomarker profile-generating processes disclosed in WO 2015/117204 provide mechanisms for selecting a combination of biomarkers, and more typically derived biomarkers, that can be used to form a biomarker profile, which in turn can be used in diagnosing the absence of BaSIRS. In this regard, the biomarker profile defines the biomarkers that should be measured (i.e., the profile biomarkers), how derived biomarker values should be determined for measured biomarker values, and then how biomarker values should be subsequently combined to generate an indicator value. The biomarker profile can also specify defined indicator value ranges that indicate a particular absence of BaSIRS.

Using the above-described methods a number of biomarkers have been identified that are particularly useful for assessing a likelihood that a subject has an absence of BaSIRS and for ruling out the presence of BaSIRS in a subject. These biomarkers are referred to herein as "RO BaSIRS biomarkers". As used herein, the term "RO BaSIRS biomarker" refers to a biomarker of the host, generally a biomarker of the host's immune system, which is altered, or whose level of expression is altered, as part of an inflammatory response to damage or insult resulting from a SIRS other than BaSIRS. The RO BaSIRS biomarkers are suitably expression products of genes (also referred to interchangeably herein as "RO BaSIRS biomarker genes"), including polynucleotide and polypeptide expression products. As used herein, polynucleotide expression products of RO BaSIRS biomarker genes are referred to herein as "RO BaSIRS biomarker polynucleotides." Polypeptide expression products of the RO BaSIRS biomarker genes are referred to herein as "RO BaSIRS biomarker polypeptides."

RO BaSIRS biomarkers are suitably selected from expression products of any one or more of the following RO BaSIRS genes: *ADAM19, ADD1, ADGRE1, AIF1, AKAP7, AKT1, AKTIP, ALDOA, AMD1, ARL2BP, ATG9A, ATP13A3, ATP6VOA1, ATP8B4, BRD7, BTG2, C21orf59, C6orf48, CCND2, CD44, CD59, CDC14A, CERK, CHPT1, CLEC4E, CLU, CNBP, COMMD4, COQ10B, COX5B, CPVL, CTDSP2, CTSA, CTSC, CTSH, CYBB, CYP20A1, DERA, DHX16, DIAPH2, DLST, EIF4A2, EIF4E2, EMP3, ENO1, FBX07, FCER1G, FGL2, FLVCR2, FTL, FURIN, FUT8, FXR1, GAPDH, GAS7, GBP2, GIMAP4, GLOD4, GNS, GRAP2, GSTO1, HEBP1, HIST1H2BM, HIST1H3C, HIST1H4L, HLA-DPA1, HMG20B, HMGN4, HOXB6, HSPA4, ID3, IFIT1, IFNGR2, IL7R, IMP3, IMPDH1, INPP1, ISG20, ITGAX, ITGB1, KATNA1, KLF2, KLRF1, LAMP1, LFNG, LHFPL2, LILRB3, LTA4H, LTF, MAP4K2, MAPK14, MAPK8IP3, MCTP1, MEGF9, METTL9, MFSD10, MICAL1, MMP8, MNT, MRPS18B, MUT, MX1, MYL9, MYOM2, NAGK, NMI, NUPL2, OBFC1, OSBPL9, PAFAH2, PARL, PDCD5, PDGFC, PHB, PHF3, PLAC8, PLEKHG3, PLEKHM2, POLR2C, PPP1CA, PPP1CB, PPP1R11, PROS1, PRPF40A, PRRG4, PSMB4, PSTPIP2, PTPN2, PUS3, R11FIP1, RAB11FIP3, RAB9A, RANBP10,* RASGRP2, *RASGRP3, RASSF7, RDX, RNASE6, RNF34, RPA2, RPS6KB2, RPS8, S100A12, S100P, SASH3, SBF1, SDF2L1, SDHC, SERTAD2, SH3BGRL, SH3GLB2, SLAMF7, SLC11A2, SLC12A9, SLC25A37, SLC2A3, SLC39A8, SLC9A3R1, SNAPC1, SORT1, SSBP2, ST3GAL5, ST3GAL6, STK38, SYNE2, TAX1BP1, TIMP1, TINF2, TLR5, TMEM106C, TMEM80, TOB1, TPP2, TRAF3IP2, USP3, VAV1, WDR33, YPEL5,* and *ZBTB17.* Non-limiting examples of nucleotide sequences for these RO BaSIRS biomarkers are listed in SEQ ID NOs: 1-179. Non-limiting examples of amino acid sequences for these RO BaSIRS biomarkers are listed in SEQ ID NOs: 180-358.

The present inventors have determined that certain RO BaSIRS biomarkers have strong diagnostic performance when combined with one or more other RO BaSIRS biomarkers. In advantageous embodiments, pairs of RO BaSIRS biomarkers have been identified that can be used to determine the indicator. Accordingly, in representative examples of this type, an indicator is determined that correlates to a ratio of RO BaSIRS biomarkers, which can be used in assessing a likelihood of a subject having an absence of RO BaSIRS, and for ruling out the presence of BaSIRS in the subject.

In these examples, the indicator-determining methods suitably include determining a pair of biomarker values, wherein each biomarker value is a value measured or derived for at least one corresponding RO BaSIRS biomarker of the subject and is at least partially indicative of a concentration of the RO BaSIRS biomarker in a sample taken from the subject. The biomarker values are typically used to determine a derived biomarker value using the pair of biomarker values, wherein the derived biomarker value is indicative of a ratio of concentrations of the pair of RO BaSIRS biomarkers. Thus, if the biomarker values denote the concentrations of the RO BaSIRS biomarkers, then the derived biomarker value will be based on a ratio of the biomarker values. However, if the biomarker values are related to the concentrations of the biomarkers, for example if they are logarithmically related by virtue of the biomarker values being based on PCR cycle times, or the like, then the biomarker values may be combined in some other manner, such as by subtracting the cycle times to determine a derived biomarker value indicative of a ratio of the concentrations of the RO BaSIRS biomarkers.

The derived biomarker value is then used to determine the indicator, either by using the derived biomarker value as an indicator value, or by performing additional processing, such as comparing the derived biomarker value to a reference or the like, as will be described in more detail below.

In some embodiments in which pairs of RO BaSIRS biomarkers are used to determine a derived biomarker value, one biomarker of a biomarker pair is selected from Group A RO BaSIRS biomarkers and the other is selected from Group B RO BaSIRS biomarkers, wherein an individual Group A RO BaSIRS biomarker is an expression product of a gene selected from the group consisting of: *DIAPH2, CYBB, SLC39A8, PRPF40A, MUT, NMI, PUS3, MNT, SLC11A2, FXR1, SNAPC1, PRRG4, SLAMF7, MAPK8IP3, GBP2, PPP1CB, TMEM80, HIST1H2BM, NAGK, HIST1H4L* and wherein an individual Group B RO BaSIRS biomarker is an expression product of a gene selected from the group consisting of: *SERTAD2, PHF3, BRD7, TOB1, MAP4K2, WDR33, BTG2, AMD1, RNASE6, RAB11FIP1, ADD1, HMG20B.*

In other embodiments in which pairs of RO BaSIRS biomarkers are used to determine a derived biomarker value, one biomarker of a biomarker pair is selected from Group C RO BaSIRS biomarkers and the other is selected from Group D RO BaSIRS biomarkers, wherein an individual Group C RO BaSIRS biomarker is an expression product of a gene selected from the group consisting of: *PARL, AIF1, PTPN2, COX5B, PSMB4, EIF4E2, RDX, DERA, CTSH, HSPA4, VAV1, PPP1CA, CPVL, PDCD5,* and wherein an individual Group D RO BaSIRS biomarker is an expression product of a gene selected from the group consisting of: *PAFAH2, IMP3, GLOD4, IL7R, ID3, KLRF1, SBF1, CCND2, LFNG, MRPS18B, HLA-DPA1, SLC9A3R1, HMGN4, C6orf48, ARL2BP, CDC14A, RPA2, ST3GAL5, EIF4A2, CERK, RASSF7, PHB, TRAF3IP2, KLF2, RAB11FIP3, C21orf59, SSBP2, GIMAP4, CYP20A1, RASGRP2, AKT1, HCP5, TPP2, SYNE2, FUT8, NUPL2, MYOM2, RPS8, RNF34, DLST, CTDSP2, EMP3, PLEKHG3, DHX16, RASGRP3, COMMD4, ISG20, POLR2C, SH3GLB2, SASH3, GRAP2, RPS6KB2, FGL2, AKAP7, SDF2L1, FBX07, MX1, IFIT1, TMEM106C, RANBP10.*

In other embodiments in which pairs of RO BaSIRS biomarkers are used to determine a derived biomarker value, one biomarker of a biomarker pair is selected from Group E RO BaSIRS biomarkers and the other is selected from Group F RO BaSIRS biomarkers, wherein an individual Group E RO BaSIRS biomarker is an expression product of a gene selected from the group consisting of: *SORT1, GAS7, FLVCR2, TLR5, FCER1G, SLC2A3, S100A12, PSTPIP2, GNS, METTL9, MMP8, MAPK14, CD59, CLEC4E, MICAL1, MCTP1, GAPDH, IMPDH1, ATP8B4, EMR1, SLC12A9, S100P, IFNGR2, PDGFC, CTSA, ALDOA, ITGAX, GSTO1, LHFPL2, LTF, SDHC, TIMP1, LTA4H, USP3, MEGF9, FURIN, ATP6VOA1, PROS1, ATG9A, PLAC8, LAMP1, COQ10B, ST3GAL6, CTSC, ENOI, OBFC1, TAX1BP1, MYL9, HIST1H3C, ZBTB17, CHPT1, SLC25A37, PLEKHM2, LILRB3, YPEL5, FTL, SH3BGRL, HOXB6, PPP1R11, CLU, HEBP1,* and wherein an individual Group F RO BaSIRS biomarker is an expression product of a gene selected from the group consisting of: *OSBPL9, CD44, AKTIP, ATP13A3, ADAM19, KATNA1, STK38, TINF2, RAB9A, INPP1, CNBP, ITGB1, MFSD10.*

In specific embodiments, the indicator-determining methods involve determining a first derived biomarker value using a first pair of biomarker values, the first derived biomarker value being indicative of a ratio of concentrations of first and second RO BaSIRS biomarkers, determining a second derived biomarker value using a second pair of biomarker values, the second derived biomarker value being indicative of a ratio of concentrations of third and fourth RO BaSIRS biomarkers, determining a third derived biomarker value using a third pair of biomarker values, the third derived biomarker value being indicative of a ratio of concentrations of fifth and sixth RO BaSIRS biomarkers and determining the indicator by combining the first, second and third derived biomarker values. Thus, in these embodiments, three pairs of derived biomarker values can be used, which can assist in increasing the ability of the indicator to reliably determine the likelihood of a subject having or not having BaSIRS.

The derived biomarker values could be combined using a combining function such as an additive model; a linear model; a support vector machine; a neural network model; a random forest model; a regression model; a genetic algorithm; an annealing algorithm; a weighted sum; a nearest neighbor model; and a probabilistic model. In some embodiments, biomarker values are measured or derived for a Group A RO BaSIRS biomarker and for a Group B RO BaSIRS biomarker, and the indicator is determined by combining the biomarker values. In some embodiments, biomarker values are measured or derived for a Group C RO BaSIRS biomarker and for a Group D RO BaSIRS biomarker, and the indicator is determined by combining the biomarker values. In some embodiments, biomarker values are measured or derived for a Group E RO BaSIRS biomarker and for a Group F RO BaSIRS biomarker, and the indicator is determined by combining the biomarker values. In still other embodiments, biomarker values are measured or derived for a Group A RO BaSIRS biomarker, for a Group B RO BaSIRS biomarker, for a Group C RO BaSIRS biomarker and for a Group D RO BaSIRS biomarker, and the indicator is determined by combining the biomarker values. In still other embodiments, biomarker values are measured or derived for a Group A RO BaSIRS biomarker, for a Group B RO BaSIRS biomarker, for a Group C RO BaSIRS biomarker, for a Group D RO BaSIRS biomarker, for a Group E RO BaSIRS biomarker and for a Group F RO BaSIRS biomarker and the indicator is determined by combining the biomarker values.

In some embodiments, the indicator is compared to an indicator reference, with a likelihood being determined in accordance with results of the comparison. The indicator reference may be derived from indicators determined for a number of individuals in a reference population. The reference population typically includes individuals having different characteristics, such as a plurality of individuals of different sexes; and/or ethnicities, with different groups being defined based on different characteristics, with the subject's indicator being compared to indicator references derived from individuals with similar characteristics. The reference population can also include a plurality of healthy individuals, a plurality of individuals suffering from BaSIRS, a plurality of individuals suffering from a SIRS other than BaSIRS (*e.g.*, ADaSIRS, CANaSIRS, TRAUMaSIRS, ANAPHYLaSIRS, SCHIZaSIRS and VaSIRS), a plurality of individuals showing clinical signs of BaSIRS, a plurality of individuals showing clinical signs of a SIRS other than BaSIRS (*e.g.*, ADaSIRS, CANaSIRS, TRAUMaSIRS, ANAPHYLaSIRS, SCHIZaSIRS and VaSIRS), and/or first and second groups of individuals, each group of individuals suffering from a respective diagnosed SIRS.

The indicator can also be used for determining a likelihood of the subject having a first or second condition, wherein the first condition is BaSIRS and the second condition is a healthy condition or a non-bacterial associated SIRS (*e.g.*, ADaSIRS, CANaSIRS, TRAUMaSIRS, ANAPHYLaSIRS, SCHIZaSIRS and VaSIRS); in other words to distinguish between these conditions. In this case, this would typically be achieved by comparing the indicator to first and second indicator references, the first and second indicator references being indicative of first and second conditions and determining the likelihood in accordance with the results of the comparison. In particular, this can include determining first and second indicator probabilities using the results of the comparisons and combining the first and second indicator probabilities, for example using a Bayes method, to determine a condition probability corresponding to the likelihood of the subject having one of the conditions. In this situation the first and second conditions could include BaSIRS and a SIRS condition other than BaSIRS, or BaSIRS and a healthy condition. In this case, the first and second indicator references are distributions of indicators determined for first and second groups of a reference population, the first and second group consisting of individuals diagnosed with the first or second condition respectively.

In specific embodiments, the indicator-determining methods of the present invention are performed using at least one electronic processing device, such as a suitably programmed computer system or the like. In this case, the electronic processing device typically obtains at least three pairs of measured biomarker values, either by receiving these from a measuring or other quantifying device, or by retrieving these from a database or the like. The processing device then determines a first derived biomarker value indicative of a ratio of concentrations of first and second immune system biomarkers, a second derived biomarker value indicative of a ratio of third and fourth immune system biomarkers, and a third derived biomarker value indicative of a ratio of fifth and sixth immune system biomarkers . The processing device then determines the indicator by combining the first, second and third derived biomarker values.

The processing device can then generate a representation of the indicator, for example by generating an alphanumeric indication of the indicator, a graphical indication of a comparison of the indicator to one or more indicator references or an alphanumeric indication of a likelihood of the subject having at least one medical condition.

The indicator-determining methods of the present invention typically include obtaining a sample from a subject, who typically has at least one clinical sign of SIRS, wherein the sample includes one or more RO BaSIRS biomarkers (*e.g.*, polynucleotide or polypeptide expression products of RO BaSIRS genes) and quantifying at least two (*e.g.*, 2, 3, 4, 5, 6, 7, 8, 9, 10 or more) of the RO BaSIRS biomarkers within the sample to determine biomarker values. This can be achieved using any suitable technique, and will depend on the nature of the RO BaSIRS biomarkers. Suitably, an individual measured or derived RO BaSIRS biomarker value corresponds to the level, abundance or amount of a respective RO BaSIRS biomarker or to a function that is applied to that level or amount. As used herein the terms "level", "abundance" and "amount" are used interchangeably herein to refer to a quantitative amount (*e.g.*, weight or moles), a semiquantitative amount, a relative amount (*e.g.*, weight % or mole % within class), a concentration, and the like. Thus, these terms encompass absolute or relative amounts or concentrations of RO BaSIRS biomarkers in a sample. For example, if the indicator in some embodiments of the indicator-determining method of the present invention, which uses a plurality of RO BaSIRS biomarkers, is based on a ratio of concentrations of the polynucleotide expression products, this process would typically include quantifying polynucleotide expression products by amplifying at least some polynucleotide expression products in the sample, determining an amplification amount representing a degree of amplification required to obtain a defined level of each of a pair of polynucleotide expression products and determining the indicator by determining a difference between the amplification amounts. In this regard, the amplification amount is generally a cycle time, a number of cycles, a cycle threshold and an amplification time. In this case, the method includes determining a first derived biomarker value by determining a difference between the amplification amounts of a first pair of polynucleotide expression products, determining a second derived biomarker value by determining a difference between the amplification amounts of a second pair of polynucleotide expression products, determining a third derived biomarker value by determining a difference between the amplification amounts of a third pair of polynucleotide expression products and determining the indicator by adding the first, second and third derived biomarker values.

In some embodiments, the likelihood that BaSIRS is absent in a subject is established by determining two or more RO BaSIRS biomarker values, wherein a RO BaSIRS biomarker value is indicative of a value measured or derived for RO BaSIRS biomarkers in a subject or in a sample taken from the subject. These biomarkers are referred to herein as "sample RO BaSIRS biomarkers". In accordance with the present invention, a sample RO BaSIRS biomarker corresponds to a reference RO BaSIRS biomarker (also referred to herein as a "corresponding RO BaSIRS biomarker"). By "corresponding RO BaSIRS biomarker" is meant a RO BaSIRS biomarker that is structurally and/or functionally similar to a reference RO BaSIRS biomarker as set forth for example in SEQ ID NOs: 1-179. Representative corresponding RO BaSIRS biomarkers include expression products of allelic variants (same locus), homologues (different locus), and orthologues (different organism) of reference RO BaSIRS biomarker genes. Nucleic acid variants of reference RO BaSIRS biomarker genes and encoded RO BaSIRS biomarker polynucleotide expression products can contain nucleotide substitutions, deletions, inversions and/or insertions. Variation can occur in either or both the coding and non-coding regions. The variations can produce both conservative and non-conservative amino acid substitutions (as compared in the encoded product). For nucleotide sequences, conservative variants include those sequences that, because of the degeneracy of the genetic code, encode the amino acid sequence of a reference RO BaSIRS polypeptide.

Generally, variants of a particular RO BaSIRS biomarker gene or polynucleotide will have at least about 40%, 45%, 50%, 51%, 52%, 53%, 54%, 55%, 56%, 57%, 58%, 59% 60%, 61%, 62%, 63%, 64%, 65%, 66%, 67%, 68%, 69% 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more sequence identity to that particular nucleotide sequence as determined by sequence alignment programs known in the art using default parameters. In some embodiments, the RO BaSIRS biomarker gene or polynucleotide displays at least about 40%, 45%, 50%, 51%, 52%, 53%, 54%, 55%, 56%, 57%, 58%, 59% 60%, 61%, 62%, 63%, 64%, 65%, 66%, 67%, 68%, 69% 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more sequence identity to a nucleotide sequence selected from any one of SEQ ID NO: 1-179.

Corresponding RO BaSIRS biomarkers also include amino acid sequences that display substantial sequence similarity or identity to the amino acid sequence of a reference RO BaSIRS biomarker polypeptide. In general, an amino acid sequence that corresponds to a reference amino acid sequence will display at least about 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 97, 88, 89, 90, 91, 92, 93, 94, 95, 96, 97, 98, 99% or even up to 100% sequence similarity or identity to a reference amino acid sequence selected from any one of SEQ ID NO: 180-358.

In some embodiments, calculations of sequence similarity or sequence identity between sequences are performed as follows:

To determine the percentage identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In some embodiments, the length of a reference sequence aligned for comparison purposes is at least 30%, usually at least 40%, more usually at least 50%, 60%, and even more usually at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide at the corresponding position in the second sequence, then the molecules are identical at that position. For amino acid sequence comparison, when a position in the first sequence is occupied by the same or similar amino acid residue (*i.e.,* conservative substitution) at the corresponding position in the second sequence, then the molecules are similar at that position.

The percentage identity between the two sequences is a function of the number of identical amino acid residues shared by the sequences at individual positions, taking into account the number of gaps, and the length of each gap, which need to be introduced for optimal alignment of the two sequences. By contrast, the percentage similarity between the two sequences is a function of the number of identical and similar amino acid residues shared by the sequences at individual positions, taking into account the number of gaps, and the length of each gap, which need to be introduced for optimal alignment of the two sequences.

The comparison of sequences and determination of percentage identity or percentage similarity between sequences can be accomplished using a mathematical algorithm. In certain embodiments, the percentage identity or similarity between amino acid sequences is determined using the Needleman and Wünsch, (1970, J. Mol. Biol. 48: 444-453) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In specific embodiments, the percent identity between nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. An non-limiting set of parameters (and the one that should be used unless otherwise specified) includes a Blossum 62 scoring matrix with a gap penalty of 12, a gap extend penalty of 4, and a frameshift gap penalty of 5.

In some embodiments, the percentage identity or similarity between amino acid or nucleotide sequences can be determined using the algorithm of E. Meyers and W. Miller (1989, Cabios, 4: 11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al., (1990, J Mol Biol., 215: 403-10). BLAST nucleotide searches can be performed with the NBLAST program, score = 100, wordlength = 12 to obtain nucleotide sequences homologous to 53010 nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al., (1997, Nucleic Acids Res, 25: 3389-3402). When utilizing BLAST and Gapped BLAST programs, the default parameters of the respective programs (*e.g.*, XBLAST and NBLAST) can be used.

Corresponding RO BaSIRS biomarker polynucleotides also include nucleic acid sequences that hybridize to reference RO BaSIRS biomarker polynucleotides, or to their complements, under stringency conditions described below. As used herein, the term "hybridizes under low stringency, medium stringency, high stringency, or very high stringency conditions" describes conditions for hybridization and washing. "Hybridization" is used herein to denote the pairing of complementary nucleotide sequences to produce a DNA-DNA hybrid or a DNA-RNA hybrid. Complementary base sequences are those sequences that are related by the base-pairing rules. In DNA, A pairs with T and C pairs with G. In RNA, U pairs with A and C pairs with G. In this regard, the terms "match" and "mismatch" as used herein refer to the hybridization potential of paired nucleotides in complementary nucleic acid strands. Matched nucleotides hybridize efficiently, such as the classical A-T and G-C base pair mentioned above. Mismatches are other combinations of nucleotides that do not hybridize efficiently.

Guidance for performing hybridization reactions can be found in Ausubel et a/., ("CURRENT PROTOCOLS IN MOLECULAR BIOLOGY", John Wiley & Sons Inc., 1994-1998), Sections 6.3.1-6.3.6. Aqueous and non-aqueous methods are described in that reference and either can be used. Reference herein to low stringency conditions include and encompass from at least about 1% v/v to at least about 15% v/v formamide and from at least about 1 M to at least about 2 M salt for hybridization at 42° C, and at least about 1 M to at least about 2 M salt for washing at 42° C. Low stringency conditions also may include 1% Bovine Serum Albumin (BSA), 1 mM EDTA, 0.5 M $NaHPO_4$ (pH 7.2), 7% SDS for hybridization at 65° C, and (i) 2 × SSC, 0.1% SDS; or (ii) 0.5% BSA, 1 mM EDTA, 40 mM $NaHPO_4$ (pH 7.2), 5% SDS for washing at room temperature. One embodiment of low stringency conditions includes hybridization in 6 × sodium chloride/sodium citrate (SSC) at about 45° C, followed by two washes in 0.2 × SSC, 0.1% SDS at least at 50° C (the temperature of the washes can be increased to 55° C for low stringency conditions). Medium stringency conditions include and encompass from at least about 16% v/v to at least about 30% v/v formamide and from at least about 0.5 M to at least about 0.9 M salt for hybridization at 42° C, and at least about 0.1 M to at least about 0.2 M salt for washing at 55° C. Medium stringency conditions also may include 1% Bovine Serum Albumin (BSA), 1 mM EDTA, 0.5 M $NaHPO_4$ (pH 7.2), 7% SDS for hybridization at 65° C, and (i) 2 × SSC, 0.1% SDS; or (ii) 0.5% BSA, 1 mM EDTA, 40 mM $NaHPO_4$ (pH 7.2), 5% SDS for washing at 60-65° C. One embodiment of medium stringency conditions includes hybridizing in 6 × SSC at about 45° C, followed by one or more washes in 0.2 × SSC, 0.1% SDS at 60° C. High stringency conditions include and encompass from at least about 31% v/v to at least about 50% v/v formamide and from about 0.01 M to about 0.15 M salt for hybridization at 42° C, and about 0.01 M to about 0.02 M salt for washing at 55° C. High stringency conditions also may include 1% BSA, 1 mM EDTA, 0.5 M $NaHPO_4$ (pH 7.2), 7% SDS for hybridization at 65° C, and (i) 0.2 × SSC, 0.1% SDS; or (ii) 0.5% BSA, 1 mM EDTA, 40 mM $NaHPO_4$ (pH 7.2), 1% SDS for washing at a temperature in excess of 65° C. One embodiment of high stringency conditions includes hybridizing in 6 × SSC at about 45° C, followed by one or more washes in 0.2 × SSC, 0.1% SDS at 65° C.

In certain embodiments, a corresponding RO BaSIRS biomarker polynucleotide is one that hybridizes to a disclosed nucleotide sequence under very high stringency conditions. One embodiment of very high stringency conditions includes hybridizing 0.5 M sodium phosphate, 7% SDS at 65° C, followed by one or more washes at 0.2 × SSC, 1% SDS at 65° C.

Other stringency conditions are well known in the art and a skilled addressee will recognize that various factors can be manipulated to optimize the specificity of the hybridization. Optimization of the stringency of the final washes can serve to ensure a high degree of hybridization. For detailed examples, see Ausubel *et al., supra* at pages 2.10.1 to 2.10.16 and Sambrook et al. (MOLECULAR CLONING. A LABORATORY MANUAL, Cold Spring Harbor Press, 1989) at sections 1.101 to 1.104.

Generally, a sample is processed prior to RO BaSIRS biomarker detection or quantification. For example, nucleic acid and/or proteins may be extracted, isolated, and/or purified from a sample prior to analysis. Various DNA, mRNA, and/or protein extraction techniques are well known to those skilled in the art. Processing may include centrifugation, ultracentrifugation, ethanol precipitation, filtration, fractionation, resuspension, dilution, concentration, *etc.* In some embodiments, methods and systems provide analysis (*e.g.*, quantification of RNA or protein biomarkers) from raw sample (*e.g.*, biological fluid such as blood, serum, *etc.)* without or with limited processing.

Methods may comprise steps of homogenizing a sample in a suitable buffer, removal of contaminants and/or assay inhibitors, adding a RO BaSIRS biomarker capture reagent (*e.g.*, a magnetic bead to which is linked an oligonucleotide complementary to a target RO BaSIRS biomarker polynucleotide), incubated under conditions that promote the association (*e.g.*, by hybridization) of the target biomarker with the capture reagent to produce a target biomarker:capture reagent complex, incubating the target biomarker:capture complex under target biomarker-release conditions. In some embodiments, multiple RO BaSIRS biomarkers are isolated in each round of isolation by adding multiple RO BaSIRS biomarker capture reagents (*e.g.*, specific to the desired biomarkers) to the solution. For example, multiple RO BaSIRS biomarker capture reagents, each comprising an oligonucleotide specific for a different target RO BaSIRS biomarker can be added to the sample for isolation of multiple RO BaSIRS biomarker. It is contemplated that the methods encompass multiple experimental designs that vary both in the number of capture steps and in the number of target RO BaSIRS biomarker captured in each capture step. In some embodiments, capture reagents are molecules, moieties, substances, or compositions that preferentially (*e.g.*, specifically and selectively) interact with a particular biomarker sought to be isolated, purified, detected, and/or quantified. Any capture reagent having desired binding affinity and/or specificity to the particular RO BaSIRS biomarker can be used in the present technology. For example, the capture reagent can be a macromolecule such as a peptide, a protein (*e.g.*, an antibody or receptor), an oligonucleotide, a nucleic acid, (*e.g.*,

nucleic acids capable of hybridizing with the RO BaSIRS biomarkers), vitamins, oligosaccharides, carbohydrates, lipids, or small molecules, or a complex thereof. As illustrative and non-limiting examples, an avidin target capture reagent may be used to isolate and purify targets comprising a biotin moiety, an antibody may be used to isolate and purify targets comprising the appropriate antigen or epitope, and an oligonucleotide may be used to isolate and purify a complementary oligonucleotide.

Any nucleic acids, including single-stranded and double-stranded nucleic acids, that are capable of binding, or specifically binding, to a target RO BaSIRS biomarker can be used as the capture reagent. Examples of such nucleic acids include DNA, RNA, aptamers, peptide nucleic acids, and other modifications to the sugar, phosphate, or nucleoside base. Thus, there are many strategies for capturing a target and accordingly many types of capture reagents are known to those in the art.

In addition, RO BaSIRS biomarker capture reagents may comprise a functionality to localize, concentrate, aggregate, *etc.* the capture reagent and thus provide a way to isolate and purify the target RO BaSIRS biomarker when captured (*e.g.*, bound, hybridized, *etc.)* to the capture reagent (*e.g.*, when a target:capture reagent complex is formed). For example, in some embodiments the portion of the capture reagent that interacts with the RO BaSIRS biomarker (*e.g.,* an oligonucleotide) is linked to a solid support (*e.g.*, a bead, surface, resin, column, and the like) that allows manipulation by the user on a macroscopic scale. Often, the solid support allows the use of a mechanical means to isolate and purify the target:capture reagent complex from a heterogeneous solution. For example, when linked to a bead, separation is achieved by removing the bead from the heterogeneous solution, *e.g.*, by physical movement. In embodiments in which the bead is magnetic or paramagnetic, a magnetic field is used to achieve physical separation of the capture reagent (and thus the target RO BaSIRS biomarker) from the heterogeneous solution.

The RO BaSIRS biomarkers may be quantified or detected using any suitable technique. In specific embodiments, the RO BaSIRS biomarkers are quantified using reagents that determine the level, abundance or amount of individual RO BaSIRS biomarkers. Non-limiting reagents of this type include reagents for use in nucleic acid- and protein-based assays.

In illustrative nucleic acid-based assays, nucleic acid is isolated from cells contained in the biological sample according to standard methodologies (Sambrook, *et al.,* 1989, *supra;* and Ausubel *et al.,* 1994, *supra*). The nucleic acid is typically fractionated (*e.g.*, poly A$^+$ RNA) or whole cell RNA. Where RNA is used as the subject of detection, it may be desired to convert the RNA to a complementary DNA. In some embodiments, the nucleic acid is amplified by a template-dependent nucleic acid amplification technique. A number of template dependent processes are available to amplify the RO BaSIRS biomarker sequences present in a given template sample. An exemplary nucleic acid amplification technique is PCR, which is described in detail in U.S. Pat. Nos. 4,683,195, 4,683,202 and 4,800,159, Ausubel *et al.* (*supra*)*,* and in Innis et al., ("PCR Protocols", Academic Press, Inc., San Diego Calif., 1990). Briefly, in PCR, two primer sequences are prepared that are complementary to regions on opposite complementary strands of the biomarker sequence. An excess of deoxynucleotide triphosphates are added to a reaction mixture along with a DNA polymerase, *e.g., Taq* polymerase. If a cognate RO BaSIRS biomarker sequence is present in a sample, the primers will bind to the biomarker and the polymerase will cause the primers to be extended along the biomarker sequence by adding on nucleotides. By raising and lowering the temperature of the reaction mixture, the extended primers will dissociate from the biomarker to form reaction products, excess primers will bind to the biomarker and to the reaction products and the process is repeated. A reverse transcriptase PCR amplification procedure may be performed in order to quantify the amount of mRNA amplified. Methods of reverse transcribing RNA into cDNA are well known and described in Sambrook *et al.,* 1989, *supra.* Alternative methods for reverse transcription utilize thermostable, RNA-dependent DNA polymerases. These methods are described in WO 90/07641. Polymerase chain reaction methodologies are well known in the art. In specific embodiments in which whole cell RNA is used, cDNA synthesis using whole cell RNA as a sample produces whole cell cDNA.

In certain advantageous embodiments, the template-dependent amplification involves quantification of transcripts in real-time. For example, RNA or DNA may be quantified using the Real-Time PCR (RT-PCR) technique (Higuchi, 1992, et al., Biotechnology 10: 413-417). By determining the concentration of the amplified products of the target DNA in PCR reactions that have completed the same number of cycles and are in their linear ranges, it is possible to determine the relative concentrations of the specific target sequence in the original DNA mixture. If the DNA mixtures are cDNAs synthesized from RNAs isolated from different tissues or cells, the relative abundance of the specific mRNA from which the target sequence was derived can be determined for the respective tissues or cells. This direct proportionality between the concentration of the PCR products and the relative mRNA abundance is only true in the linear range of the PCR reaction. The final concentration of the target DNA in the plateau portion of the curve is determined by the availability of reagents in the reaction mix and is independent of the original concentration of target DNA. In specific embodiments, multiplexed, tandem PCR (MT-PCR) is employed, which uses a two-step process for gene expression profiling from small quantities of RNA or DNA, as described for example in US Pat. Appl. Pub. No. 20070190540. In the first step, RNA is converted into cDNA and amplified using multiplexed gene specific primers. In the second step each individual gene is quantitated by RT-PCR. Real-time PCR is typically performed using any PCR instrumentation available in the art. Typically, instrumentation used in real-time PCR data collection and analysis comprises a thermal cycler, optics for fluorescence excitation and emission collection, and optionally a computer and data acquisition and analysis software.

In some embodiments of RT-PCR assays, a TaqMan® probe is used for quantitating nucleic acid. Such assays may use energy transfer ("ET"), such as fluorescence resonance energy transfer ("FRET"), to detect and quantitate the synthesized PCR product. Typically, the TaqMan® probe comprises a fluorescent label (e.g., a fluorescent dye) coupled to one end (e.g., the 5'-end) and a quencher molecule is coupled to the other end (e.g., the 3'-end), such that the fluorescent label and the quencher are in close proximity, allowing the quencher to suppress the fluorescence signal of the dye via FRET. When a polymerase replicates the chimeric amplicon template to which the fluorescent labeled probe is bound, the 5'-nuclease of the polymerase cleaves the probe, decoupling the fluorescent label and the quencher so that label signal (such as fluorescence) is detected. Signal (such as fluorescence) increases with each PCR cycle proportionally to the amount of probe that is cleaved.

TaqMan® probes typically comprise a region of contiguous nucleotides having a sequence that is identically present in or complementary to a region of a RO BaSIRS biomarker polynucleotide such that the probe is specifically hybridizable to the resulting PCR amplicon. In some embodiments, the probe comprises a region of at least 6 contiguous nucleotides having a sequence that is fully complementary to or identically present in a region of a target RO BaSIRS biomarker polynucleotide, such as comprising a region of at least 8 contiguous nucleotides, at least 10 contiguous nucleotides, at least 12 contiguous nucleotides, at least 14 contiguous nucleotides, or at least 16 contiguous nucleotides having a sequence that is complementary to or identically present in a region of a target RO BaSIRS biomarker polynucleotide to be detected and/or quantitated.

In addition to the TaqMan® assays, other real-time PCR chemistries useful for detecting PCR products in the methods presented herein include, but are not limited to, Molecular Beacons, Scorpion probes and intercalating dyes, such as SYBR Green, EvaGreen, thiazole orange, YO-PRO, TO-PRO, etc. For example, Molecular Beacons, like TaqMan® probes, use FRET to detect and quantitate a PCR product via a probe having a fluorescent label (e.g., a fluorescent dye) and a quencher attached at the ends of the probe. Unlike TaqMan® probes, however, Molecular Beacons remain intact during the PCR cycles. Molecular Beacon probes form a stem-loop structure when free in solution, thereby allowing the fluorescent label and quencher to be in close enough proximity to cause fluorescence quenching. When the Molecular Beacon hybridizes to a target, the stem-loop structure is abolished so that the fluorescent label and the quencher become separated in space and the fluorescent label fluoresces. Molecular Beacons are available, e.g., from Gene Link™ (see www.genelink.com/newsite/products/mbintro.asp).

In some embodiments, Scorpion probes can be used as both sequence-specific primers and for PCR product detection and quantitation. Like Molecular Beacons, Scorpion probes form a stem-loop structure when not hybridized to a target nucleic acid. However, unlike Molecular Beacons, a Scorpion probe achieves both sequence-specific priming and PCR product detection. A fluorescent label (e.g., a fluorescent dye molecule) is attached to the 5'-end of the Scorpion probe, and a quencher is attached to the 3'-end. The 3' portion of the probe is complementary to the extension product of the PCR primer, and this complementary portion is linked to the 5'-end of the probe by a non-amplifiable moiety. After the Scorpion primer is extended, the target-specific sequence of the probe binds to its complement within the extended amplicon, thus opening up the stem-loop structure and allowing the fluorescent label on the 5'-end to fluoresce and generate a signal. Scorpion probes are available from, e.g., Premier Biosoft International (see www.premierbiosoft.com/tech_notes/Scorpion.html).

In some embodiments, labels that can be used on the FRET probes include colorimetric and fluorescent dyes such as Alexa Fluor dyes, BODIPY dyes, such as BODIPY FL; Cascade Blue; Cascade Yellow; coumarin and its derivatives, such as 7-amino-4-methylcoumarin, aminocoumarin and hydroxycoumarin; cyanine dyes, such as Cy3 and Cy5; eosins and erythrosins; fluorescein and its derivatives, such as fluorescein isothiocyanate; macrocyclic chelates of lanthanide ions, such as Quantum Dye™; Marina Blue; Oregon Green; rhodamine dyes, such as rhodamine red, tetramethylrhodamine and rhodamine 6G; Texas Red; fluorescent energy transfer dyes, such as thiazole orange-ethidium heterodimer; and, TOTAB.

Specific examples of dyes include, but are not limited to, those identified above and the following: Alexa Fluor 350, Alexa Fluor 405, Alexa Fluor 430, Alexa Fluor 488, Alexa Fluor 500. Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, and, Alexa Fluor 750; amine-reactive BODIPY dyes, such as BODIPY 493/503, BODIPY 530/550, BODIPY 558/568, BODIPY 564/570, BODIPY 576/589, BODIPY 581/591, BODIPY 630/650, BODIPY 650/655, BODIPY FL, BODIPY R6G, BODIPY TMR, and, BODIPY-TR; Cy3, Cy5, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, 2',4',5',7'-Tetrabromosulfonefluorescein, and TET.

Examples of dye/quencher pairs (i.e., donor/acceptor pairs) include, but are not limited to, fluorescein/tetramethylrhodamine; IAEDANS/fluorescein; EDANS/dabcyl; fluorescein/fluorescein; BODIPY FL/BODIPY FL; fluorescein/QSY 7 or QSY 9 dyes. When the donor and acceptor are the same, FRET may be detected, in some embodiments, by fluorescence depolarization. Certain specific examples of dye/quencher pairs (i.e., donor/acceptor pairs) include, but are not limited to, Alexa Fluor 350/Alexa Fluor488; Alexa Fluor 488/Alexa Fluor 546; Alexa Fluor 488/Alexa Fluor 555; Alexa Fluor 488/Alexa Fluor 568; Alexa Fluor 488/Alexa Fluor 594; Alexa Fluor 488/Alexa Fluor 647; Alexa Fluor 546/Alexa Fluor 568; Alexa Fluor 546/Alexa Fluor 594; Alexa Fluor 546/Alexa Fluor 647; Alexa Fluor 555/Alexa Fluor 594; Alexa Fluor 555/Alexa Fluor 647; Alexa Fluor 568/Alexa Fluor 647; Alexa Fluor 594/Alexa Fluor 647; Alexa Fluor 350/QSY35; Alexa Fluor 350/dabcyl; Alexa Fluor 488/QSY 35; Alexa Fluor 488/dabcyl; Alexa Fluor 488/QSY 7 or QSY 9; Alexa Fluor 555/QSY 7 or QSY9; Alexa Fluor 568/QSY 7 or QSY 9; Alexa Fluor 568/QSY 21; Alexa Fluor 594/QSY 21; and Alexa Fluor 647/QSY 21. In some embodiments, the same quencher may be used for multiple dyes, for example, a broad spectrum quencher, such as an Iowa Black® quencher (Integrated DNA Technologies, Coralville, Iowa) or a Black Hole Quencher™ (BHQ™; Sigma-Aldrich, St. Louis, Mo.).

In some embodiments, for example, in a multiplex reaction in which two or more moieties (such as amplicons) are detected simultaneously, each probe comprises a detectably different dye such that the dyes may be distinguished when detected simultaneously in the same reaction. One skilled in the art can select a set of detectably different dyes for use in a multiplex reaction. In some embodiments, multiple target RO BaSIRS biomarker polynucleotides are detected and/or quantitated in a single multiplex reaction. In some embodiments, each probe that is targeted to a different RO BaSIRS biomarker polynucleotide is spectrally distinguishable when released from the probe. Thus, each target RO BaSIRS biomarker polynucleotide is detected by a unique fluorescence signal.

Specific examples of fluorescently labeled ribonucleotides useful in the preparation of real-time PCR probes for use in some embodiments of the methods described herein are available from Molecular Probes (Invitrogen), and these include, Alexa Fluor 488-5-UTP, Fluorescein-12-UTP, BODIPY FL-14-UTP, BODIPY TMR-14-UTP, Tetramethylrhodamine-6-UTP, Alexa Fluor 546-14-UTP, Texas Red-5-UTP, and BODIPY TR-14-UTP. Other fluorescent ribonucleotides are available from Amersham Biosciences (GE Healthcare), such as Cy3-UTP and Cy5-UTP.

Examples of fluorescently labeled deoxyribonucleotides useful in the preparation of real-time PCR probes for use in the methods described herein include Dinitrophenyl (DNP)-1'-dUTP, Cascade Blue-7-dUTP, Alexa Fluor 488-5-dUTP, Fluorescein-12-dUTP, Oregon Green 488-5-dUTP, BODIPY FL-14-dUTP, Rhodamine Green-5-dUTP, Alexa Fluor 532-5-dUTP, BODIPY TMR-14-dUTP, Tetramethylrhodamine-6-dUTP, Alexa Fluor 546-14-dUTP, Alexa Fluor 568-5-dUTP, Texas Red-12-dUTP, Texas Red-5-dUTP, BODIPY TR-14-dUTP, Alexa Fluor 594-5-dUTP, BODIPY 630/650-14-dUTP, BODIPY 650/665-14-dUTP; Alexa Fluor 488-7-OBEA-dCTP, Alexa Fluor 546-16-OBEA-dCTP, Alexa Fluor 594-7-OBEA-dCTP, Alexa Fluor 647-12-OBEA-dCTP. Fluorescently labeled nucleotides are commercially available and can be purchased from, *e.g.*, Invitrogen.

In certain embodiments, target nucleic acids are quantified using blotting techniques, which are well known to those of skill in the art. Southern blotting involves the use of DNA as a target, whereas Northern blotting involves the use of RNA as a target. Each provides different types of information, although cDNA blotting is analogous, in many aspects, to blotting or RNA species. Briefly, a probe is used to target a DNA or RNA species that has been immobilized on a suitable matrix, often a filter of nitrocellulose. The different species should be spatially separated to facilitate analysis. This often is accomplished by gel electrophoresis of nucleic acid species followed by "blotting" on to the filter. Subsequently, the blotted target is incubated with a probe (usually labeled) under conditions that promote denaturation and rehybridization. Because the probe is designed to base pair with the target, the probe will bind a portion of the target sequence under renaturing conditions. Unbound probe is then removed, and detection is accomplished as described above. Following detection/quantification, one may compare the results seen in a given subject with a control reaction or a statistically significant reference group or population of control subjects as defined herein. In this way, it is possible to correlate the amount of RO BaSIRS biomarker nucleic acid detected with the progression or severity of the disease.

Also contemplated are biochip-based technologies such as those described by Hacia et al. (1996, Nature Genetics 14: 441-447) and Shoemaker et al. (1996, Nature Genetics 14: 450-456). Briefly, these techniques involve quantitative methods for analyzing large numbers of genes rapidly and accurately. By tagging genes with oligonucleotides or using fixed nucleic acid probe arrays, one can employ biochip technology to segregate target molecules as high-density arrays and screen these molecules on the basis of hybridization. See also Pease et al. (1994, Proc. Natl. Acad. Sci. U.S.A. 91: 5022-5026); Fodor et al. (1991, Science 251: 767-773). Briefly, nucleic acid probes to RO BaSIRS biomarker polynucleotides are made and attached to biochips to be used in screening and diagnostic methods, as outlined herein. The nucleic acid probes attached to the biochip are designed to be substantially complementary to specific expressed RO BaSIRS biomarker nucleic acids, *i.e.,* the target sequence (either the target sequence of the sample or to other probe sequences, for example in sandwich assays), such that hybridization of the target sequence and the probes of the present invention occur. This complementarity need not be perfect; there may be any number of base pair mismatches, which will interfere with hybridization between the target sequence and the nucleic acid probes of the present invention. However, if the number of mismatches is so great that no hybridization can occur under even the least stringent of hybridization conditions, the sequence is not a complementary target sequence. In certain embodiments, more than one probe per sequence is used, with either overlapping probes or probes to different sections of the target being used. That is, two, three, four or more probes, with three being desirable, are used to build in a redundancy for a particular target. The probes can be overlapping (*i.e.* have some sequence in common), or separate.

In an illustrative biochip analysis, oligonucleotide probes on the biochip are exposed to or contacted with a nucleic acid sample suspected of containing one or more RO BaSIRS biomarker polynucleotides under conditions favoring specific hybridization. Sample extracts of DNA or RNA, either single or double-stranded, may be prepared from fluid suspensions of biological materials, or by grinding biological materials, or following a cell lysis step which includes, but is not limited to, lysis effected by treatment with SDS (or other detergents), osmotic shock, guanidinium isothiocyanate and lysozyme. Suitable DNA, which may be used in the method of the invention, includes cDNA. Such DNA may be prepared by any one of a number of commonly used protocols as for example described in Ausubel, *et al.,* 1994, *supra,* and Sambrook, *et al.,* 1989, *supra.*

Suitable RNA, which may be used in the method of the invention, includes messenger RNA, complementary RNA transcribed from DNA (cRNA) or genomic or subgenomic RNA. Such RNA may be prepared using standard protocols as for example described in the relevant sections of Ausubel, *et al.* 1994, *supra* and Sambrook, *et al.* 1989, *supra).*

cDNA may be fragmented, for example, by sonication or by treatment with restriction endonucleases. Suitably, cDNA is fragmented such that resultant DNA fragments are of a length greater than the length of the immobilized oligonucleotide probe(s) but small enough to allow rapid access thereto under suitable hybridization conditions. Alternatively, fragments of cDNA may be selected and amplified using a suitable nucleotide amplification technique, as described for example above, involving appropriate random or specific primers.

Usually the target RO BaSIRS biomarker polynucleotides are detectably labeled so that their hybridization to individual probes can be determined. The target polynucleotides are typically detectably labeled with a reporter molecule illustrative examples of which include chromogens, catalysts, enzymes, fluorochromes, chemiluminescent molecules, bioluminescent molecules, lanthanide ions (*e.g.*, $Eu^{34}$), a radioisotope and a direct visual label. In the case of a direct visual label, use may be made of a colloidal metallic or non-metallic particle, a dye particle, an enzyme or a substrate, an organic polymer, a latex particle, a liposome, or other vesicle containing a signal producing substance and the like. Illustrative labels of this type include large colloids, for example, metal colloids such as those from gold, selenium, silver, tin and titanium oxide. In some embodiments in which an enzyme is used as a direct visual label, biotinylated bases are incorporated into a target polynucleotide.

The hybrid-forming step can be performed under suitable conditions for hybridizing oligonucleotide probes to test nucleic acid including DNA or RNA. In this regard, reference may be made, for example, to NUCLEIC ACID HYBRIDIZATION, A PRACTICAL APPROACH (Homes and Higgins, eds.) (IRL press, Washington D.C., 1985). In general, whether hybridization takes place is influenced by the length of the oligonucleotide probe and the polynucleotide sequence under test, the pH, the temperature, the concentration of mono- and divalent cations, the proportion of G and C nucleotides in the hybrid-forming region, the viscosity of the medium and the possible presence of denaturants. Such variables also influence the time required for hybridization. The preferred conditions will therefore depend upon the particular application. Such empirical conditions, however, can be routinely determined without undue experimentation.

After the hybrid-forming step, the probes are washed to remove any unbound nucleic acid with a hybridization buffer. This washing step leaves only bound target polynucleotides. The probes are then examined to identify which probes have hybridized to a target polynucleotide.

The hybridization reactions are then detected to determine which of the probes has hybridized to a corresponding target sequence. Depending on the nature of the reporter molecule associated with a target polynucleotide, a signal may be instrumentally detected by irradiating a fluorescent label with light and detecting fluorescence in a fluorimeter; by providing for an enzyme system to produce a dye which could be detected using a spectrophotometer; or detection of a dye particle or a colored colloidal metallic or non-metallic particle using a reflectometer; in the case of using a radioactive label or chemiluminescent molecule employing a radiation counter or autoradiography. Accordingly, a detection means may be adapted to detect or scan light associated with the label which light may include fluorescent, luminescent, focused beam or laser light. In such a case, a charge couple device (CCD) or a photocell can be used to scan for emission of light from a probe:target polynucleotide hybrid from each location in the micro-array and record the data directly in a digital computer. In some cases, electronic detection of the signal may not be necessary. For example, with enzymatically generated color spots associated with nucleic acid array format, visual examination of the array will allow interpretation of the pattern on the array. In the case of a nucleic acid array, the detection means is suitably interfaced with pattern recognition software to convert the pattern of signals from the array into a plain language genetic profile. In certain embodiments, oligonucleotide probes specific for different RO BaSIRS biomarker polynucleotides are in the form of a nucleic acid array and detection of a signal generated from a reporter molecule on the array is performed using a 'chip reader'. A detection system that can be used by a 'chip reader' is described for example by Pirrung et al. (U.S. Patent No. 5,143,854). The chip reader will typically also incorporate some signal processing to determine whether the signal at a particular array position or feature is a true positive or maybe a spurious signal. Exemplary chip readers are described for example by Fodor et al. (U.S. Patent No., 5,925,525). Alternatively, when the array is made using a mixture of individually addressable kinds of labeled microbeads, the reaction may be detected using flow cytometry.

In certain embodiments, the RO BaSIRS biomarker is a target RNA (*e.g.*, mRNA) or a DNA copy of the target RNA whose level or abundance is measured using at least one nucleic acid probe that hybridizes under at least low, medium, or high stringency conditions to the target RNA or to the DNA copy, wherein the nucleic acid probe comprises at least 15 (*e.g.*, 15, 16, 17, 18, 19, 20, 21, 22, 23, 24, 25, 26, 27, 28, 29, 30, or more) contiguous nucleotides of RO BaSIRS biomarker polynucleotide. In some embodiments, the measured level or abundance of the target RNA or its DNA copy is normalized to the level or abundance of a reference RNA or a DNA copy of the reference RNA. Suitably, the nucleic acid probe is immobilized on a solid or semi-solid support. In illustrative examples of this type, the nucleic acid probe forms part of a spatial array of nucleic acid probes. In some embodiments, the level of nucleic acid probe that is bound to the target RNA or to the DNA copy is measured by hybridization (*e.g.*, using a nucleic acid array). In other embodiments, the level of nucleic acid probe that is bound to the target RNA or to the DNA copy is measured by nucleic acid amplification (*e.g.*, using a polymerase chain reaction (PCR)). In still other embodiments, the level of nucleic acid probe that is bound to the target RNA or to the DNA copy is measured by nuclease protection assay.

Sequencing technologies such as Sanger sequencing, pyrosequencing, sequencing by ligation, massively parallel sequencing, also called "Next-generation sequencing" (NGS), and other high-throughput sequencing approaches with or without sequence amplification of the target can also be used to detect or quantify the presence of RO BaSIRS biomarker polynucleotides in a sample. Sequence-based methods can provide further information regarding alternative splicing and sequence variation in previously identified genes. Sequencing technologies include a number of steps that are grouped broadly as template preparation, sequencing, detection and data analysis. Current methods for template preparation involve randomly breaking genomic DNA into smaller sizes from which each fragment is immobilized to a support. The immobilization of spatially separated fragment allows thousands to billions of sequencing reaction to be performed simultaneously. A sequencing step may use any of a variety of methods that are commonly known in the art. One specific example of a sequencing step uses the addition of nucleotides to the complementary strand to provide the DNA sequence. The detection steps range from measuring bioluminescent signal of a synthesized fragment to four-color imaging of single molecule. In some embodiments in which NGS is used to detect or quantify the presence of RO BaSIRS nucleic acid biomarker in a sample, the methods are suitably selected from semiconductor sequencing (Ion Torrent; Personal Genome Machine); Helicos True Single Molecule Sequencing (tSMS) (Harris et a/. 2008, Science 320:106-109); 454 sequencing (Roche) (Margulies et al. 2005, Nature, 437, 376-380); SOLiD technology (Applied Biosystems); SOLEXA sequencing (Illumina); single molecule, real-time (SMRT™) technology of Pacific Biosciences; nanopore sequencing (Soni and Meller, 2007. Clin Chem 53: 1996-2001); DNA nanoball sequencing; sequencing using technology from Dover Systems (Polonator), and technologies that do not require amplification or otherwise transform native DNA prior to sequencing (*e.g.*, Pacific Biosciences and Helicos), such as nanopore-based strategies (*e.g.*, Oxford Nanopore, Genia Technologies, and Nabsys).

In other embodiments, RO BaSIRS biomarker protein levels are assayed using protein-based assays known in the art. For example, when RO BaSIRS biomarker protein is an enzyme, the protein can be quantified based upon its catalytic activity or based upon the number of molecules of the protein contained in a sample. Antibody-based techniques may be employed including, for example, immunoassays, such as the enzyme-linked immunosorbent assay (ELISA) and the radioimmunoassay (RIA).

In specific embodiments, protein-capture arrays that permit simultaneous detection and/or quantification of a large number of proteins are employed. For example, low-density protein arrays on filter membranes, such as the universal protein array system (Ge, 2000 Nucleic Acids Res. 28(2):e3) allow imaging of arrayed antigens using standard ELISA techniques and a scanning charge-coupled device (CCD) detector. Immuno-sensor arrays have also been developed that enable the simultaneous detection of clinical analytes. It is now possible using protein arrays, to profile protein expression in bodily fluids, such as in sera of healthy or diseased subjects, as well as in subjects pre- and post-drug treatment.

Exemplary protein capture arrays include arrays comprising spatially addressed antigen-binding molecules, commonly referred to as antibody arrays, which can facilitate extensive parallel analysis of numerous proteins defining a proteome or subproteome. Antibody arrays have been shown to have the required properties of specificity and acceptable background, and some are available commercially (*e.g.*, BD Biosciences, Clontech, Bio-Rad and Sigma). Various methods for the preparation of antibody arrays have been reported (see, *e.g.*, Lopez et al., 2003 J. Chromatogram. B 787:19-27; Cahill, 2000 Trends in Biotechnology 7:47-51; U.S. Pat. App. Pub. 2002/0055186; U.S. Pat. App. Pub. 2003/0003599; PCT publication WO 03/062444; PCT publication WO 03/077851; PCT publication WO 02/59601; PCT publication WO 02/39120; PCT publication WO 01/79849; PCT publication WO 99/39210). The antigen-binding molecules of such arrays may recognize at least a subset of proteins expressed by a cell or population of cells, illustrative examples of which include growth factor receptors, hormone receptors, neurotransmitter receptors, catecholamine receptors, amino acid derivative receptors, cytokine receptors, extracellular matrix receptors, antibodies, lectins, cytokines, serpins, proteases, kinases, phosphatases, ras-like GTPases, hydrolases, steroid hormone receptors, transcription factors, heatshock transcription factors, DNA-binding proteins, zinc-finger proteins, leucine-zipper proteins, homeodomain proteins, intracellular signal transduction modulators and effectors, apoptosisrelated factors, DNA synthesis factors, DNA repair factors, DNA recombination factors and cellsurface antigens.

Individual spatially distinct protein-capture agents are typically attached to a support surface, which is generally planar or contoured. Common physical supports include glass slides, silicon, microwells, nitrocellulose or PVDF membranes, and magnetic and other microbeads.

Particles in suspension can also be used as the basis of arrays, providing they are coded for identification; systems include color coding for microbeads (*e.g.*, available from Luminex, Bio-Rad and Nanomics Biosystems) and semiconductor nanocrystals (*e.g.*, QDots™, available from Quantum Dots), and barcoding for beads (UltraPlex™, available from Smartbeads) and multimetal microrods (Nanobarcodes™ particles, available from Surromed). Beads can also be assembled into planar arrays on semiconductor chips (*e.g.*, available from LEAPS technology and BioArray Solutions). Where particles are used, individual protein-capture agents are typically attached to an individual particle to provide the spatial definition or separation of the array. The particles may then be assayed separately, but in parallel, in a compartmentalized way, for example in the wells of a microtiter plate or in separate test tubes.

In operation, a protein sample, which is optionally fragmented to form peptide fragments (see, *e.g.*, U.S. Pat. App. Pub. 2002/0055186), is delivered to a protein-capture array under conditions suitable for protein or peptide binding, and the array is washed to remove unbound or non-specifically bound components of the sample from the array. Next, the presence or amount of protein or peptide bound to each feature of the array is detected using a suitable detection system. The amount of protein bound to a feature of the array may be determined relative to the amount of a second protein bound to a second feature of the array. In certain embodiments, the amount of the second protein in the sample is already known or known to be invariant.

In specific embodiments, the RO BaSIRS biomarker is a target polypeptide whose level is measured using at least one antigen-binding molecule that is immuno-interactive with the target polypeptide. In these embodiments, the measured level of the target polypeptide is normalized to the level of a reference polypeptide. Suitably, the antigen-binding molecule is immobilized on a solid or semi-solid support. In illustrative examples of this type, the antigen-binding molecule forms part of a spatial array of antigen-binding molecule. In some embodiments, the level of antigen-binding molecule that is bound to the target polypeptide is measured by immunoassay (*e.g.*, using an ELISA).

All the essential reagents required for detecting and quantifying the RO BaSIRS biomarkers of the invention may be assembled together in a kit. In some embodiments, the kit comprises a reagent that permits quantification of at least one RO BaSIRS biomarker. In some embodiments the kit comprises: (i) a reagent that allows quantification (*e.g.*, determining the level or abundance) of a first RO BaSIRS biomarker; and (ii) a reagent that allows quantification (*e.g.*, determining the level or abundance) of a second RO BaSIRS biomarker, wherein the first and second biomarkers have a mutual correlation in respect of the absence of BaSIRS that lies within a mutual correlation range of between ±0.9, and wherein a combination of respective biomarker values for the first and second RO BaSIRS biomarkers that are measured or derived for a subject has a performance value greater than or equal to a performance threshold representing the ability of the combination of the first and second RO BaSIRS biomarkers to diagnose the absence of BaSIRS, or to provide a prognosis for a non-BaSIRS condition (*e.g.*, a SIRS condition other than BaSIRS), the performance threshold being a variance explained of at least 0.3. In some embodiments, the kit further comprises (iii) a reagent that allows quantification (*e.g.*, determining the level or abundance) of a third RO BaSIRS biomarker; and (iv) a reagent that allows quantification (*e.g.*, determining the level or abundance) of a fourth RO BaSIRS biomarker, wherein the third and fourth RO BaSIRS biomarkers have a mutual correlation in respect of the absence of BaSIRS that lies within a mutual correlation range of between ±0.9, and wherein a combination of respective biomarker values for the third and fourth RO BaSIRS biomarkers that are measured or derived for a subject has a performance value greater than or equal to a performance threshold representing the ability of the combination of the third and fourth RO BaSIRS biomarkers to diagnose the absence of BaSIRS, or to provide a prognosis for a non-BaSIRS condition (*e.g.*, a SIRS condition other than BaSIRS), the performance threshold being a variance explained of at least 0.3. In some embodiments, the kit further comprises (v) a reagent that allows quantification (*e.g.*, determining the level or abundance) of a fifth RO BaSIRS biomarker; and (vi) a reagent that allows quantification (*e.g.*, determining the level or abundance) of a sixth RO BaSIRS biomarker, wherein the fifth and sixth RO BaSIRS biomarkers have a mutual correlation in respect of the absence of BaSIRS that lies within a mutual correlation range of between ±0.9, and wherein a combination of respective biomarker values for the fifth and sixth RO BaSIRS biomarkers that are measured or derived for a subject has a performance value greater than or equal to a performance threshold representing the ability of the combination of the fifth and sixth RO BaSIRS biomarkers to diagnose the absence of BaSIRS, or to provide a prognosis for a non-BaSIRS condition (*e.g.*, a SIRS condition other than BaSIRS), the performance threshold being a variance explained of at least 0.3.

In the context of the present invention, "kit" is understood to mean a product containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components contained in the kit. The instructions can be in the form of printed material or in the form of an electronic support capable of storing instructions such that they can be read by a subject, such as electronic storage media (magnetic disks, tapes and the like), optical media (CD-ROM, DVD) and the like. Alternatively or in addition, the media can contain Internet addresses that provide the instructions.

Reagents that allow quantification of a RO BaSIRS biomarker include compounds or materials, or sets of compounds or materials, which allow quantification of the RO BaSIRS biomarker. In specific embodiments, the compounds, materials or sets of compounds or materials permit determining the expression level of a gene (*e.g.*, RO BaSIRS biomarker gene), including without limitation the extraction of RNA material, the determination of the level of a corresponding RNA, *etc.*, primers for the synthesis of a corresponding cDNA, primers for amplification of DNA, and/or probes capable of specifically hybridizing with the RNAs (or the corresponding cDNAs) encoded by the genes, TaqMan probes, *etc.*

The kits may also optionally include appropriate reagents for detection of labels, positive and negative controls, washing solutions, blotting membranes, microtiter plates, dilution buffers and the like. For example, a nucleic acid-based detection kit may include (i) a RO BaSIRS biomarker polynucleotide (which may be used as a positive control), (ii) a primer or probe that specifically hybridizes to a RO BaSIRS biomarker polynucleotide. Also included may be enzymes suitable for amplifying nucleic acids including various polymerases (reverse transcriptase, *Taq,* Sequenase™, DNA ligase *etc.* depending on the nucleic acid amplification technique employed), deoxynucleotides and buffers to provide the necessary reaction mixture for amplification. Such kits also generally will comprise, in suitable means, distinct containers for each individual reagent and enzyme as well as for each primer or probe. Alternatively, a protein-based detection kit may include (i) a RO BaSIRS biomarker polypeptide (which may be used as a positive control), (ii) an antibody that binds specifically to a RO BaSIRS biomarker polypeptide. The kit can also feature various devices *(e.g.,* one or more) and reagents *(e.g.,* one or more) for performing one of the assays described herein; and/or printed instructions for using the kit to quantify the expression of a RO BaSIRS biomarker gene and/or carry out an indicator-determining method, as broadly described above and elsewhere herein.

The reagents described herein, which may be optionally associated with detectable labels, can be presented in the format of a microfluidics card, a chip or chamber, a microarray or a kit adapted for use with the assays described in the examples or below, *e.g.*, RT-PCR or Q PCR techniques described herein.

The reagents also have utility in compositions for detecting and quantifying the biomarkers of the invention. For example, a reverse transcriptase may be used to reverse transcribe RNA transcripts, including mRNA, in a nucleic acid sample, to produce reverse transcribed transcripts, including reverse transcribed mRNA (also referred to as "cDNA"). In specific embodiments, the reverse transcribed mRNA is whole cell reverse transcribed mRNA (also referred to herein as "whole cell cDNA"). The nucleic acid sample is suitably derived from components of the immune system, representative examples of which include components of the innate and adaptive immune systems as broadly discussed for example above. In specific embodiments, the reverse transcribed RNA is derived blood cells (*e.g.*, peripheral blood cells). Suitably, the reverse transcribed RNA is derived leukocytes.

The reagents are suitably used to quantify the reverse transcribed transcripts. For example, oligonucleotide primers that hybridize to the reverse transcribed transcript can be used to amplify at least a portion of the reverse transcribed transcript *via* a suitable nucleic acid amplification technique, *e.g.*, RT-PCR or qPCR techniques described herein. Alternatively, oligonucleotide probes may be used to hybridize to the reverse transcribed transcript for the quantification, using a nucleic acid hybridization analysis technique (*e.g.*, microarray analysis), as described for example above. Thus, in some embodiments, a respective oligonucleotide primer or probe is hybridized to a complementary nucleic acid sequence of a reverse transcribed transcript in the compositions of the invention. The compositions typically comprise labeled reagents for detecting and/or quantifying the reverse transcribed transcripts. Representative reagents of this type include labeled oligonucleotide primers or probes that hybridize to RNA transcripts or reverse transcribed RNA, labeled RNA, labeled reverse transcribed RNA as well as labeled oligonucleotide linkers or tags (e.g., a labeled RNA or DNA linker or tag) for labeling (e.g., end labeling such as 3' end labeling) RNA or reverse transcribed RNA. The primers, probes, RNA or reverse transcribed RNA *(i.e.,* cDNA) (whether labeled or non-labeled) may be immobilized or free in solution. Representative reagents of this type include labeled oligonucleotide primers or probes that hybridize to reverse transcribed and transcripts as well as labeled reverse transcribed transcripts. The label can be any reporter molecule as known in the art, illustrative examples of which are described above and elsewhere herein.

The present invention also encompasses non-reverse transcribed RNA embodiments in which cDNA is not made and the RNA transcripts are directly the subject of the analysis. Thus, in other embodiments, reagents are suitably used to quantify RNA transcripts directly. For example, oligonucleotide probes can be used to hybridize to transcripts for quantification of immune system biomarkers of the invention, using a nucleic acid hybridization analysis technique (e.g., microarray analysis), as described for example above. Thus, in some embodiments, a respective oligonucleotide probe is hybridized to a complementary nucleic acid sequence of an immune system biomarker transcript in the compositions of the invention. In illustrative examples of this type, the compositions may comprise labeled reagents that hybridize to transcripts for detecting and/or quantifying the transcripts. Representative reagents of this type include labeled oligonucleotide probes that hybridize to transcripts as well as labeled transcripts. The primers or probes may be immobilized or free in solution.

The present invention also extends to the management of SIRS, or prevention of progression to SIRS with at least one clinical sign of SIRS. A subject positively identified as having an absence of BaSIRS is either not exposed to treatment or exposed to a non-BaSIRS treatment, including a treatment for SIRS conditions other than BaSIRS, such as but not limited to, a treatment for ADaSIRS, CANaSIRS, TRAUMaSIRS, ANAPHYLaSIRS, SCHIZaSIRS or VaSIRS. Representative treatments of this type typically include administration of vasoactive compounds, steroids, anti tumour necrosis factor agents, recombinant protein C and anti-viral compounds such as Aciclovir, Brivudine, Cidofovir, Famciclovir, Fomivirsen, Foscarnet, Ganciclovir, HDP-CDV, Idoxuridine, Letermovir, Maribavir, Penciclovir, Resiquimod, Sorivudine, Trifluridine, Tromantadine, Valaciclovir, Valganciclovir, Vidarabine or salts and combinations thereof. Non-limiting therapies for non-bacterium associated SIRS conditions are disclosed for example by Healy (2002, Ann. Pharmacother. 36(4): 648-54) and Brindley (2005, CJEM. 7(4): 227) and Jenkins (2006, J Hosp Med. 1(5): 285-295). In representative embodiments in which BaSIRS is ruled out, the subject is not exposed to antibiotics.

Typically, the therapeutic agents will be administered in pharmaceutical (or veterinary) compositions together with a pharmaceutically acceptable carrier and in an effective amount to achieve their intended purpose. The dose of active compounds administered to a subject should be sufficient to achieve a beneficial response in the subject over time such as a reduction in, or relief from, the symptoms of BaSIRS. The quantity of the pharmaceutically active compounds(s) to be administered may depend on the subject to be treated inclusive of the age, sex, weight and general health condition thereof. In this regard, precise amounts of the active compound(s) for administration will depend on the judgment of the practitioner. In determining the effective amount of the active compound(s) to be administered in the treatment or prevention of BaSIRS, the medical practitioner or veterinarian may evaluate severity of any symptom or clinical sign associated with the presence of BaSIRS or degree of BaSIRS including, inflammation, blood pressure anomaly, tachycardia, tachypnea fever, chills, vomiting, diarrhea, skin rash, headaches, confusion, muscle aches, seizures. In any event, those of skill in the art may readily determine suitable dosages of the therapeutic agents and suitable treatment regimens without undue experimentation.

The therapeutic agents may be administered in concert with adjunctive (palliative) therapies to increase oxygen supply to major organs, increase blood flow to major organs and/or to reduce the inflammatory response. Illustrative examples of such adjunctive therapies include non-steroidal-anti-inflammatory drugs (NSAIDs), intravenous saline and oxygen.

The present invention also contemplates the use of the indicator-determining methods, apparatus, compositions and kits disclosed herein in methods for managing a subject with at least one clinical sign of SIRS. These methods (also referred to herein as "management methods") generally comprise not exposing the subject to a treatment regimen for specifically treating BaSIRS based on an indicator obtained from an indicator-determining method, wherein the indicator is indicative of the absence of BaSIRS in the subject, and of ruling out the likelihood of the presence of BaSIRS in the subject, and wherein the indicator-determining method is an indicator-determining method as broadly described above and elsewhere herein. In specific embodiments, the management methods comprise: (a) determining a plurality of biomarker values, each biomarker value being indicative of a value measured or derived for at least two (*e.g.*, 2, 3, 4, 5, 6, 7, 8, 9, 10, or more) RO BaSIRS biomarker of the subject; (b) determining an indicator using a combination of the plurality of biomarker values, the indicator being at least partially indicative of the absence of BaSIRS, wherein: (i) at least two (*e.g.*, 2, 3, 4, 5, 6, 7, 8, 9, 10, or more) RO BaSIRS biomarkers have a mutual correlation in respect of the absence of BaSIRS that lies within a mutual correlation range, the mutual correlation range being between ±0.9; and (ii) the indicator has a performance value greater than or equal to a performance threshold representing the ability of the indicator to diagnose the absence of BaSIRS, the performance threshold being indicative of an explained variance of at least 0.3; and (c) not exposing the subject to a treatment regimen for specifically treating BaSIRS and/or exposing the subject to a non-BaSIRS treatment, or not exposing the subject to a treatment.

In advantageous embodiments, the management methods comprise: (1) determining a plurality of measured biomarker values, each measured biomarker value being a measured value of a RO BaSIRS biomarker of the subject; and (2) applying a function to at least one of the measured biomarker values to determine at least one derived biomarker value, the at least one derived biomarker value being indicative of a value of a corresponding derived RO BaSIRS biomarker. The function suitably includes at least one of: (a) multiplying two biomarker values; (b) dividing two biomarker values; (c) adding two biomarker values; (d) subtracting two biomarker values; (e) a weighted sum of at least two biomarker values; (f) a log sum of at least two biomarker values; and (g) a sigmoidal function of at least two biomarker values.

The present invention also contemplates methods in which the indicator-determining method of the invention is implemented using one or more processing devices. In some embodiments, these methods comprise: (1) determining a pair of biomarker values, the pair of biomarker values being selected from the group consisting of: (a) a first pair of biomarker values indicative of a concentration of polynucleotide expression products of a Group A RO BaSIRS biomarker gene (*e.g., DIAPH2*) and a Group B RO BaSIRS biomarker gene (*e.g., SERTAD2);* and (b) a second pair of biomarker values indicative of a concentration of polynucleotide expression products of a Group C RO BaSIRS biomarker gene (*e.g., PARL*) gene and a Group D RO BaSIRS biomarker gene (*e.g., PAFAH2);* and (c) a third pair of biomarker values indicative of a concentration of polynucleotide expression products of a Group E RO BaSIRS biomarker gene (*e.g.,*

*SORT1)* gene and a Group F RO BaSIRS biomarker gene (*e.g., OSBPL9*)*; (2)* determining an indicator indicative of a ratio of the concentrations of the polynucleotide expression products using all three biomarker values; (3) retrieving previously determined first, second and third indicator references from a database, the first, second and third indicator references being determined based on indicators determined from first, second and third groups of a reference population, one of the groups consisting of individuals diagnosed with a non-BaSIRS SIRS condition; (4) comparing the indicator to the first, second and third indicator references; (5) using the results of the comparison to determine a probability indicative of the subject having or not having BaSIRS; and (6) generating a representation of the probability, the representation being displayed to a user to allow the user to assess the likelihood of a biological subject having an absence of BaSIRS or not.

Similarly apparatus can be provided for determining the likelihood of a subject having an absence of BaSIRS, the apparatus including: (A) a sampling device that obtains a sample taken from a subject, the sample including polynucleotide expression products; (B) a measuring device that quantifies polynucleotide expression products within the sample to determine three biomarker values, the three biomarker values being selected from the group consisting of: (a) a first pair of biomarker values indicative of a concentration of polynucleotide expression products of a Group A RO BaSIRS biomarker gene (*e.g., DIAPH2*) and a Group B RO BaSIRS biomarker gene (*e.g., SERTAD2);* and (b) a second pair of biomarker values indicative of a concentration of polynucleotide expression products of a Group C RO BaSIRS biomarker gene (*e.g., PARL*) gene and a Group D RO BaSIRS biomarker gene (*e.g., PAFAH2);* and (c) a third pair of biomarker values indicative of a concentration of polynucleotide expression products of a Group E RO BaSIRS biomarker gene (*e.g., SORT1*) gene and a Group F RO BaSIRS biomarker gene (*e.g., OSBPL9*);(C) at least one processing device that: (i) receives an indication of the pair of biomarker values from the measuring device; (ii) determines an indicator using a ratio of the concentration of the first, second and third polynucleotide expression products using the biomarker values; (iii) compares the indicator to at least one indicator reference; (iv) determines a likelihood of the subject having or not having BaSIRS condition using the results of the comparison; and (v) generates a representation of the indicator and the likelihood for display to a user.

The present invention also encompasses methods for differentiating between BaSIRS and another SIRS other than BaSIRS in a subject. These methods suitably comprise: (a) obtaining a sample taken from a subject showing a clinical sign of SIRS, the sample including polynucleotide expression products; (b) in a measuring device: (i) amplifying at least some polynucleotide expression products in the sample; (ii) determining an amplification amount representing a degree of amplification required to obtain a defined level of polynucleotide expression products including: amplification amounts for a first pair of polynucleotide expression products of of a Group A RO BaSIRS biomarker gene (*e.g., DIAPH2*) and a Group B RO BaSIRS biomarker gene (*e.g., SERTAD2);* and amplification amounts for a second pair of polynucleotide expression products of a Group C RO BaSIRS biomarker gene (*e.g., PARL*) gene and a Group D RO BaSIRS biomarker gene (*e.g., PAFAH2);* and amplification amounts for a third pair of polynucleotide expression products of a Group E RO BaSIRS biomarker gene (*e.g., SORT1*) gene and a Group F RO BaSIRS biomarker gene (*e.g., OSBPL9);* (c) in a processing system: (i) retrieving the amplification amounts; (ii) determining an indicator by: determining a first derived biomarker value indicative of a ratio of concentrations of the first pair of polynucleotide expression products by determining a difference between the amplification amounts for the first pair; determining a second derived biomarker value indicative of a ratio of concentrations of the second pair of polynucleotide expression products by determining a difference between the amplification amounts for the second pair; determining a third derived biomarker value indicative of a ratio of concentrations of the third pair of polynucleotide expression products by determining a difference between the amplification amounts for the third pair; (d) determining the indicator by adding the first, second and third derived biomarker values; (e) retrieving previously determined first, second and third indicator references from a database, wherein the first, second and third indicator references are distributions of indicators determined for first and second groups of a reference population, the first and second groups consisting of individuals diagnosed with BaSIRS and SIRS conditions other than BaSIRS, respectively; (f) comparing the indicator to the first and second indicator references; (g) using the results of the comparison to determine a probability of the subject being classified within the first or second group; (h) generating a representation at least partially indicative of the indicator and the probability; and (i) providing the representation to a user to allow the user to assess the likelihood of a subject having or not having BaSIRS or the other SIRS condition.

Additionally, methods can be provided for determining an indicator used in assessing a likelihood of a subject having an absence of BaSIRS. These methods suitably include: (1) determining a plurality of biomarker values, each biomarker value being indicative of a value measured or derived for at least one corresponding RO BaSIRS biomarker of the subject and being at least partially indicative of a concentration of the RO BaSIRS biomarker in a sample taken from the subject; (2) determining the indicator using a combination of the plurality of biomarker values, wherein: at least two biomarkers have a mutual correlation in respect of an absence of BaSIRS that lies within a mutual correlation range, the mutual correlation range being between $\pm 0.9$; and the indicator has a performance value greater than or equal to a performance threshold representing the ability of the indicator to diagnose the absence of BaSIRS, the performance threshold being indicative of an explained variance of at least 0.3.

In order that the invention may be readily understood and put into practical effect, particular preferred embodiments will now be described by way of the following non-limiting examples.

**EXAMPLES**

EXAMPLE 1

**GROUPS A, B, C, D, E AND F BIOMARKERS. BIOMARKERS ARE GROUPED BASED ON THEIR CORRELATION TO DIAPH2, SERTAD2, PARL, PAFAH2, SORT1 AND OSBPL9**

Three pairs of derived biomarkers (DIAPH2 / SERTAD2; PARL / PAFAH2; SORT1 / OSBPL9) were discovered that provided the highest AUC across all of bacterial datasets studied. Biomarkers were then allocated to one of six Groups, as individual biomarkers, based on their correlation to either DIAPH2 (Group A), SERTAD2 (Group B), PARL (Group C), PAFAH2 (Group D), SORT1 (Group E) or OSBPL9 (Group F), as presented in Table 5. Calculated Negative Predictive Values (NPV) and Areas Under Curve (AUC) for 200 derived biomarkers at a set BaSIRS prevalence of 10% and 5% are presented in Table 6 and Table 7. The NPV of these 200 derived biomarkers increases as the prevalence of BaSIRS decreases, so all those listed would perform well in an emergency room setting where the prevalence of BaSIRS is estimated to be closer to 4%.

EXAMPLE 2

**RO BASIRS BIOMARKER DERIVATION (DERIVED BIOMARKERS)**

An illustrative process for the identification of emergency room (ER) RO BaSIRS biomarkers for use in diagnostic algorithms will now be described.

Using publicly available datasets (from Gene Expression Omnibus, GEO) the aim was to find specific biomarkers that differentiated between those subjects with BaSIRS and those subjects that are either healthy, have a known viral infection, or have other known non-bacterial inflammation. First, biomarkers with strong diagnostic potential were generated that were able to differentiate BaSIRS and healthy subjects (Set A biomarkers) - see Table 1 for a list of the GEO datasets used to generate these biomarkers. Secondly, biomarkers with strong diagnostic potential were generated that were able to differentiate BaSIRS and subjects with non-bacterial systemic inflammation, including viral infection, autoimmune disease and trauma (Set B biomarkers) - see Table 2 for a list of the GEO datasets used to generate these biomarkers. Thirdly, biomarkers with diagnostic potential were generated that were able to differentiate non-bacterial systemic inflammation and healthy subjects (Set C biomarkers). Set A and B biomarkers were then pooled, since they are able to differentiate BaSIRS from other infections and systemic inflammation, and Set C biomarkers were subtracted from this pool. Thus, the formula for generating RO BaSIRSspecific biomarkers in this instance was (A+B)-C.

A list of the public datasets used to generate biomarker Sets A, B and C can be found in Tables 1 and 2. Each of the public datasets were quality control screened prior to inclusion in each Set to ensure that no artifacts existed (such as batch effect). The primary tool used to determine the quality of each dataset was Principal Component Analysis (PCA).

Once appropriate datasets had been selected a search for derived biomarkers (as a ratio) was implemented. For inclusion in biomarker Sets A and B the derived biomarkers were required to obtain a significant Area Under Curve (AUC) in each of the eight RO BaSIRS datasets individually (rather than including any derived biomarker that reached a significant AUC in any dataset). The total number of derived biomarkers considered initially in Sets A and B was over 18 million. For inclusion in biomarker Set C the derived biomarkers were required to obtain an Area Under Curve (AUC) higher than 0.8.

Following selection of derived biomarkers in Sets A, B and C the derived biomarkers in Set C were taken from the pool of derived biomarkers that made up Sets A and B (12,379,842 ratios). That is, the formula (A+B)-C was performed. Table 2 shows the percent overlap of significant derived biomarkers for each dataset in Set C when compared to the derived biomarkers that made up Sets A and B. The largest overlap with RO BaSIRS derived biomarkers was found between significant derived biomarkers found in major trauma (55%). Such overlap was to be expected since it has been published that major trauma causes a "genomic storm" response in gene expression changes in peripheral blood (Xiao, W., Mindrinos, M. N., Seok, J., Cuschieri, J., Cuenca, A. G., Gao, H., et al. (2011). A genomic storm in critically injured humans. Journal of Experimental Medicine, 208(13), 2581-2590). After only allowing ratios in the clinical datasets above an AUC of 0.8 and then subtraction of the C ratios, only 111929 derived biomarkers remained (of 12 million). Thus, it can be considered that these remaining derived biomarkers are specific to BaSIRS in a heterogenous SIRS patient population with low prevalence of BaSIRS. Such specificity to BaSIRS provides strong negative predictive value, or an ability to rule out BaSIRS. The AUC of the best single derived biomarker in this final set was 0.896 (SORT1 / CD81).

With respect to host response markers, a non-limiting example of how biomarkers were identified will now be described. For the purpose of illustration and in general, the process as described in Australian Provisional Patent Application number AU2014900363 ("Biomarker signature method and apparatus and kits therefor") was used to select biomarkers that provided the theoretical best diagnostic biomarkers, selected from combinations including measured and/or derived biomarkers using publicly available datasets (Gene Expression Omnibus, GEO) that contain patient cohorts of known status, including bacterial infection, non-bacterial inflammation and healthy conditions; GSE30119, GSE33341, GSE16129, GSE25504, GSE40586, GSE6269, GSE40012, GSE40396, GSE17755, GSE19301, GSE35846, GSE36809, GSE38485, GSE47655 and GSE52428. All datasets used fitted the following criteria; peripheral blood samples were used, appropriate controls were used, an appropriate number of samples were used to provide significance following False-Discovery Rate (FDR) adjustment, all data passed standard quality control metrics, principle component analysis did not reveal any artifacts or potential biases. As a first step biomarkers and derived biomarkers with good performance (mean Under Curve, AUC >0.8 across all bacterial infection datasets) for separating cohorts with known bacterial infection from non-bacterial inflammation and from healthy were selected. Secondly, biomarkers and derived biomarkers with good performance (any biomarker with an AUC >0.78 for separating cohorts with known non-bacterial inflammation and from healthy were selected. The latter were then subtracted from the former to ensure that the remaining biomarkers and derived biomarkers were specific to bacterial infection. In total, over 12 million ratio combinations were generated in the first step resulted in ~4000 ratios that hold valuable information in the diagnosis of BaSIRS vs non-bacterial SIRS in an ED cohort. To further reduce number of biomarkers and to reduce the collinearity in the system, a between-ratio correlation cutoff of 0.7 was used which resulted in 200 final ratios following subtraction and filtering steps. Using machine learning methods the best combinations of biomarkers and derived biomarkers was then determined.

The performance of the top three derived biomarkers, singly and in combination, that are best capable of separating BaSIRS and non-bacterial SIRS are shown in the Figures 1, 2, 3 and 5. Additional lists of top performing derived biomarkers (as measured by AUC and NPV) are also presented in Table 6 and Table 7.

EXAMPLE 3

**RO BASIRS BIOMARKER DERIVATION (COMBINATION OF DERIVED BIOMARKERS)**

Following filtering out of derived biomarkers non-specific to BaSIRS various machine learning methods were applied to identify the optimal combination of derived biomarkers that provided the greatest AUC. Machine learning methods used included; random forests, support vector machines, logistic regression and greedy forward selection. After applying all of these methods individually it was found that they all performed equally well when restricting the model to the use of five derived biomarkers or less. This latter restriction was applied for practicality and to ensure ability to reduce the biomarkers to a working assay. Ultimately greedy forward selection was used, which resulted in a number of sets of derived biomarkers with high AUCs (see Figure 4).

Following the application of the greedy search algorithm the top three derived biomarker combinations identified were DIAPH2 / SERTAD2; PARL / PAFAH2; SORT1 / OSBPL9. The combination of these three biomarker ratios gave an AUC of 0.94 for separating BaSIRS from non-BaSIRS SIRS conditions, which was considered to be the minimal set of derived biomarkers with optimal commercial utility. Optimal commercial utility in this instance means consideration of the following non-limiting factors; diagnostic performance, clinical utility, diagnostic noise (introduced by using too many derived biomarkers), transferability to available molecular chemistries (e.g., PCR, microarray, DNA sequencing), transferability to available point-of-care platforms (e.g. Biocartis Idylla, Cepheid GeneXpert, Becton Dickinson BD Max, Curetis Unyvero, Oxford Nanopore Technologies MinION), cost of assay manufacture (the more reagents and biomarkers the larger the cost), ability to multiplex biomarkers, availability of suitable reporter dyes, complexity of results interpretation.

EXAMPLE 4

**RO BASIRS BIOMARKER PERFORMANCE (DERIVED BIOMARKERS AND COMBINED DERIVED BIOMARKERS)**

The performance (AUC and NPV) of the top 200 derived biomarkers at a defined BaSIRS prevalence of 10% and 5% is shown in Table 6 and Table 7. The NPV of each of these derived biomarkers in practice could possibly be higher than that shown in these tables because the prevalence of suspected BaSIRS in emergency rooms has been shown to be approximately 4% (Niska, R., Bhuiya, F., & Xu, J. (2010). National hospital ambulatory medical care survey: 2007 emergency department summary. Natl Health Stat Report, 26(26), 1-31). The lower the prevalence the higher the NPV of these derived biomarkers.

Following a greedy search the best performing individual derived biomarker was DIAPH2 / SERTAD2 with an AUC of 0.863. The best second unique derived biomarker to add to the first derived biomarker was PARL / PAFAH2. The AUC obtained across the normalized dataset using these two derived biomarkers was 0.92, an 0.057 improvement over the use of a single derived biomarker. The addition of a third derived biomarker (SORT1 / OSBPL9) improved the AUC by 0.02 to 0.94 (AUC ROC plots for these derived biomarkers are shown in Figure 1). It is possible that the addition of more derived biomarkers created overfitting and noise (see Figure 4). Thus, it was considered that the optimal commercial RO BaSIRS signature consists of the following three derived biomarkers: DIAPH2 / SERTAD2; PARL/ PAFAH2; SORT1 / OSBPL9.

Figure 2, Figure 3 and Figure 5 show plots demonstrating the performance of the top three derived biomarkers in each of the RO BaSIRS datasets, in the RO BaSIRS datasets combined, and in a dataset consisting of samples collected from a clinical trial performed by the applicants respectively. This latter dataset involved collecting samples from patients presenting to emergency with fever.

EXAMPLE 5

## RO BASIRS BIOMARKER PROFILES (GROUPING)

The BaSIRS biomarker profiles can be grouped into derived biomarkers and combinations of derived biomarkers.
There are six biomarkers in the best performing three derived biomarker signature: DIAPH2 / SERTAD2; PARL / PAFAH2; SORT1 / OSBPL9, 102 derived biomarkers with an NPV > 0.95 (prevalence 10%) and 179 unique biomarkers. For each unique biomarker, a correlation coefficient was calculated. Table 5 lists 179 unique biomarkers and their correlation to each of the six biomarkers in the top performing three-derived biomarker signature. Each set of biomarkers make up Groups A, B, C, D, E and F respectively.
The best combination of derived biomarkers was determined to be: DIAPH2 / SERTAD2; PARL / PAFAH2; SORT1 / OSBPL9 (Group G).

EXAMPLE 6

## ANALYSIS OF DERIVED BIOMARKERS

Performance (AUC and NPV) of 200 derived biomarkers at a set prevalence of 10% is shown in Table 6, and of these, 102 have NPV greater than 0.95. Performance of DIAPH2 / SERTAD2; PARL / PAFAH2; SORT1 / OSBPL9 across each of the BaSIRS datasets is shown in Figure 2. Performance of DIAPH2 / SERTAD2; PARL / PAFAH2 and SORT1 / OSBPL9 are shown in Figure 1 and 3.
Numerators and denominators that appear more than once in the top 200 derived biomarkers are listed in Table 9. The two most common numerators include TLR5 and MMP8, and the two most common denominators include ILR7 and CCND2.

EXAMPLE 7

## VALIDATION OF DERIVED BIOMARKERS IN AN INDEPENDENT DATASET

A dataset used for validation also satisfied the conditions stated above and was derived from a clinical trial within an ED based at University College London. Patients (n=36) were included in they presented with fever (Figure 5a). Retrospective clinical microbiology results were used to categorize subjects into three groups, including: positive microbiology from a sterile site, positive virology, negative microbiology (and not on antibiotics). Figure 5a presents a box and whisker plot using the combined derived biomarkers of DIAPH2 / SERTAD2; PARL / PAFAH2 and SORT1 / OSBPL9 on this patient population. The AUC between patients with positive microbiology and all others (viral positive and negative microbiology) was 0.904 in this validation set. Figures 5b and 5c represent box and whisker plots for two signatures validated in an expanded patient cohort from the same independent clinical trial involving patients presenting to an ED at University College London (see Figures 5b and 5c). Patients (n=59) were included in they presented with fever and were admitted to hospital. Retrospective clinical microbiology results were used to categorize subjects into three groups, including: positive microbiology from a sterile site ("bacterial, n=32), positive virology ("viral", n=14), negative microbiology, not on antibiotics and recovered without incident ("No positive micro, no Abx", n=13). Only those patients suspected of having a viral infection were tested for viruses using either PCR or serology. Figures 5b and 5c present a box and whisker plots using the combined derived biomarkers of a) DIAPH2 / SERTAD2; PARL / PAFAH2 and SORT1 / OSBPL9 or, b) DIAPH2 / IL7R; GBP2 / GIMAP4; TLR5 / FGL2 on this patient population. Performance of individual ratios in each of these signatures can be found in Table 6. This patient population does not fully represent the intended use of the RO BaSIRS since the patients were all admitted to hospital with a clinical suspicion of infection. However, the AUCs between "bacterial" vs "viral" and "bacterial" vs "indeterminate" for signatures a and b were respectively; 0.79, 0.65 and 0.93, 0.83. Negative Predictive Values (NPV) for bacterial vs other for signatures a and b were 0.975 and 0.978 respectively at a sepsis prevalence of 4%. A better patient cohort to truly test the clinical utility of the RO BaSIRS biomarkers would be to compare those patients that had an initial suspicion of infection but were not admitted to hospital (and were not admitted at a later date) to those that were admitted that had a confirmed diagnosis of BaSIRS.

EXAMPLE 8

**EXAMPLE APPLICATIONS OF RO BASIRS BIOMARKER PROFILES**

Use of the above described biomarkers and resulting RO BaSIRS biomarker profiles in patient populations and benefits in respect of differentiating various conditions, will now be described.

An assay capable of excluding BaSIRS in patients presenting to emergency departments can be used to help appropriately triage such patients (to ensure appropriate management, therapy and procedures are employed), as part of efforts to ensure judicious use of antibiotic and anti-viral compounds, and determination of the aetiology of systemic inflammation when due to a bacterial infection.

**Example Use of SeptiCyte Triage In an ED Patient Population**

In 2010, approximately 130 million people presented to emergency departments in the USA and the third most common primary reason for the visit was fever (5.6 million people had a fever (>38 °C) and for 5 million people it was the primary reason for the visit) (Niska R, Bhuiya F, Xu J (2010) National hospital ambulatory medical care survey: 2007 emergency department summary. Natl Health Stat Report 26: 1-31). Of those patients with a fever, 664,000 had a fever of unknown origin - that is, the cause of the fever was not obvious at presentation. However, fever as a presenting clinical sign has a reasonable correlation to a final diagnosis of the presence of an infection (van Laar, P. J., & Cohen, J. (2003). A prospective study of fever in the accident and emergency department. Clinical Microbiology and Infection : the Official Publication of the European Society of Clinical Microbiology and Infectious Diseases, 9(8), 878-880; Manning, L. V., &Touquet, R. (1988). The relevance of pyrexia in adults as a presenting symptom in the accident and emergency department. Archives of Emergency Medicine, 5(2), 86-90). As such, in patients presenting to emergency with a fever, it is important to rule out an infection so that unnecessary procedures (including admission), diagnostic tests and therapies are not performed or administered. By example, as part of diagnosing the reason for the emergency department visit in 2010 in the USA, 48,614,000 complete blood counts (CBC) were performed and 5.3 million blood cultures were taken. In 3.65 million patients presenting the primary diagnosis was "infectious" and in approximately 25% of cases (32.4 million) antibiotics were administered. 13.5% of all people presenting to emergency were admitted to hospital. Clinicians in emergency need to determine the answer to a number of questions quickly, including: what is the reason for the visit, is the reason for the visit an infection, does the patient need to be admitted? The diagnosis, treatment and management of patients with a fever, BaSIRS or SIRS due to other causes are different. By way of example, a patient with a fever without other systemic inflammation clinical signs, negative for BaSIRS, and no obvious source of bacterial infection may be sent home, or provided with other non-hospital services, without further hospital treatment. However, a patient with a fever may have early BaSIRS, and not admitting such a patient and aggressively treating with antibiotics may put their life at risk.

The difference in the number of patients presenting to emergency that are ultimately diagnosed with an "infection" (3.65 million) and the number treated with antibiotics (32.4 million) suggests the following; 1) diagnostic tools that determine the presence or absence of an infection are not available, or are not being used, or are not accurate enough, or do not provide strong enough negative predictive value, or are not providing accurate information that can be acted on within a reasonable timeframe 2) when it comes to suspected infection, and because of the acute nature of infections, clinicians err on the side of caution by administering antibiotics. Further, in a study performed in the Netherlands on patients presenting to emergency with fever, 36.6% of patients admitted to hospital had a suspected bacterial infection (that is, it was not confirmed) (Limper M, Eeftinck Schattenkerk D, de Kruif MD, van Wissen M, Brandjes DPM, et al. (2011) One-year epidemiology of fever at the Emergency Department. Neth J Med 69: 124-128). This suggests that a large proportion of patients presenting to emergency are admitted to hospital without a diagnosis. The biomarkers and derived biomarkers outlined in this patent can identify those SIRS patients with a bacterial infection from those without a bacterial infection with high negative predictive value, assisting medical practitioners in triaging patients presenting with fever or other clinical signs of systemic inflammation. Such effective triage tools make best use of scarce hospital resources, including staff, equipment and therapies. Accurate triage decision-making also ensures that patients requiring hospital treatment are given it, and those that don't are provided with other appropriate services.

**Excluding BaSIRS in the immunocompromised and neutropenic**

Patients on chemotherapy / immunosuppressants for the management of tumors or transplants are often immunocompromised and/or develop a neutropenia, with or without fever. Such patients are often outpatients and present to emergency departments with clinical signs of SIRS. Such patients need to be managed carefully and it is important to be able to diagnose or exclude the presence of microbial and opportunistic infections so that appropriate therapies and procedures can be implemented in the shortest possible time (de Naurois, J., Novitzky-Basso, I., Gill, M. J., Marti, F. M., Cullen, M. H., Roila, F., On behalf of the ESMO Guidelines Working Group. (2010). Management of febrile neutropenia: ESMO Clinical Practice Guidelines. Annals of Oncology, 21(Supplement 5), v252-v256; Kasiske, B. L., Vazquez, M. A., Harmon, W. E., Brown, R. S., Danovitch, G. M., Gaston, R. S., et al. (2000, October). Recommendations for the outpatient surveillance of renal transplant recipients. American Society of Transplantation. Journal of the American Society of Nephrology, 11, S1-S86). The biomarkers detailed in this patent can exclude the presence of BaSIRS and could therefore be useful in monitoring immunocompromised patients to; 1) enable early and appropriate treatment if required 2) reduce the use of inappropriate therapies, procedures and management in immunocompromised patients without BaSIRS.

### *Antibiotic stewardship*

In patients suspected of having a systemic infection (viral, bacterial, fungal, parasitic) a clinical diagnosis and treatment regimen is provided by the physician(s) at the time the patient presents and often in the absence of any results from diagnostic tests. This is done in the interests of rapid treatment and positive patient outcomes. However, such an approach leads to over-prescribing of antibiotics irrespective of whether the patient has a microbial infection or not. Clinician diagnosis of BaSIRS is reasonably reliable (0.88) in children but only with respect to differentiating between patients ultimately shown to be blood culture positive and those that were judged to be unlikely to have an infection at the time antibiotics were administered (Fischer, J. E., Harbarth, S., Agthe, A. G., Benn, A., Ringer, S. A., Goldmann, D. A., & Fanconi, S. (2004). Quantifying uncertainty: physicians' estimates of infection in critically ill neonates and children. Clinical Infectious Diseases : an Official Publication of the Infectious Diseases Society of America, 38(10), 1383-1390). In Fischer et al., (2004), 54% of critically ill children were put on antibiotics during their hospital stay, of which only 14% and 16% had proven systemic bacterial infection or localized infection respectively. In this study, 53% of antibiotic treatment courses for critically ill children were for those that had an unlikely infection and 38% were antibiotic treatment courses for critically ill children as a rule-out treatment episode. Clearly, pediatric physicians err on the side of caution with respect to treating critically ill patients by placing all patients suspected of an infection on antibiotics - 38% of all antibiotics used in critically ill children are used on the basis of ruling out BaSIRS, that is, are used as a precaution. Antibiotics are also widely prescribed and overused in adult patients as reported in Braykov et al., 2014 (Braykov, N. P., Morgan, D. J., Schweizer, M. L., Uslan, D. Z., Kelesidis, T., Weisenberg, S. A., et al. (2014). Assessment of empirical antibiotic therapy optimisation in six hospitals: an observational cohort study. The Lancet Infectious Diseases, 14(12), 1220-1227). In this study, across six US hospitals over four days in 2009 and 2010, 60% of all patients admitted received antibiotics. Of those patients prescribed antibiotics 30% were afebrile and had a normal white blood cell count and where therefore prescribed antibiotics as a precaution. As such, an assay that can accurately diagnose an absence of BaSIRS in patients presenting with non-pathognomonic clinical signs of infection would be clinically useful and may lead to more appropriate use of antibiotics and anti-herpes viral therapies.

EXAMPLE 9

**FIRST EXAMPLE WORKFLOW FOR DETERMINING HOST RESPONSE**

A first example workflow for measuring host response to ensure that a SIRS is not BaSIRS will now be described. The workflow involves a number of steps depending upon availability of automated platforms. The assay uses quantitative, real-time determination of the amount of each host immune cell RNA transcript in the sample based on the detection of fluorescence on a qRT-PCR instrument (e.g. Applied Biosystems 7500 Fast Dx Real-Time PCR Instrument, Applied Biosystems, Foster City, CA, catalogue number 440685; K082562). Transcripts are each reverse-transcribed, amplified, detected, and quantified in a separate reaction well using a probe that is visualized in the FAM channel (by example). Such reactions can be run as single-plexes (one probe for one transcript per tube), multiplexed (multiple probes for multiple transcripts in one tube), one-step (reverse transcription and PCR are performed in the same tube), or two-step (reverse transcription and PCR performed as two separate reactions in two tubes). A score is calculated using interpretive software provided separately to the kit but designed to integrate with RT-PCR machines.

The workflow below describes the use of manual processing and a pre-prepared kit.

Pre-analytical

Blood collection

Total RNA isolation

Analytical

Reverse transcription (generation of cDNA)

qPCR preparation

qPCR

Software, Interpretation of Results and Quality Control

Output.

Kit Contents

Diluent

RT Buffer

RT Enzyme Mix

qPCR Buffer

Primer/Probe Mix

AmpliTaq Gold® (or similar)

High Positive Control

Low Positive Control

Negative Control

Blood Collection

The specimen used is a 2.5 mL sample of blood collected by venipuncture using the PAXgene® collection tubes within the PAXgene® Blood RNA System (Qiagen, kit catalogue # 762164; Becton Dickinson, Collection Tubes catalogue number 762165; K042613). An alternate collection tube is Tempus® (Life Technologies).

Total RNA Isolation

Blood (2.5 mL) collected into a PAXgene RNA tube is processed according to the manufacturer's instructions. Briefly, 2.5mL sample of blood collected by venipuncture using the PAXgene™ collection tubes within the PAXgene™ Blood RNA System (Qiagen, kit catalogue # 762164; Becton Dickinson, Collection Tubes catalogue number 762165; K042613). Total RNA isolation is performed using the procedures specified in the PAXgene™ Blood RNA kit (a component of the PAXgene™ Blood RNA System). The extracted RNA is then tested for purity and yield (for example by running an A $_{260/280}$ ratio using a Nanodrop® (Thermo Scientific)) for which a minimum quality must be (ratio > 1.6). RNA should be adjusted in concentration to allow for a constant input volume to the reverse transcription reaction (below). RNA should be processed immediately or stored in single-use volumes at or below -70°C for later processing.

Reverse Transcription

Determine the appropriate number of reaction equivalents to be prepared (master mix formulation) based on a plate map and the information provided directly below. Each clinical specimen is run in singleton.
Each batch run desirably includes the following specimens:

• High Control, Low Control, Negative Control, and No Template Control (Test Diluent instead of sample) in singleton each

Program the ABI 7500 Fast Dx Instrument as detailed below.

• Launch the software.

• Click Create New Document

• In the New Document Wizard, select the following options:

    i. Assay: Standard Curve (Absolute Quantitation)

    ii. Container: 96-Well Clear

    iii. Template: Blank Document (or select a laboratory-defined template)

    iv. Run Mode: Standard 7500

    v. Operator: Enter operator's initials

    vi. Plate name: [default]

• Click Finish

• Select the Instrument tab in the upper left

• In the Thermal Cycler Protocol area, Thermal Profile tab, enter the following times:

    i. 25° C for 10 minutes

    ii. 45° C for 45 minutes

    iii. 93° C for 10 minutes

    iv. Hold at 25° C for 60 minutes

In a template-free area, remove the test Diluent and RT-qPCR Test RT Buffer to room temperature to thaw. Leave the RT-qPCR Test RT Enzyme mix in the freezer and/or on a cold block.

In a template-free area, assemble the master mix in the order listed below.

<div align="center">

RT Master Mix - Calculation:

| | Per well | × N |
|---|---|---|
| RT-qPCR Test RT Buffer | 3.5 μL | 3.5 × N |
| RT-qPCR Test RT Enzyme mix | 1.5 μL | 1.5 × N |
| Total Volume | 5 μL | 5 × N |

</div>

Gently vortex the master mix then pulse spin. Add the appropriate volume (5 μL) of the RT Master Mix into each well at room temperature.

Remove clinical specimens and control RNAs to thaw. (If the specimens routinely take longer to thaw, this step may be moved upstream in the validated method.)

Vortex the clinical specimens and control RNAs, then pulse spin. Add 10 μL of control RNA or RT-qPCR Test Diluent to each respective control or negative well.

Add 10 μL of sample RNA to each respective sample well (150 ng total input for RT; $OD_{260}/OD_{280}$ ratio greater than 1.6). Add 10 μL of RT-qPCR Test Diluent to the respective NTC well.

Note: The final reaction volume per well is 15 μL.

| RT Master Mix | Samples |
| --- | --- |
| | 5 µL |
| RNA sample | 10 µL |
| Total Volume (per well) | 15 µL |

Mix by gentle pipetting. Avoid forming bubbles in the wells.

Cover wells with a seal.

Spin the plate to remove any bubbles (1 minute at 400 × g).

Rapidly transfer to ABI 7500 Fast Dx Instrument pre-programmed as detailed above.

Click Start. Click Save and Continue. Before leaving the instrument, it is recommended to verify that the run started successfully by displaying a time under Estimated Time Remaining.

qPCR master mix may be prepared to coincide roughly with the end of the RT reaction. For example, start about 15 minutes before this time. See below.

When RT is complete (i.e. resting at 25 °C; stop the hold at any time before 60 minutes is complete), spin the plate to collect condensation (1 minute at 400 × g).

qPCR Preparation

Determine the appropriate number of reaction equivalents to be prepared (master mix formulation) based on a plate map and the information provided in RT Preparation above.

Program the ABI 7500 Fast Dx with the settings below.

a) Launch the software.

b) Click Create New Document

c) In the New Document Wizard, select the following options:

i. Assay: Standard Curve (Absolute Quantitation)

ii. Container: 96-Well Clear

iii. Template: Blank Document (or select a laboratory-defined template)

iv. Run Mode: Standard 7500

v. Operator: Enter operator's initials

vi. Plate name: Enter desired file name

d) Click Next

e) In the Select Detectors dialog box:

i. Select the detector for the first biomarker, and then click Add>>.

ii. Select the detector second biomarker, and then click Add>>, etc.

iii. Passive Reference: ROX

f) Click Next

g) Assign detectors to appropriate wells according to plate map.

i. Highlight wells in which the first biomarker assay will be assigned

ii. Click use for the first biomarker detector

iii. Repeat the previous two steps for the other biomarkers

iv. Click Finish

h) Ensure that the Setup and Plate tabs are selected

i) Select the Instrument tab in the upper left

j) In the Thermal Cycler Protocol area, Thermal Profile tab, perform the following actions, with the results shown in Figure 9:

i. Delete Stage 1 (unless this was completed in a laboratory-defined template).

ii. Enter sample volume of 25 μL.

iii. 95 °C 10 minutes

iv. 40 cycles of 95 °C for 15 seconds, 63 °C for 1 minute

v. Run Mode: Standard 7500

vi. Collect data using the "stage 2, step 2 (63.0@1:00)" setting

k) Label the wells as below using this process: Right click over the plate map, then select Well Inspector. With the Well Inspector open, select a well or wells. Click back into the Well Inspector and enter the Sample Name. Close the Well Inspector when completed.

i. CONH for High Control

ii. CONL for Low Control

iii. CONN for Negative Control

iv. NTC for No Template Control

v. [Accession ID] for clinical specimens

l) Ensure that detectors and quenchers are selected as listed below.

i. FAM for DIAPH2 biomarker 1; quencher=none

ii. FAM for SERTAD2 biomarker 2; quencher=none

iii. FAM for PARL; biomarker 3; quencher=none

iv. FAM for PAFAH2; biomarker 4; quencher=none

v. FAM for SORT1; biomarker 5; quencher=none

vi. FAM for OSBPL9; biomarker 6; quencher=none

vii. Select "ROX" for passive reference

<u>qPCR</u>

In a template-free area, remove the assay qPCR Buffer and assay Primer/Probe Mixes for each target to room temperature to thaw. Leave the assay AmpliTaq Gold in the freezer and/or on a cold block.

Still in a template-free area, prepare qPCR Master Mixes for each target in the listed order at room temperature.

<u>qPCR Master Mixes - Calculation Per Sample</u>

|  | Per well | $\times$ N |
|---|---|---|
| qPCR Buffer | 11 $\mu$L | 11 $\times$ N |
| Primer/Probe Mix | 3.4 $\mu$L | 3.4 $\times$ N |
| AmpliTaq Gold® | 0.6 $\mu$L | 0.6 $\times$ N |
| Total Volume | 15 $\mu$L | 15 $\times$ N |

Example forward (F) and reverse (R) primers and probes (P) and their final reaction concentration for measuring six host response transcripts to BaSIRS biomarkers are contained in the following table (F, forward; R, reverse; P, probe).

| Reagent | 5'-3' Sequence | Reaction mM |
|---|---|---|
| DIAPH2-F | GTCCATGAAGAGAATCAATTGGTC | 360 |
| DIAPH2-R | AACTTGTCTTCTTTGACTCTTAACC | 360 |
| DIAPH2-P | CCCACAGAATTATCTGAGAACTG | 50 |
| SERTAD2-F | GTTCCCAGGTGGAGCTGCATG | 360 |
| SERTAD2-R | CCTTCCAGCCCATCTTCATGCTC | 360 |
| SERTAD2-P | ATGTTGGGTAAAGGAGGAAAACGG | 50 |
| PARL-F | CATCTTGGGGGAGCTCTTTTTGG | 360 |
| PARL-R | CACCACTACTGTCCAATCCCAGT | 360 |
| PARL-P | GGAAGAACAGGGAGCCGCTAG | 50 |
| PAFAH2-F | CGGGCCATGTTGGCCTTC | 360 |
| PAFAH2-R | CTGGGGTGAGCGACGGT | 360 |
| PAFAH2-P | CAGAAGCACCTCGACCTGAAAG | 50 |
| SORT1 | GATGCTTTGGACACAGCCTCCC | 360 |
| SORT1 | TGCTGGGTCCAGCTCCTCTG | 360 |
| SORT1 | GATGACTCAGATGAGGACCTCTTGG | 50 |
| OSBPL9 | GATCAGAACGAGTATGAATCCCGC | 360 |
| OSBPL9 | CCCACTGAATTTCCTTCTCCTTCC | 360 |
| OSBPL9 | ACTGAAGCAAAGCACAGGCTTG | 50 |

Gently mix the master mixes by flicking or by vortexing, and then pulse spin. Add 15 $\mu$L of qPCR Master Mix to each well at room temperature.

In a template area, add 130 $\mu$L of SeptiCyte Triage Test Diluent to each cDNA product from the RT Reaction. Reseal the plate tightly and vortex the plate to mix thoroughly.

Add 10 $\mu$L of diluted cDNA product to each well according to the plate layout.

Mix by gentle pipetting. Avoid forming bubbles in the wells.

Cover wells with an optical seal.

Spin the plate to remove any bubbles (1 minute at 400 $\times$ g).

Place on real-time thermal cycler pre-programmed with the settings above.

Click Start. Click Save and Continue. Before leaving the instrument, it is recommended to verify that the run started successfully by displaying a time under Estimated Time Remaining.

Note: Do not open the qPCR plate at any point after amplification has begun. When amplification has completed, discard the unopened plate.

<u>Software. Interpretation of Results and Quality Control</u>

Software is specifically designed to integrate with the output of PCR machines and to apply an algorithm based on the use of multiple biomarkers. The software takes into account appropriate controls and reports results in a desired format.

When the run has completed on the ABI 7500 Fast Dx Instrument, complete the steps below in the application 7500 Fast System with 21 CFR Part 11 Software, ABI software SDS v1.4.

Click on the Results tab in the upper left corner.

Click on the Amplification Plot tab in the upper left corner.

In the Analysis Settings area, select an auto baseline and manual threshold for all targets. Enter 0.01 as the threshold.

Click on the Analyse button on the right in the Analysis Settings area.

From the menu bar in the upper left, select File then Close.

Complete the form in the dialog box that requests a reason for the change. Click OK.

Transfer the data file (.sds) to a separate computer running the specific assay RT-qPCR Test Software.

Launch the assay RT-qPCR Test Software. Log in.

From the menu bar in the upper left, select File then Open.

Browse to the location of the transferred data file (.sds). Click OK.

The data file will then be analysed using the assay's software application for interpretation of results.

Interpretation of Results and Quality Control

*Results*

Launch the interpretation software. Software application instructions are provided separately.

Following upload of the .sds file, the Software will automatically generate classifier scores for controls and clinical specimens.

Controls

The Software compares each CON (control) specimen (CONH, CONL, CONN) to its expected result. The controls are run in singleton.

| Control specimen | | |
|---|---|---|
| Designation | Name | Expected result |
| CONH | High Control | Score range |
| CONL | Low Control | Score range |
| CONN | Negative Control | Score range |
| NTC | No Template Control | Fail (no Ct for all targets) |

If CONH, CONL, and/or CONN fail the batch run is invalid and no data will be reported for the clinical specimens. This determination is made automatically by the interpretive software. The batch run should be repeated starting with either a new RNA preparation or starting at the RT reaction step.

If NTC yields a result other than Fail (no Ct for all targets), the batch run is invalid and no data may be reported for the clinical specimens. This determination is made by visual inspection of the run data. The batch run should be repeated starting with either a new RNA preparation or starting at the RT reaction step.

If a second batch run fails, please contact technical services. If both the calibrations and all controls are valid, then the batch run is valid and specimen results will be reported.

Specimens

Note that a valid batch run may contain both valid and invalid specimen results.

Analytical criteria (e.g. Ct values) that qualify each specimen as passing or failing (using pre-determined data) are called automatically by the software.

Scores out of range - reported.

Quality Control

Singletons each of the Negative Control, Low Positive Control, and High Positive Control must be included in each batch run. The batch is valid if no flags appear for any of these controls.

A singleton of the No Template Control is included in each batch run and Fail (no Ct for all targets) is a valid result indicating no amplifiable material was detectable in the well.

The negative control must yield a Negative result. If the negative control is flagged as Invalid, then the entire batch run is invalid.

The low positive and high positive controls must fall within the assigned ranges. If one or both of the positive controls are flagged as Invalid, then the entire batch run is invalid.

EXAMPLE 10

**EXAMPLE OUTPUT**

A possible example output from the software for a RO BaSIRS assay is presented Figure 6. The format of such a report depends on many factors including; quality control, regulatory authorities, cut-off values, the algorithm used, laboratory and clinician requirements, likelihood of misinterpretation.

In this instance the assay is called "SeptiCyte Triage". The result is reported as a number (5.9), a position on a 0-10 scale, and a probability of the patient having an absence of BaSIRS, or not, based on historical results and the use of a pre-determined cut-off (using results from clinical studies). Results of controls within the assay may also be reported. Other information that could be reported might include: previous results and date and time of such results, a prognosis, a scale that provides cut-off values for historical testing results that separate the conditions of healthy, non-bacterial SIRS and BaSIRS such that those patients with higher scores are considered to have more severe BaSIRS. The reporting of results in this fashion would allow clinicians to see the probability of a patient having BaSIRS to enable ruling out BaSIRS with confidence.

EXAMPLE 11

**SECOND EXAMPLE WORKFLOW**

A second example workflow will now be described. Machines have been, and are being, developed that are capable of processing a patient sample at point-of-care, or near point-of-care. Such machines require few molecular biology skills to run and are aimed at non-technical users. The idea is that the sample would be pipetted directly into a disposable cartridge(s) that is/are then inserted into the machine. For determining a specific host response the cartridge will need to extract high quality RNA from the cells in the sample for use with an appropriately designed composition to allow reverse transcription followed by RT-PCR. The machines are designed for minimum user interaction such that the user presses "Start" and within 1-3 hours results are generated. The cartridges contains all of the required reagents to perform host cell nucleic acid extraction (RNA), reverse transcription, and qRT-PCR, and the machine has appropriate software incorporated to allow use of algorithms to interpret each result and combine results, and final interpretation and printing of results.

Fresh, whole, anti-coagulated blood can be pipetted into a specialized cartridge (e.g. cartridges designed for Enigma ML machine by Enigma Diagnostics Limited (Enigma Diagnostics Limited, Building 224, Tetricus Science Park, Dstl, Porton Down, Salisbury, Wiltshire SP4 0JQ) or similar (Unyvero, Curetis AG, Max-Eyth-Str. 42 71088 Holzgerlingen, Germany)), and on-screen instructions followed to test for differentiating a BaSIRS from other forms of SIRS. Inside the machine RNA is first extracted from the whole blood and is then converted into cDNA. The cDNA is then used in qRT-PCR reactions. The reactions are followed in real time and Ct values calculated. On-board software generates a result output (see, Figure 6). Appropriate quality control measures for RNA quality, no template controls, high and low template controls and expected Ct ranges ensure that results are not reported erroneously.

EXAMPLE 12

**EXAMPLE ALGORITHM COMBINING DERIVED BIOMARKERS FOR ASSESSING A SUSPECTED BASIRS**

Derived biomarkers can be used in combination to increase the diagnostic power for separating various conditions. Determining which markers to use, and how many, for separating various conditions can be achieved by calculating Area Under Curve (AUC).

Biomarker ratios (derived markers) can be used in combination to increase the diagnostic power for separating BaSIRS and SIRS due to other causes. Determining which markers to use, and how many, for separating various conditions can be achieved by calculating Area Under Curve (AUC).

Figure 4 shows the effect on AUC (in this instance for separating BaSIRS and SIRS due to other causes) of adding derived biomarkers to the diagnostic signature for separating subjects with and without BaSIRS in Gene Expression Omnibus (GEO) datasets. Diagnostic power (as measured by AUC, Y axis) of a single derived biomarker starts at around 0.86 and increases as derived markers are added to a maximum of around 0.96. However, beyond the use of three derived markers (AUC ~ 0.94) there is likely overfitting, or introduction of noise. For commercial development of derived markers other factors come into play such as cost-effectiveness, assay complexity and capabilities of the qRT-PCR platform. In this example, the addition of derived biomarkers beyond three or four does not significantly improve performance, adds little additional information and likely runs the risk of data over-fitting and addition of noise. Thus, for commercial purposes, a combination of the three best derived markers provides a balance between maximal AUC and over-fitting.

As such, and by example, a six-biomarker signature (three derived biomarkers) offers the appropriate balance between simplicity, practicality and commercial risk for separating BaSIRS and SIRS due to other causes. Further, an equation using six biomarkers weighs each marker equally which also provides additional robustness in cases of analytical or clinical variability.

One example equation that provides good diagnostic power for separating BaSIRS and SIRS due to other causes is (where the value for each biomarker is a Ct value):

$$\text{“Diagnostic Score”} = (DIAPH2 - SERTAD2) + (PARL - PAFAH2) + (SORT1 - OSBPL9)$$

Box and whisker plots using these six biomarkers for six GEO datasets are shown in Figure 6 showing good separation between controls (lower box and whiskers - those subjects without BaSIRS) and cases (higher box and whiskers - those subjects with confirmed BaSIRS).

Note: each marker in the Diagnostic Score above is the Log 2 transformed concentration of the marker in the sample.

The disclosure of every patent, patent application, and publication cited herein is hereby incorporated herein by reference in its entirety.

The citation of any reference herein should not be construed as an admission that such reference is available as "Prior Art" to the instant application.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

## TABLES

Table 1: List and condition description of public datasets (GEO) used to find the best performing BaSIRS derived biomarkers for use in a triage setting, including the number of subjects in each cohort (in brackets).

| Dataset | Condition |
|---|---|
| GSE30119 | Staphylococcus aureus (99) vs Healthy (44) |
| GSE33341 | S. aureus, E coli (51) vs Healthy (43) |
| GSE16129 | S. aureus (42) vs Healthy (10) |
| GSE25504 | Bacterial (26) vs Healthy (37) |
| GSE40586 | Bacterial meningitis (21) vs Healthy (18) |
| GSE40012 | Bacterial pneumonia (19) vs Viral and SIRS (115) |

Table 2: List and condition description of public datasets (GEO) used to find the best performing non-bacterial SIRS derived biomarkers. These were then subtracted from the BaSIRS derived biomarkers identified from the datasets in Table 1. Note that other datasets were used to derive a set of specific viral derived biomarkers which were also subtracted from the BaSIRS derived biomarkers identified from the datasets in Table 1.

| Dataset | Description | Cases vs Controls | Overlapping Ratios with BaSIRS |
|---|---|---|---|
| GSE17755 | Arthritis; Lupus | 191 vs 53 | 2346 (8%) |
| GSE19301 | Asthma | 166 vs 394 | 1 (<1%) |
| GSE35846 | Race, gender, BMI | 190 vs 0 | 1180 (4%) |
| GSE36809 | Trauma | 167 vs 37 | 16944 (55%) |
| GSE38485 | Schizophrenia | 106 vs 96 | 13 (<1%) |
| GSE47655 | Anaphylaxis | 6 vs 5 | 733 (2%) |
| GSE52428 | Influenza | 41 vs 39 | 6401 (21%) |

Table 3: The mean cumulative performance (AUC) in the BaSIRS datasets of the derived biomarkers (that comprise the three derived biomarker signature) when each are added sequentially.

| Derived Biomarker | Cumulative AUC |
|---|---|
| DIAPH2 : SERTAD2 | 0.863 |
| PARL : PAFAH2 | 0.92 |
| SORT1 : OSBPL9 | 0.94 |

Table 4: Results of greedy searches to find the best performing derived biomarkers (when added sequentially up to 10) using the combined bacterial datasets. Three different cut-off values were used (AUC 70, 80 and 90) for derived biomarkers in the non-bacterial datasets. Using a low cut-off value in the non-bacterial datasets resulted in more derived biomarkers that were taken from the pool of derived biomarkers identified using the bacterial datasets. Hence, the total numbers of derived biomarkers remaining after subtraction were 92, 493 and 3257 for cut-off values of 70, 80 and 90 respectively. The best combination of derived biomarkers with the maximum AUC, maximum specificity, minimum noise and highest commercial utility was considered to be DIAPH2 / SERTAD2; PARL / PAFAH2; SORT1 / OSBPL9 obtained after the third greedy search iteration.

| Greedy Search Iteration | Cut-off | Mean AUC | Combination |
|---|---|---|---|
| 1 | 70 | 0.863 | DIAPH2_SERTAD2 |
| 2 | 70 | 0.920 | PARL_PAFAH2+DIAPH2_SERTAD2 |
| 3 | 70 | 0.940 | PARL_PAFAH2+SORT1_OSBPL9+DIAPH2_SERTAD2 |
| 4 | 70 | 0.952 | PARL_PAFAH2+SORT1_OSBPL9+CHPT1_RANBP10+DIAPH2_SERTAD2 |
| 5 | 70 | 0.956 | PARL_PAFAH2+SORT1_OSBPL9+FLVCR2_KATNA1+CHPT1_RANBP10+DIAPH2_SERTAD2 |
| 6 | 70 | 0.959 | PARL_PAFAH2+SORT1_OSBPL9+GAS7_RAB11FIP1 + FLVCR2_KATNA1+CHPT1_RANBP10+DIAPH2_SERTAD2 |
| 7 | 70 | 0.962 | PARL_PAFAH2+SORT1_OSBPL9+GAS7_RAB11FIP1+FLVCR2_KATNA1+CHPT1_RANBP10+ DIAPH2_SERTAD2+PRRG4 _GLOD4 |
| 8 | 70 | 0.963 | PARL_PAFAH2+SORT1_OSBPL9+GAS7_RAB11FIP1+FLVCR2_KATNA1+CHPT1_RANBP10+ DIAPH2_SERTAD2+PRRG4 _GLOD4+FURIN_RANBP10 |
| 9 | 70 | 0.964 | CHPT1_FBX07+PARL_ PAFAH2+SORT1_OSBPL9+GAS7_RAB11FIP1+FLVCR2_KATNA1+CHPT1_RANBP10+ DIAPH2_S ERTAD2+PRRG4_GLOD4+FURIN_RANBP10 |
| 10 | 70 | 0.965 | CHPT1_FBX07+PARL_ PAFAH2+SORT1_OSBPL9+GAS7_RAB11FIP1+SORT1_CNBP+FLVCR2_KATNA1+CH PT1_RANBP 10+DIAPH2_SERTAD2+PRRG4_GLOD4+FURIN_RANBP10 |
| 1 | 80 | 0.873 | SLC25A28_ID3 |
| 2 | 80 | 0.917 | SLC25A28_ID3+SORT1_INPP1 |
| 3 | 80 | 0.937 | DIAPH2_SERTAD2+SLC25A28_ID3+SORT1_INPP1 |
| 4 | 80 | 0.947 | RHEB_IMP3+DIAPH2_SERTAD2+SLC25A28_ID3+SORT1_INPP1 |
| 5 | 80 | 0.952 | DIAPH2_CRLF3+RHEB_IMP3+DIAPH2_SERTAD2+SLC25A28_ID3+SORT1_INPP1 |

| Greedy Search Iteration | Cut-off | Mean AUC | Combination |
|---|---|---|---|
| 6 | 80 | 0.956 | DIAPH2_CRLF3+RHEB_ IMP3+DIAPH2_SERTAD2+HOXB6_PAFAH2+SLC25A28_ID3+SORT1_INPP1 |
| 7 | 80 | 0.959 | CHPT1_FBXO7+DIAPH2_CRLF3+RHEB_ IMP3+DIAPH2_SERTAD2+HOXB6_PAFAH2+SLC25A28_ID3+SORT1_INPP1 |
| 8 | 80 | 0.961 | CHPT1_FBX07+DIAPH2_CRLF3+SORT1_OSBPL9+RHEB_ IMP3+DIAPH2_SERTAD2+HOXB6_PAFAH2+SLC25A28_ID3 |
| | | | +SORT1_INPP1 |
| 9 | 80 | 0.963 | CHPT1_FBXO7+DIAPH2_CRLF3+SMPDL3A_BTG2+SORT1_OSBPL9+RHEB_ IMP3+DIAPH2_SERTAD2+HOXB6_PAFAH 2+SLC25A28_ID3+SORT1_INPP1 |
| 10 | 80 | 0.965 | CHPT1_FBXO7+DIAPH2_CRLF3+SMPDL3A_BTG2+SORT1_OSBPL9+RHEB_ IMP3+DIAPH2_SERTAD2+HOXB6_PAFAH 2+FURIN_ RANBP10+SLC25A28_ID3+SORT1_INPP1 |
| 1 | 90 | 0.893 | DIAPH2_PAFAH2 |
| 2 | 90 | 0.927 | SORT1_OSBPL9+DIAPH2_PAFAH2 |
| 3 | 90 | 0.944 | SORT1_OSBPL9+CHPT1_RANBP10+DIAPH2_PAFAH2 |
| 4 | 90 | 0.950 | SORT1_OSBPL9+CHPT1_RANBP10+SMPDL3A_SYPL1+DIAPH2_PAFAH2 |
| 5 | 90 | 0.954 | SORT1_OSBPL9+GAS7_RAB11FIP1+CHPT1_RANBP10+SMPDL3A_ SYPL1+DIAPH2_PAFAH2 |
| 6 | 90 | 0.956 | SORT1_OSBPL9+GAS7_RAB11FIP1+HIST1H2BK_ WDR33+CHPT1_RANBP10+SMPDL3A_SYPL1+DIAPH2_PAFAH2 |
| 7 | 90 | 0.959 | SORT1_OSBPL9+GAS7_RAB11FIP1+HIST1H2BK_ WDR33+CHPT1_RANBP10+SMPDL3A_SYPL1+DIAPH2_CCR3+DIAP H2_PAFAH2 |
| 8 | 90 | 0.960 | SORT1_OSBPL9+GAS7_RAB11FIP1+HIST1H2BK_WDR33+CHPT1_RANBP10+MUT_ ACTL6A+SMPDL3A_SYPL1+DIAP H2_CCR3+DIAPH2_PAFAH2 |
| 9 | 90 | 0.962 | SORT1_OSBPL9+GAS7_RAB11FIP1+FLVCR2_KATNA1+HIST1H2BK_ WDR33+CHPT1_RANBP10+MUT_ACTL6A+SMPD L3A_ SYPL1+DIAPH2_CCR3+DIAPH2_PAFAH2 |

(continued)

| Greedy Search Iteration | Cut-off | Mean AUC | Combination |
|---|---|---|---|
| 10 | 90 | 0.964 | SORT1_OSBPL9+GAS7_RAB11FIP1+FLVCR2_KATNAI+HIST1H2BK_ WDR33+CHPT1_RANBP10+ MUT_ACTL6A+SMPD L3A_ SYPL1+SORT1_AKAP7+DIAPH2_CCR3+DIAPH2_PAFAH2 |

Table 5 (a and b): Groups of derived biomarkers (A-F) based on their correlation to each individual biomarker in the three derived biomarker signature of DIAPH2 / SERTAD2; PARL / PAFAH2; SORT1 / OSBPL9. Groups A-C are contained in Table 5a and Groups D-F are contained in Table 5b. A DNA SEQ ID# is provided for each biomarker HUGO gene symbol.

Table 5a

| Group A | | | Group B | | | Group C | | |
|---|---|---|---|---|---|---|---|---|
| Symbol | DNA SEQ ID | Correlation to DIAPH2 | Symbol | DNA SEQ ID | Correlation to SERTAD2 | Symbol | DNA SEQ ID | Correlation to PARL |
| CYBB | 36 | 0.626 | PHF3 | 115 | 0.380 | AIF1 | 4 | 0.516 |
| FXR1 | 53 | 0.351 | BRD7 | 15 | 0.327 | PTPN2 | 128 | 0.478 |
| GBP2 | 56 | 0.280 | TOB1 | 172 | 0.326 | COX5B | 30 | 0.439 |
| HIST1H2BM | 63 | 0.141 | MAP4K2 | 90 | 0.285 | PSMB4 | 126 | 0.429 |
| HIST1H4L | 65 | 0.061 | WDR33 | 177 | 0.269 | EIF4E2 | 43 | 0.426 |
| MAPK8IP3 | 92 | 0.289 | BTG2 | 16 | 0.243 | RDX | 137 | 0.403 |
| MNT | 99 | 0.395 | AMD1 | 9 | 0.237 | DERA | 38 | 0.376 |
| MUT | 101 | 0.421 | RNASE6 | 138 | 0.224 | CTSH | 35 | 0.366 |
| NAGK | 105 | 0.125 | RAB11FIP1 | 130 | 0.183 | HSPA4 | 70 | 0.292 |
| NMI | 106 | 0.419 | ADD1 | 2 | 0.158 | VAV1 | 176 | 0.240 |
| PPP1CB | 121 | 0.239 | HMG20B | 67 | 0.139 | PPP1CA | 120 | 0.223 |
| PRPF40A | 124 | 0.469 | | | | CPVL | 31 | 0.223 |
| PRRG4 | 125 | 0.291 | | | | PDCD5 | 112 | 0.191 |
| PUS3 | 129 | 0.417 | | | | | | |
| SLAMF7 | 152 | 0.290 | | | | | | |
| SLC11A2 | 153 | 0.388 | | | | | | |
| SLC39A8 | 157 | 0.510 | | | | | | |
| SNAPC1 | 159 | 0.317 | | | | | | |
| TMEM80 | 171 | 0.215 | | | | | | |

Table 5b

| Group D | | | Group E | | | Group F | | |
|---|---|---|---|---|---|---|---|---|
| Symbol | DNA SEQ ID | Correlation to PAFAH2 | Symbol | DNA SEQ ID | Correlation to SORT1 | Symbol | DNA SEQ ID | Correlation to OSBPL9 |
| IMP3 | 75 | 0.651 | GAS7 | 55 | 0.765 | CD44 | 20 | 0.487 |
| GLOD4 | 58 | 0.634 | FLVCR2 | 49 | 0.762 | AKTIP | 7 | 0.477 |
| IL7R | 74 | 0.632 | TLR5 | 169 | 0.761 | ATP13A3 | 12 | 0.473 |
| ID3 | 71 | 0.604 | FCER1G | 47 | 0.753 | ADAM19 | 1 | 0.429 |
| KLRF1 | 83 | 0.604 | SLC2A3 | 156 | 0.740 | KATNA1 | 81 | 0.408 |
| SBF1 | 146 | 0.573 | S100A12 | 143 | 0.730 | STK38 | 164 | 0.407 |
| CCND2 | 19 | 0.563 | PSTPIP2 | 127 | 0.709 | TINF2 | 168 | 0.326 |
| LFNG | 85 | 0.562 | GNS | 59 | 0.698 | RAB9A | 132 | 0.321 |
| MRPS18B | 100 | 0.551 | METTL9 | 95 | 0.695 | INPP1 | 77 | 0.261 |
| HLA-DPA1 | 66 | 0.545 | MMP8 | 98 | 0.685 | CNBP | 27 | 0.240 |
| SLC9A3R1 | 158 | 0.542 | MAPK14 | 91 | 0.678 | ITGB1 | 80 | 0.193 |
| HMGN4 | 68 | 0.537 | CD59 | 21 | 0.675 | MFSD10 | 96 | 0.173 |
| C6orf48 | 18 | 0.529 | CLEC4E | 25 | 0.673 | | | |
| ARL2BP | 10 | 0.522 | MICAL1 | 97 | 0.658 | | | |
| CDC14A | 22 | 0.517 | MCTP1 | 93 | 0.644 | | | |
| RPA2 | 140 | 0.503 | GAPDH | 54 | 0.640 | | | |
| ST3GAL5 | 162 | 0.502 | IMPDH1 | 76 | 0.638 | | | |
| EIF4A2 | 42 | 0.501 | ATP8B4 | 14 | 0.623 | | | |
| CERK | 23 | 0.496 | EMR1 | 44 | 0.618 | | | |
| RASSF7 | 136 | 0.491 | SLC12A9 | 154 | 0.610 | | | |
| PHB | 114 | 0.488 | S100P | 144 | 0.603 | | | |
| TRAF3IP2 | 174 | 0.487 | IFNGR2 | 73 | 0.594 | | | |
| KLF2 | 82 | 0.485 | PDGFC | 113 | 0.592 | | | |
| RAB11FIP3 | 131 | 0.476 | CTSA | 33 | 0.559 | | | |

(continued)

| Group D | | | Group E | | | Group F | | |
|---|---|---|---|---|---|---|---|---|
| Symbol | DNA SEQ ID | Correlation to PAFAH2 | Symbol | DNA SEQ ID | Correlation to SORT1 | Symbol | DNA SEQ ID | Correlation to OSBPL9 |
| C21orf59 | 17 | 0.475 | ALDOA | 8 | 0.552 | | | |
| SSBP2 | 161 | 0.473 | ITGAX | 79 | 0.549 | | | |
| GIMAP4 | 57 | 0.437 | GSTO1 | 61 | 0.545 | | | |
| CYP20A1 | 37 | 0.428 | LHFPL2 | 86 | 0.526 | | | |
| RASGRP2 | 134 | 0.427 | LTF | 89 | 0.515 | | | |
| AKT1 | 6 | 0.413 | SDHC | 148 | 0.493 | | | |
| HCP5 | 61 | 0.388 | TIMP1 | 167 | 0.484 | | | |
| TPP2 | 173 | 0.386 | LTA4H | 88 | 0.474 | | | |
| SYNE2 | 165 | 0.383 | USP3 | 175 | 0.460 | | | |
| FUT8 | 52 | 0.369 | MEGF9 | 94 | 0.456 | | | |
| NUPL2 | 107 | 0.361 | FURIN | 51 | 0.442 | | | |
| MYOM2 | 104 | 0.360 | ATP6V0A1 | 13 | 0.425 | | | |
| RPS8 | 142 | 0.355 | PROS1 | 123 | 0.424 | | | |
| RNF34 | 139 | 0.342 | ATG9A | 11 | 0.398 | | | |
| DLST | 41 | 0.329 | PLAC8 | 116 | 0.394 | | | |
| CTDSP2 | 32 | 0.310 | LAMP1 | 84 | 0.393 | | | |
| EMP3 | 44 | 0.306 | COQ10B | 29 | 0.393 | | | |
| PLEKHG3 | 117 | 0.274 | ST3GAL6 | 163 | 0.391 | | | |
| DHX16 | 39 | 0.271 | CTSC | 34 | 0.391 | | | |
| RASGRP3 | 135 | 0.232 | ENO1 | 45 | 0.389 | | | |
| COMMD4 | 28 | 0.223 | OBFC1 | 108 | 0.382 | | | |
| ISG20 | 78 | 0.222 | TAX1BP1 | 166 | 0.375 | | | |
| POLR2C | 119 | 0.204 | MYL9 | 103 | 0.350 | | | |
| SH3GLB2 | 151 | 0.187 | HIST1H3C | 64 | 0.287 | | | |

| Group D | | | Group E | | | Group F | | |
|---|---|---|---|---|---|---|---|---|
| Symbol | DNA SEQ ID | Correlation to PAFAH2 | Symbol | DNA SEQ ID | Correlation to SORT1 | Symbol | DNA SEQ ID | Correlation to OSBPL9 |
| SASH3 | 145 | 0.182 | ZBTB17 | 179 | 0.281 | | | |
| GRAP2 | 60 | 0.157 | CHPT1 | 24 | 0.279 | | | |
| RPS6KB2 | 141 | 0.154 | SLC25A37 | 155 | 0.266 | | | |
| FGL2 | 48 | 0.154 | PLEKHM2 | 118 | 0.266 | | | |
| AKAP7 | 5 | 0.141 | LILRB3 | 87 | 0.261 | | | |
| SDF2L1 | 147 | 0.136 | YPEL5 | 178 | 0.226 | | | |
| FBXO7 | 46 | 0.116 | FTL | 50 | 0.205 | | | |
| MX1 | 102 | 0.112 | SH3BGRL | 150 | 0.163 | | | |
| IFIT1 | 72 | 0.062 | HOXB6 | 69 | 0.144 | | | |
| TMEM106C | 170 | 0.057 | PPP1R11 | 122 | 0.139 | | | |
| RANBP10 | 133 | 0.045 | CLU | 26 | 0.136 | | | |
| | | | HEBP1 | 62 | 0.125 | | | |

EP 3 998 345 A1

54

Table 6: Performance of 200 derived biomarkers at a set sepsis prevalence of 10%. Performance measures include Area Under Curve (AUC) and Negative Predictive Value (NPV). The NPV of these derived biomarkers increases as the prevalence of sepsis decreases, so all those listed would perform well in an emergency room setting where the prevalence of sepsis is estimated to be closer to 4% (see Table 7).

| Derived Biomarker | AUC | NPV | AUC (upper) | AUC (lower) | NPV (lower) | NPV (upper) |
|---|---|---|---|---|---|---|
| AIF1_HMGN4 | 0.809 | 94.599 | 0.697 | 0.905 | 92.208 | 97.370 |
| ALDOA_MAP4K2 | 0.813 | 95.000 | 0.663 | 0.936 | 95.000 | 100.000 |
| ATG9A_RAB11FIP3 | 0.802 | 95.254 | 0.698 | 0.889 | 92.203 | 98.649 |
| ATP13A3_IL7R | 0.839 | 95.044 | 0.707 | 0.936 | 92.303 | 97.648 |
| ATP6V0A1_RASSF7 | 0.808 | 94.884 | 0.673 | 0.913 | 91.856 | 97.532 |
| ATP8B4_CCND2 | 0.833 | 94.937 | 0.734 | 0.923 | 92.396 | 97.562 |
| CD44 GIMAP4 | 0.748 | 95.034 | 0.591 | 0.911 | 90.562 | 100.000 |
| CD44_HLA-DPA1 | 0.751 | 95.065 | 0.576 | 0.897 | 91.661 | 98.552 |
| CD44_IL7R | 0.781 | 95.030 | 0.652 | 0.906 | 92.500 | 97.503 |
| CD44_RPA2 | 0.760 | 95.100 | 0.625 | 0.917 | 92.578 | 98.552 |
| CD59_GIMAP4 | 0.803 | 94.977 | 0.638 | 0.925 | 91.765 | 97.532 |
| CDC14A_CCND2 | 0.587 | 92.613 | 0.439 | 0.719 | 66.667 | 100.000 |
| CDC14A_IL7R | 0.611 | 93.349 | 0.456 | 0.766 | 80.000 | 100.000 |
| CHPT1_FBXO7 | 0.819 | 94.665 | 0.715 | 0.929 | 91.949 | 97.720 |
| CHPT1_RANBP10 | 0.800 | 95.205 | 0.672 | 0.914 | 92.400 | 97.531 |
| CLEC4E_MX1 | 0.718 | 94.976 | 0.551 | 0.877 | 91.070 | 98.279 |
| CLEC4E_SYNE2 | 0.793 | 94.971 | 0.673 | 0.922 | 91.775 | 98.651 |
| CLU_CCND2 | 0.758 | 94.854 | 0.613 | 0.870 | 91.341 | 97.468 |
| CLU_IL7R | 0.760 | 94.633 | 0.643 | 0.868 | 91.543 | 97.260 |
| COQ10B_TRAF3IP2 | 0.799 | 94.810 | 0.650 | 0.923 | 91.566 | 97.590 |
| COX5B_PHB | 0.832 | 94.855 | 0.730 | 0.926 | 92.308 | 97.677 |
| CPVL_IL7R | 0.677 | 94.613 | 0.539 | 0.789 | 80.000 | 100.000 |
| CTSA_DLST | 0.822 | 94.991 | 0.678 | 0.933 | 92.456 | 97.620 |
| CTSA_HMG20B | 0.821 | 94.629 | 0.706 | 0.936 | 92.130 | 97.592 |
| CTSC_CCND2 | 0.799 | 95.082 | 0.642 | 0.907 | 92.016 | 97.588 |
| CTSH_IL7R | 0.787 | 95.016 | 0.661 | 0.885 | 92.000 | 97.608 |
| CYBB_BRD7 | 0.805 | 95.164 | 0.667 | 0.915 | 75.000 | 100.000 |
| DERA_HMGN4 | 0.827 | 94.719 | 0.718 | 0.935 | 91.860 | 97.648 |
| DIAPH2_CCND2 | 0.894 | 95.000 | 0.805 | 0.964 | 95.000 | 100.000 |
| DIAPH2_HLA-DPA1 | 0.862 | 94.896 | 0.749 | 0.953 | 91.574 | 97.620 |
| DIAPH2_IL7R | 0.871 | 94.999 | 0.777 | 0.948 | 92.130 | 97.647 |
| DIAPH2_PHF3 | 0.822 | 94.627 | 0.727 | 0.911 | 91.856 | 97.438 |
| DIAPH2_RAB9A | 0.806 | 94.974 | 0.669 | 0.918 | 92.041 | 97.619 |
| DIAPH2_RNASE6 | 0.792 | 95.025 | 0.668 | 0.898 | 91.667 | 97.335 |
| DIAPH2_SERTAD2 | 0.857 | 94.759 | 0.726 | 0.935 | 91.860 | 97.593 |

(continued)

| Derived Biomarker | AUC | NPV | AUC (upper) | AUC (lower) | NPV (lower) | NPV (upper) |
|---|---|---|---|---|---|---|
| DIAPH2_ST3GAL5 | 0.818 | 94.839 | 0.683 | 0.941 | 92.130 | 97.592 |
| DIAPH2_STK38 | 0.795 | 95.399 | 0.671 | 0.880 | 91.539 | 98.572 |
| EIF4E2_C21orf59 | 0.821 | 95.255 | 0.717 | 0.923 | 92.098 | 98.685 |
| EMR1_AKT1 | 0.786 | 94.876 | 0.667 | 0.891 | 91.775 | 98.571 |
| EN01_IL7R | 0.803 | 94.555 | 0.653 | 0.903 | 91.886 | 97.468 |
| FCER1G_CD44 | 0.740 | 95.150 | 0.596 | 0.858 | 90.909 | 98.439 |
| FCER1G_CDC14A | 0.847 | 95.200 | 0.751 | 0.932 | 92.130 | 97.592 |
| FCER1G_MX1 | 0.780 | 94.926 | 0.566 | 0.931 | 91.837 | 97.678 |
| FCER1G_SDHC | 0.673 | 95.181 | 0.523 | 0.789 | 88.868 | 100.000 |
| FLVCR2_KATNA1 | 0.837 | 95.209 | 0.700 | 0.947 | 92.045 | 97.802 |
| FTL_CCND2 | 0.748 | 95.191 | 0.634 | 0.868 | 91.803 | 98.662 |
| FTL_IL7R | 0.770 | 95.203 | 0.654 | 0.872 | 92.000 | 98.509 |
| FURIN_ADD1 | 0.824 | 95.043 | 0.694 | 0.931 | 92.203 | 97.500 |
| FURIN_BTG2 | 0.830 | 94.872 | 0.733 | 0.918 | 92.771 | 97.590 |
| FURIN_RANBP10 | 0.824 | 94.819 | 0.716 | 0.912 | 92.573 | 97.373 |
| FURIN_SH3GLB2 | 0.826 | 95.000 | 0.693 | 0.938 | 95.000 | 100.000 |
| FUT8_IL7R | 0.593 | 94.288 | 0.431 | 0.764 | 80.000 | 100.000 |
| FXR1_EIF4A2 | 0.816 | 95.052 | 0.690 | 0.927 | 91.760 | 97.561 |
| GAPDH_COMMD4 | 0.794 | 95.061 | 0.622 | 0.922 | 92.295 | 97.778 |
| GAPDH_PPP1CA | 0.802 | 94.851 | 0.684 | 0.915 | 91.358 | 98.668 |
| GAPDH_RPS6KB2 | 0.789 | 95.186 | 0.640 | 0.919 | 91.775 | 98.701 |
| GAS7_ADD1 | 0.809 | 94.939 | 0.672 | 0.935 | 91.561 | 97.698 |
| GAS7_RAB11FIP1 | 0.855 | 95.397 | 0.719 | 0.950 | 92.748 | 97.648 |
| GBP2_GIMAP4 | 0.808 | 95.089 | 0.657 | 0.926 | 92.593 | 97.590 |
| GBP2_HCP5 | 0.796 | 94.955 | 0.684 | 0.913 | 91.765 | 98.630 |
| GBP2_MX1 | 0.709 | 94.867 | 0.533 | 0.858 | 87.097 | 100.000 |
| GNS_PLEKHG3 | 0.833 | 95.069 | 0.726 | 0.937 | 92.126 | 97.676 |
| GSTO1_RASGRP3 | 0.802 | 94.904 | 0.654 | 0.923 | 91.667 | 97.619 |
| GSTO1_SDF2L1 | 0.793 | 94.946 | 0.668 | 0.913 | 79.750 | 100.000 |
| HEBP1_SSBP2 | 0.824 | 95.019 | 0.690 | 0.926 | 91.238 | 97.620 |
| HIST1H2BM_CCND2 | 0.757 | 95.085 | 0.622 | 0.858 | 92.374 | 98.463 |
| HIST1H2BM_IL7R | 0.755 | 94.864 | 0.620 | 0.865 | 91.524 | 98.462 |
| HIST1H3C_IL7R | 0.804 | 95.337 | 0.701 | 0.897 | 92.758 | 98.593 |
| HOXB6_PAFAH2 | 0.832 | 94.632 | 0.728 | 0.910 | 91.517 | 97.502 |
| HSPA4_IMP3 | 0.820 | 94.753 | 0.704 | 0.904 | 92.102 | 97.404 |
| IFNGR2_CCND2 | 0.841 | 95.248 | 0.715 | 0.931 | 92.400 | 97.562 |
| IFNGR2_HLA-DPA1 | 0.812 | 94.909 | 0.663 | 0.935 | 91.954 | 98.687 |

(continued)

| Derived Biomarker | AUC | NPV | AUC (upper) | AUC (lower) | NPV (lower) | NPV (upper) |
|---|---|---|---|---|---|---|
| IFNGR2_IL7R | 0.823 | 94.941 | 0.684 | 0.918 | 91.945 | 97.561 |
| IMPDH1_BTG2 | 0.837 | 95.128 | 0.701 | 0.940 | 93.231 | 97.701 |
| ITGAX_RASGRP2 | 0.791 | 95.077 | 0.668 | 0.892 | 92.303 | 97.468 |
| ITGB1_IL7R | 0.708 | 95.114 | 0.571 | 0.846 | 83.284 | 100.000 |
| LAMP1_HLA-DPA1 | 0.760 | 94.964 | 0.598 | 0.895 | 91.667 | 98.630 |
| LAMP1_IL7R | 0.775 | 94.949 | 0.635 | 0.900 | 75.000 | 100.000 |
| LHFPL2_ISG20 | 0.804 | 94.887 | 0.678 | 0.899 | 91.635 | 97.502 |
| LILRB3_IL7R | 0.816 | 94.833 | 0.684 | 0.926 | 79.167 | 100.000 |
| LTA4H_CCND2 | 0.812 | 95.186 | 0.682 | 0.921 | 92.176 | 98.595 |
| LTA4H_CERK | 0.809 | 95.000 | 0.706 | 0.905 | 95.000 | 100.000 |
| LTA4H_CPVL | 0.761 | 94.870 | 0.628 | 0.860 | 91.983 | 97.470 |
| LTA4H_RPS8 | 0.847 | 94.744 | 0.725 | 0.948 | 92.473 | 96.703 |
| LTA4H_ST3GAL5 | 0.760 | 95.048 | 0.583 | 0.903 | 91.347 | 97.583 |
| LTA4H_TMEM106C | 0.708 | 95.071 | 0.566 | 0.836 | 90.843 | 98.173 |
| LTA4H_WDR33 | 0.784 | 95.012 | 0.664 | 0.916 | 91.549 | 98.632 |
| LTF_MAP4K2 | 0.777 | 95.138 | 0.624 | 0.909 | 91.949 | 98.684 |
| MAPK14 GIMAP4 | 0.827 | 95.325 | 0.711 | 0.909 | 92.395 | 97.500 |
| MAPK14_IL7R | 0.836 | 94.894 | 0.710 | 0.917 | 91.954 | 97.534 |
| MAPK14_MX1 | 0.715 | 94.947 | 0.568 | 0.868 | 92.423 | 98.465 |
| MAPK8IP3_IMP3 | 0.819 | 95.259 | 0.705 | 0.932 | 92.551 | 98.670 |
| MCTP1_AMD1 | 0.845 | 95.150 | 0.699 | 0.957 | 74.583 | 100.000 |
| MCTP1_TOB1 | 0.841 | 95.250 | 0.733 | 0.933 | 92.209 | 98.719 |
| MEGF9_CCND2 | 0.785 | 95.203 | 0.656 | 0.907 | 92.830 | 97.531 |
| MEGF9_CDC14A | 0.754 | 95.057 | 0.628 | 0.895 | 71.429 | 100.000 |
| MEGF9_GIMAP4 | 0.763 | 94.755 | 0.609 | 0.893 | 91.760 | 97.588 |
| MEGF9_HLA-DPA1 | 0.758 | 95.055 | 0.589 | 0.900 | 92.762 | 97.590 |
| MEGF9_IL7R | 0.786 | 95.082 | 0.640 | 0.893 | 92.857 | 97.674 |
| METTL9_AKTIP | 0.808 | 95.102 | 0.680 | 0.904 | 91.876 | 97.593 |
| MICAL1_DHX16 | 0.805 | 94.977 | 0.680 | 0.903 | 92.195 | 97.470 |
| MICAL1_STK38 | 0.801 | 95.014 | 0.692 | 0.905 | 92.405 | 97.526 |
| MMP8_CCND2 | 0.851 | 94.945 | 0.744 | 0.946 | 91.954 | 97.701 |
| MMP8_CD44 | 0.681 | 95.171 | 0.550 | 0.817 | 89.617 | 100.000 |
| MMP8_CTSC | 0.628 | 93.515 | 0.476 | 0.781 | 90.284 | 96.777 |
| MMP8_ENO1 | 0.657 | 94.033 | 0.498 | 0.810 | 89.186 | 97.738 |
| MMP8_FGL2 | 0.745 | 95.048 | 0.598 | 0.892 | 91.892 | 98.554 |
| MMP8_FTL | 0.675 | 95.472 | 0.527 | 0.840 | 88.199 | 100.000 |
| MMP8_FUT8 | 0.830 | 95.077 | 0.682 | 0.941 | 92.495 | 97.671 |

(continued)

| Derived Biomarker | AUC | NPV | AUC (upper) | AUC (lower) | NPV (lower) | NPV (upper) |
|---|---|---|---|---|---|---|
| MMP8_IL7R | 0.854 | 94.995 | 0.753 | 0.941 | 91.667 | 97.623 |
| MMP8_ITGB1 | 0.762 | 95.044 | 0.621 | 0.884 | 91.364 | 98.388 |
| MMP8_LAMP1 | 0.668 | 95.074 | 0.525 | 0.798 | 86.894 | 100.000 |
| MMP8_PLAC8 | 0.615 | 92.780 | 0.452 | 0.764 | 89.987 | 95.062 |
| MMP8_RPS8 | 0.845 | 94.742 | 0.711 | 0.947 | 92.218 | 96.591 |
| MMP8_SDHC | 0.626 | 94.167 | 0.465 | 0.773 | 66.667 | 100.000 |
| MMP8_ST3GAL5 | 0.783 | 95.070 | 0.646 | 0.895 | 92.194 | 97.468 |
| MMP8_TMEM106C | 0.792 | 95.070 | 0.678 | 0.897 | 91.755 | 97.503 |
| MMP8_TPP2 | 0.786 | 94.959 | 0.666 | 0.892 | 92.400 | 97.438 |
| MMP8_VAV1 | 0.644 | 94.122 | 0.489 | 0.787 | 87.500 | 100.000 |
| MNT_KLF2 | 0.823 | 94.677 | 0.704 | 0.904 | 91.856 | 97.375 |
| MNT_SLC9A3R1 | 0.797 | 94.996 | 0.662 | 0.918 | 91.561 | 98.702 |
| MUT_NUPL2 | 0.794 | 95.276 | 0.701 | 0.890 | 92.303 | 98.489 |
| MYL9_GRAP2 | 0.830 | 95.275 | 0.723 | 0.945 | 92.857 | 97.647 |
| MYL9_KLF2 | 0.828 | 95.000 | 0.729 | 0.921 | 95.000 | 100.000 |
| NMI_MX1 | 0.707 | 95.147 | 0.594 | 0.847 | 88.000 | 100.000 |
| OBFC1_C6orf48 | 0.789 | 95.003 | 0.668 | 0.909 | 92.651 | 97.561 |
| PARL_PAFAH2 | 0.832 | 94.723 | 0.703 | 0.925 | 91.949 | 97.697 |
| PDGFC_CCND2 | 0.853 | 95.770 | 0.724 | 0.940 | 93.182 | 97.701 |
| PDGFC_FUT8 | 0.823 | 94.969 | 0.705 | 0.924 | 92.853 | 97.647 |
| PDGFC_IL7R | 0.859 | 95.136 | 0.740 | 0.935 | 92.218 | 97.755 |
| PDGFC_ITGB1 | 0.796 | 94.980 | 0.705 | 0.891 | 92.396 | 97.503 |
| PLEKHM2_SBF1 | 0.832 | 94.929 | 0.685 | 0.925 | 92.649 | 97.675 |
| PPP1CB_PAFAH2 | 0.805 | 94.706 | 0.658 | 0.915 | 91.856 | 97.622 |
| PROS1_MYOM2 | 0.793 | 95.000 | 0.671 | 0.901 | 95.000 | 100.000 |
| PROS1_WDR33 | 0.786 | 94.905 | 0.684 | 0.869 | 92.303 | 97.338 |
| PRPF40A_MRPS18B | 0.782 | 94.822 | 0.641 | 0.906 | 91.860 | 97.470 |
| PRRG4_GLOD4 | 0.838 | 95.228 | 0.735 | 0.935 | 92.292 | 97.673 |
| PSMB4 IMP3 | 0.832 | 95.047 | 0.704 | 0.927 | 91.744 | 98.703 |
| PSTPIP2_AKAP7 | 0.816 | 94.930 | 0.687 | 0.925 | 91.662 | 97.438 |
| PTPN2_CYP20A1 | 0.819 | 95.000 | 0.679 | 0.913 | 95.000 | 100.000 |
| PUS3_PAFAH2 | 0.832 | 95.234 | 0.710 | 0.936 | 92.121 | 98.686 |
| S100A12_POLR2C | 0.780 | 95.052 | 0.665 | 0.885 | 91.892 | 98.510 |
| S100P_GIMAP4 | 0.798 | 95.217 | 0.637 | 0.932 | 74.583 | 100.000 |
| S100P_HLA-DPA1 | 0.802 | 94.985 | 0.675 | 0.907 | 91.453 | 97.403 |
| S100P_IL7R | 0.842 | 94.982 | 0.724 | 0.941 | 92.130 | 97.702 |
| SH3BGRL_GLOD4 | 0.790 | 95.143 | 0.627 | 0.924 | 91.765 | 98.593 |

(continued)

| Derived Biomarker | AUC | NPV | AUC (upper) | AUC (lower) | NPV (lower) | NPV (upper) |
|---|---|---|---|---|---|---|
| SLC11A2_ID3 | 0.845 | 95.105 | 0.747 | 0.921 | 92.222 | 97.619 |
| SLC12A9_CTDSP2 | 0.829 | 95.177 | 0.733 | 0.915 | 92.674 | 98.633 |
| SLC25A37_FBXO7 | 0.816 | 95.088 | 0.713 | 0.931 | 91.755 | 98.668 |
| SLC2A3_ADAM19 | 0.813 | 94.857 | 0.650 | 0.914 | 91.458 | 97.535 |
| SLC2A3_MFSD10 | 0.772 | 94.829 | 0.611 | 0.882 | 91.870 | 97.470 |
| SLC39A8_CCND2 | 0.865 | 94.713 | 0.759 | 0.952 | 91.489 | 97.676 |
| SLC39A8_IL7R | 0.852 | 95.299 | 0.719 | 0.938 | 91.949 | 97.620 |
| SLC39A8_LFNG | 0.806 | 94.936 | 0.655 | 0.921 | 92.378 | 97.439 |
| SLC39A8_WDR33 | 0.816 | 95.142 | 0.677 | 0.947 | 92.676 | 98.702 |
| SNAPC1_IL7R | 0.800 | 95.001 | 0.674 | 0.915 | 92.570 | 98.688 |
| SORT1_CNBP | 0.857 | 94.991 | 0.758 | 0.940 | 92.905 | 97.620 |
| SORT1_INPP1 | 0.811 | 94.668 | 0.698 | 0.910 | 92.027 | 96.631 |
| SORT1_NAGK | 0.801 | 94.573 | 0.688 | 0.909 | 92.093 | 97.372 |
| SORT1_OSBPL9 | 0.825 | 95.073 | 0.682 | 0.941 | 92.126 | 98.766 |
| SORT1_PDCD5 | 0.830 | 95.024 | 0.706 | 0.933 | 92.303 | 97.702 |
| SORT1_PPP1R11 | 0.792 | 95.009 | 0.682 | 0.926 | 91.463 | 97.531 |
| SORT1_SASH3 | 0.792 | 95.434 | 0.648 | 0.909 | 92.674 | 98.650 |
| SORT1_TINF2 | 0.795 | 94.799 | 0.650 | 0.917 | 91.954 | 97.561 |
| ST3GAL6_KLRF1 | 0.812 | 95.008 | 0.702 | 0.918 | 91.954 | 97.592 |
| TAX1BP1_NUPL2 | 0.802 | 94.928 | 0.689 | 0.901 | 91.458 | 97.503 |
| TIMP1_EMP3 | 0.804 | 95.149 | 0.694 | 0.903 | 92.308 | 98.631 |
| TIMP1_IL7R | 0.848 | 94.932 | 0.751 | 0.928 | 92.500 | 97.532 |
| TLR5_CPVL | 0.777 | 94.789 | 0.645 | 0.906 | 91.239 | 97.522 |
| TLR5_CTSH | 0.658 | 94.708 | 0.517 | 0.808 | 75.000 | 100.000 |
| TLR5_DIAPH2 | 0.558 | 94.871 | 0.385 | 0.738 | 74.583 | 100.000 |
| TLR5_ENO1 | 0.666 | 95.005 | 0.499 | 0.805 | 77.639 | 100.000 |
| TLR5_FGL2 | 0.788 | 94.815 | 0.652 | 0.892 | 91.122 | 98.593 |
| TLR5_FTL | 0.705 | 95.000 | 0.557 | 0.852 | 91.063 | 98.362 |
| TLR5_FUT8 | 0.841 | 94.932 | 0.715 | 0.939 | 91.760 | 97.592 |
| TLR5_GBP2 | 0.667 | 94.997 | 0.499 | 0.856 | 80.000 | 100.000 |
| TLR5_HIST1_H2BM | 0.727 | 94.972 | 0.589 | 0.870 | 91.045 | 98.462 |
| TLR5_HIST1H3C | 0.660 | 95.055 | 0.501 | 0.785 | 87.458 | 100.000 |
| TLR5_HIST1H4L | 0.711 | 95.181 | 0.562 | 0.839 | 87.500 | 100.000 |
| TLR5_HLA-DPA1 | 0.828 | 94.893 | 0.715 | 0.940 | 92.385 | 97.641 |
| TLR5_IFIT1 | 0.756 | 95.063 | 0.603 | 0.906 | 81.818 | 100.000 |
| TLR5_IFNGR2 | 0.630 | 94.879 | 0.476 | 0.754 | 83.333 | 100.000 |
| TLR5_ITGB1 | 0.788 | 94.871 | 0.657 | 0.888 | 91.760 | 97.436 |

(continued)

| Derived Biomarker | AUC | NPV | AUC (upper) | AUC (lower) | NPV (lower) | NPV (upper) |
|---|---|---|---|---|---|---|
| TLR5_MX1 | 0.768 | 94.964 | 0.613 | 0.910 | 92.192 | 98.650 |
| TLR5_NMI | 0.644 | 94.892 | 0.522 | 0.773 | 89.730 | 100.000 |
| TLR5_PLAC8 | 0.640 | 94.258 | 0.491 | 0.802 | 88.537 | 100.000 |
| TLR5_RDX | 0.712 | 95.106 | 0.551 | 0.853 | 87.473 | 100.000 |
| TLR5_SDHC | 0.678 | 95.081 | 0.532 | 0.805 | 88.889 | 100.000 |
| TLR5_SLAMF7 | 0.681 | 95.347 | 0.524 | 0.840 | 89.617 | 100.000 |
| TLR5_ST3GAL5 | 0.808 | 94.993 | 0.693 | 0.925 | 92.209 | 98.704 |
| TLR5_TMEM106C | 0.767 | 95.122 | 0.603 | 0.890 | 92.208 | 97.487 |
| TLR5_TPP2 | 0.811 | 95.247 | 0.671 | 0.933 | 75.000 | 100.000 |
| TLR5_VAV1 | 0.692 | 95.083 | 0.556 | 0.833 | 66.667 | 100.000 |
| TMEM106C_IL7R | 0.683 | 94.808 | 0.562 | 0.797 | 91.379 | 98.214 |
| TMEM80_IMP3 | 0.818 | 94.948 | 0.697 | 0.917 | 92.561 | 97.562 |
| TPP2_IL7R | 0.658 | 95.008 | 0.515 | 0.782 | 83.333 | 100.000 |
| USP3_RNF34 | 0.808 | 95.276 | 0.702 | 0.918 | 92.948 | 98.593 |
| VAV1_IL7R | 0.805 | 94.652 | 0.694 | 0.886 | 91.949 | 97.561 |
| YPEL5_ARL2BP | 0.793 | 95.099 | 0.621 | 0.914 | 92.593 | 97.590 |
| ZBTB17_ID3 | 0.839 | 95.050 | 0.717 | 0.937 | 66.667 | 100.000 |

Table 7: Performance of 200 derived biomarkers at a set sepsis prevalence of 5%. Performance measures include Area Under Curve (AUC) and Negative Predictive Value (NPV).

| Derived Biomarker | AUC | NPV | AUC (upper) | AUC (lower) | NPV (lower) | NPV (upper) |
|---|---|---|---|---|---|---|
| AIF1_HMGN4 | 0.797 | 95.000 | 0.610 | 0.924 | 95.000 | 95.000 |
| ALDOA_MAP4K2 | 0.808 | 95.038 | 0.621 | 0.956 | 95.000 | 95.000 |
| ATG9A_RAB11FIP3 | 0.810 | 95.019 | 0.674 | 0.924 | 95.000 | 95.000 |
| ATP13A3_IL7R | 0.846 | 95.038 | 0.692 | 0.971 | 95.000 | 95.000 |
| ATP6V0A1_RASSF7 | 0.832 | 95.096 | 0.686 | 0.960 | 95.000 | 95.960 |
| ATP8B4_CCND2 | 0.827 | 95.067 | 0.646 | 0.954 | 95.000 | 95.960 |
| CD44 GIMAP4 | 0.737 | 95.010 | 0.531 | 0.943 | 95.000 | 95.000 |
| CD44_HLA-DPA1 | 0.746 | 95.096 | 0.567 | 0.939 | 95.000 | 95.960 |
| CD44_IL7R | 0.779 | 95.125 | 0.595 | 0.943 | 95.000 | 95.960 |
| CD44_RPA2 | 0.780 | 95.058 | 0.604 | 0.931 | 95.000 | 95.960 |
| CD44_RPA2 | 0.777 | 95.058 | 0.528 | 0.943 | 95.000 | 95.960 |
| CD59_GIMAP4 | 0.831 | 95.086 | 0.632 | 0.956 | 95.000 | 95.960 |
| CDC14A_CCND2 | 0.594 | 95.003 | 0.333 | 0.832 | 94.949 | 95.000 |
| CDC14A_IL7R | 0.617 | 95.038 | 0.340 | 0.842 | 95.000 | 95.000 |
| CHPT1_FBXO7 | 0.813 | 95.048 | 0.676 | 0.937 | 95.000 | 95.048 |

(continued)

| Derived Biomarker | AUC | NPV | AUC (upper) | AUC (lower) | NPV (lower) | NPV (upper) |
|---|---|---|---|---|---|---|
| CHPT1_RANBP10 | 0.792 | 95.058 | 0.602 | 0.941 | 95.000 | 95.960 |
| CHPT1_RANBP10 | 0.800 | 95.058 | 0.584 | 0.960 | 95.000 | 95.960 |
| CLEC4E_MX1 | 0.704 | 95.086 | 0.439 | 0.941 | 95.000 | 95.960 |
| CLEC4E_MX1 | 0.751 | 95.086 | 0.496 | 0.950 | 95.000 | 95.960 |
| CLEC4E_SYNE2 | 0.780 | 95.010 | 0.556 | 0.929 | 94.949 | 95.000 |
| CLU_CCND2 | 0.749 | 95.053 | 0.591 | 0.895 | 94.898 | 95.920 |
| CLU_IL7R | 0.767 | 95.020 | 0.602 | 0.917 | 94.949 | 95.000 |
| COQ10B_TRAF3IP2 | 0.793 | 95.048 | 0.574 | 0.952 | 95.000 | 95.048 |
| COX5B_PHB | 0.846 | 95.000 | 0.703 | 0.975 | 95.000 | 95.000 |
| CPVL_IL7R | 0.695 | 95.048 | 0.498 | 0.897 | 95.000 | 95.048 |
| CTSA_DLST | 0.826 | 95.040 | 0.658 | 0.965 | 94.949 | 95.918 |
| CTSA_HMG20B | 0.815 | 95.010 | 0.665 | 0.950 | 95.000 | 95.000 |
| CTSC_CCND2 | 0.806 | 95.059 | 0.581 | 0.937 | 94.949 | 95.918 |
| CTSH_IL7R | 0.801 | 95.058 | 0.617 | 0.935 | 95.000 | 95.048 |
| CYBB_BRD7 | 0.794 | 95.010 | 0.619 | 0.939 | 95.000 | 95.000 |
| DERA_HMGN4 | 0.818 | 95.117 | 0.677 | 0.950 | 94.949 | 95.960 |
| DIAPH2_CCND2 | 0.878 | 95.043 | 0.728 | 0.979 | 94.949 | 95.918 |
| DIAPH2_HLA-DPA1 | 0.849 | 95.019 | 0.690 | 0.971 | 95.000 | 95.000 |
| DIAPH2_IL7R | 0.886 | 95.086 | 0.774 | 0.975 | 95.000 | 95.960 |
| DIAPH2_PHF3 | 0.815 | 95.038 | 0.623 | 0.933 | 95.000 | 95.000 |
| DIAPH2_RAB9A | 0.795 | 95.038 | 0.583 | 0.945 | 95.000 | 95.000 |
| DIAPH2_RNASE6 | 0.773 | 95.029 | 0.551 | 0.929 | 95.000 | 95.000 |
| DIAPH2_SERTAD2 | 0.850 | 95.049 | 0.701 | 0.947 | 94.949 | 95.918 |
| DIAPH2_ST3GAL5 | 0.832 | 95.000 | 0.633 | 0.962 | 95.000 | 95.000 |
| DIAPH2_STK38 | 0.781 | 95.029 | 0.627 | 0.901 | 95.000 | 95.000 |
| EIF4E2_C21orf59 | 0.808 | 95.046 | 0.644 | 0.933 | 94.898 | 95.918 |
| EMR1_AKT1 | 0.772 | 95.125 | 0.584 | 0.918 | 95.000 | 95.960 |
| EN01_IL7R | 0.784 | 95.048 | 0.613 | 0.922 | 94.949 | 95.960 |
| FCER1G_CD44 | 0.731 | 95.014 | 0.519 | 0.893 | 94.949 | 95.000 |
| FCER1G_CDC14A | 0.843 | 95.000 | 0.720 | 0.952 | 95.000 | 95.000 |
| FCER1G_MX1 | 0.793 | 95.029 | 0.568 | 0.948 | 95.000 | 95.000 |
| FCER1G_SDHC | 0.667 | 95.034 | 0.421 | 0.870 | 94.949 | 95.048 |
| FLVCR2_KATNA1 | 0.835 | 95.029 | 0.671 | 0.967 | 95.000 | 95.000 |
| FTL_CCND2 | 0.733 | 95.068 | 0.534 | 0.900 | 94.898 | 95.918 |
| FTL_IL7R | 0.757 | 95.010 | 0.593 | 0.906 | 95.000 | 95.000 |
| FURIN_ADD1 | 0.842 | 95.000 | 0.707 | 0.945 | 95.000 | 95.000 |
| FURIN_BTG2 | 0.834 | 95.125 | 0.691 | 0.945 | 95.000 | 95.960 |

(continued)

| Derived Biomarker | AUC | NPV | AUC (upper) | AUC (lower) | NPV (lower) | NPV (upper) |
|---|---|---|---|---|---|---|
| FURIN_RANBP10 | 0.828 | 95.010 | 0.650 | 0.975 | 95.000 | 95.000 |
| FURIN_SH3GLB2 | 0.811 | 95.077 | 0.650 | 0.952 | 95.000 | 95.960 |
| FUT8_IL7R | 0.564 | 95.010 | 0.345 | 0.805 | 95.000 | 95.000 |
| FXR1_EIF4A2 | 0.836 | 95.000 | 0.678 | 0.954 | 95.000 | 95.000 |
| GAPDH_COMMD4 | 0.810 | 95.000 | 0.595 | 0.956 | 95.000 | 95.000 |
| GAPDH_PPP1CA | 0.787 | 95.058 | 0.623 | 0.918 | 95.000 | 95.960 |
| GAPDH_RPS6KB2 | 0.816 | 95.019 | 0.642 | 0.962 | 95.000 | 95.000 |
| GAS7_ADD1 | 0.805 | 95.000 | 0.628 | 0.958 | 95.000 | 95.000 |
| GAS7_RAB11FIP1 | 0.851 | 95.029 | 0.654 | 0.989 | 95.000 | 95.000 |
| GBP2_GIMAP4 | 0.810 | 95.039 | 0.648 | 0.931 | 94.898 | 95.918 |
| GBP2_HCP5 | 0.789 | 95.010 | 0.625 | 0.937 | 94.898 | 95.878 |
| GBP2_MX1 | 0.690 | 95.053 | 0.482 | 0.895 | 94.949 | 95.918 |
| GNS_PLEKHG3 | 0.829 | 95.000 | 0.670 | 0.958 | 95.000 | 95.000 |
| GNS_PLEKHG3 | 0.845 | 95.000 | 0.678 | 0.963 | 95.000 | 95.000 |
| GSTO1_RASGRP3 | 0.817 | 95.077 | 0.613 | 0.960 | 95.000 | 95.960 |
| GSTO1_SDF2L1 | 0.792 | 95.067 | 0.623 | 0.929 | 95.000 | 95.960 |
| HEBP1_SSBP2 | 0.824 | 94.924 | 0.656 | 0.958 | 79.333 | 100.000 |
| HIST1H2BM_CCND2 | 0.748 | 94.994 | 0.520 | 0.908 | 94.949 | 95.000 |
| HIST1H2BM_IL7R | 0.768 | 95.002 | 0.555 | 0.927 | 94.949 | 95.000 |
| HIST1H3C_IL7R | 0.793 | 95.000 | 0.627 | 0.912 | 95.000 | 100.000 |
| HOXB6_PAFAH2 | 0.829 | 95.063 | 0.656 | 0.945 | 94.949 | 95.920 |
| HSPA4_IMP3 | 0.813 | 95.000 | 0.616 | 0.950 | 95.000 | 95.000 |
| HSPA4_IMP3 | 0.818 | 95.000 | 0.636 | 0.947 | 95.000 | 95.000 |
| HSPA4_IMP3 | 0.819 | 95.000 | 0.658 | 0.935 | 95.000 | 95.000 |
| HSPA4_IMP3 | 0.825 | 95.000 | 0.669 | 0.947 | 95.000 | 95.000 |
| IFNGR2_CCND2 | 0.829 | 95.058 | 0.623 | 0.969 | 95.000 | 95.960 |
| IFNGR2_HLA-DPA1 | 0.807 | 95.010 | 0.605 | 0.960 | 95.000 | 95.000 |
| IFNGR2_IL7R | 0.830 | 95.048 | 0.638 | 0.958 | 95.000 | 95.048 |
| IMPDH1_BTG2 | 0.831 | 95.010 | 0.634 | 0.985 | 95.000 | 95.000 |
| IMPDH1_BTG2 | 0.840 | 95.010 | 0.679 | 0.979 | 95.000 | 95.000 |
| ITGAX_RASGRP2 | 0.813 | 95.003 | 0.612 | 0.952 | 94.949 | 95.000 |
| ITGB1_IL7R | 0.674 | 95.063 | 0.484 | 0.910 | 94.949 | 95.960 |
| LAMP1_HLA-DPA1 | 0.752 | 95.077 | 0.535 | 0.941 | 95.000 | 95.960 |
| LAMP1_IL7R | 0.777 | 95.029 | 0.600 | 0.935 | 95.000 | 95.000 |
| LHFPL2_ISG20 | 0.790 | 95.038 | 0.623 | 0.912 | 95.000 | 95.000 |
| LILRB3_IL7R | 0.808 | 95.010 | 0.602 | 0.952 | 95.000 | 95.000 |
| LTA4H_CCND2 | 0.822 | 95.097 | 0.686 | 0.950 | 94.949 | 95.920 |

(continued)

| Derived Biomarker | AUC | NPV | AUC (upper) | AUC (lower) | NPV (lower) | NPV (upper) |
|---|---|---|---|---|---|---|
| LTA4H_CERK | 0.789 | 95.106 | 0.624 | 0.939 | 95.000 | 95.960 |
| LTA4H_CPVL | 0.753 | 95.058 | 0.524 | 0.929 | 95.000 | 95.960 |
| LTA4H_RPS8 | 0.839 | 95.067 | 0.619 | 0.992 | 95.000 | 95.960 |
| LTA4H_ST3GAL5 | 0.754 | 95.010 | 0.549 | 0.941 | 95.000 | 95.000 |
| LTA4H_TMEM106C | 0.688 | 95.058 | 0.416 | 0.910 | 95.000 | 95.960 |
| LTA4H_WDR33 | 0.794 | 95.000 | 0.585 | 0.950 | 95.000 | 95.000 |
| LTF_MAP4K2 | 0.770 | 95.010 | 0.547 | 0.948 | 95.000 | 95.000 |
| MAPK14 GIMAP4 | 0.833 | 95.019 | 0.667 | 0.939 | 95.000 | 95.000 |
| MAPK14_IL7R | 0.823 | 95.058 | 0.689 | 0.931 | 95.000 | 95.960 |
| MAPK14_IL7R | 0.842 | 95.058 | 0.693 | 0.958 | 95.000 | 95.960 |
| MAPK14_IL7R | 0.828 | 95.058 | 0.659 | 0.949 | 95.000 | 95.960 |
| MAPK14_MX1 | 0.727 | 95.048 | 0.497 | 0.935 | 95.000 | 95.048 |
| MAPK8IP3_IMP3 | 0.817 | 95.029 | 0.624 | 0.964 | 95.000 | 95.000 |
| MCTP1_AMD1 | 0.836 | 95.048 | 0.681 | 0.969 | 95.000 | 95.048 |
| MCTP1_TOB1 | 0.847 | 95.230 | 0.667 | 0.954 | 95.000 | 95.960 |
| MEGF9_CCND2 | 0.790 | 95.061 | 0.577 | 0.949 | 94.949 | 95.918 |
| MEGF9_CDC14A | 0.752 | 95.067 | 0.547 | 0.929 | 95.000 | 95.960 |
| MEGF9_GIMAP4 | 0.760 | 95.004 | 0.561 | 0.941 | 94.949 | 95.000 |
| MEGF9_HLA-DPA1 | 0.769 | 95.063 | 0.535 | 0.945 | 94.949 | 95.918 |
| MEGF9_IL7R | 0.779 | 95.017 | 0.526 | 0.946 | 74.583 | 100.000 |
| METTL9_AKTIP | 0.814 | 95.001 | 0.665 | 0.929 | 94.898 | 95.000 |
| MICAL1_DHX16 | 0.808 | 95.029 | 0.600 | 0.935 | 95.000 | 95.000 |
| MICAL1_STK38 | 0.812 | 95.019 | 0.648 | 0.941 | 95.000 | 95.000 |
| MICAL1_STK38 | 0.817 | 95.019 | 0.658 | 0.931 | 95.000 | 95.000 |
| MMP8_CCND2 | 0.857 | 95.164 | 0.666 | 0.962 | 95.000 | 95.960 |
| MMP8_CD44 | 0.664 | 95.051 | 0.431 | 0.872 | 94.949 | 95.920 |
| MMP8_CTSC | 0.616 | 95.002 | 0.374 | 0.836 | 94.845 | 95.918 |
| MMP8_ENO1 | 0.629 | 95.049 | 0.423 | 0.861 | 94.898 | 95.878 |
| MMP8_FGL2 | 0.736 | 95.065 | 0.561 | 0.912 | 94.949 | 95.920 |
| MNP8_FTL | 0.674 | 95.056 | 0.390 | 0.845 | 94.898 | 95.918 |
| MMP8_FUT8 | 0.834 | 95.019 | 0.666 | 0.981 | 95.000 | 95.000 |
| MMP8_IL7R | 0.859 | 95.058 | 0.711 | 0.954 | 94.949 | 95.920 |
| MMP8_ITGB1 | 0.762 | 95.038 | 0.507 | 0.924 | 95.000 | 95.000 |
| MMP8_LAMP1 | 0.642 | 95.022 | 0.429 | 0.843 | 94.898 | 95.044 |
| MMP8_PLAC8 | 0.587 | 95.141 | 0.340 | 0.849 | 94.947 | 95.960 |
| MMP8_RPS8 | 0.856 | 95.058 | 0.667 | 0.969 | 95.000 | 95.960 |
| MMP8_SDHC | 0.651 | 95.006 | 0.460 | 0.847 | 94.898 | 95.000 |

(continued)

| Derived Biomarker | AUC | NPV | AUC (upper) | AUC (lower) | NPV (lower) | NPV (upper) |
|---|---|---|---|---|---|---|
| MMP8_ST3GAL5 | 0.765 | 95.048 | 0.602 | 0.922 | 95.000 | 95.000 |
| MMP8_TMEM106C | 0.813 | 95.000 | 0.595 | 0.960 | 95.000 | 95.000 |
| MMP8_TPP2 | 0.807 | 95.086 | 0.614 | 0.947 | 95.000 | 95.960 |
| MMP8_TPP2 | 0.792 | 95.086 | 0.601 | 0.945 | 95.000 | 95.960 |
| MMP8_VAV1 | 0.623 | 95.062 | 0.366 | 0.862 | 94.949 | 95.960 |
| MNT_KLF2 | 0.806 | 95.066 | 0.595 | 0.939 | 94.949 | 95.918 |
| MNT_SLC9A3R1 | 0.791 | 95.106 | 0.600 | 0.927 | 95.000 | 95.960 |
| MUT_NUPL2 | 0.789 | 95.010 | 0.625 | 0.916 | 95.000 | 95.000 |
| MUT_NUPL2 | 0.773 | 95.010 | 0.598 | 0.901 | 95.000 | 95.000 |
| MYL9_GRAP2 | 0.814 | 95.000 | 0.607 | 0.964 | 95.000 | 100.000 |
| MYL9_KLF2 | 0.834 | 95.000 | 0.678 | 0.937 | 95.000 | 100.000 |
| NMI_MX1 | 0.671 | 95.019 | 0.467 | 0.910 | 95.000 | 95.000 |
| OBFC1_C6orf48 | 0.802 | 95.108 | 0.526 | 0.966 | 94.949 | 95.960 |
| PARL_PAFAH2 | 0.830 | 95.010 | 0.675 | 0.968 | 95.000 | 95.000 |
| PARL_PAFAH2 | 0.819 | 95.010 | 0.646 | 0.947 | 95.000 | 95.000 |
| PDGFC_CCND2 | 0.848 | 95.048 | 0.698 | 0.956 | 95.000 | 95.048 |
| PDGFC_FUT8 | 0.823 | 95.086 | 0.601 | 0.956 | 95.000 | 95.960 |
| PDGFC_IL7R | 0.844 | 95.038 | 0.678 | 0.973 | 95.000 | 95.000 |
| PDGFC_ITGB1 | 0.778 | 95.038 | 0.611 | 0.922 | 95.000 | 95.000 |
| PLEKHM2_SBF1 | 0.825 | 95.084 | 0.669 | 0.935 | 94.845 | 95.918 |
| PPP1CB_PAFAH2 | 0.798 | 95.042 | 0.613 | 0.945 | 94.949 | 95.920 |
| PROS1_MYOM2 | 0.793 | 95.048 | 0.599 | 0.916 | 95.000 | 95.048 |
| PROS1_WDR33 | 0.788 | 95.058 | 0.612 | 0.917 | 95.000 | 95.960 |
| PRPF40A_MRPS18B | 0.796 | 95.048 | 0.575 | 0.948 | 95.000 | 95.048 |
| PRRG4_GLOD4 | 0.837 | 95.058 | 0.627 | 0.966 | 95.000 | 95.960 |
| PSMB4_IMP3 | 0.837 | 95.010 | 0.661 | 0.958 | 95.000 | 95.000 |
| PSTPIP2_AKAP7 | 0.825 | 95.010 | 0.672 | 0.962 | 95.000 | 95.000 |
| PTPN2_CYP20A1 | 0.824 | 95.048 | 0.664 | 0.954 | 95.000 | 95.048 |
| PUS3_PAFAH2 | 0.842 | 95.038 | 0.682 | 0.954 | 95.000 | 95.000 |
| S100A12_POLR2C | 0.783 | 95.048 | 0.618 | 0.923 | 95.000 | 95.000 |
| S100P_GIMAP4 | 0.780 | 95.019 | 0.568 | 0.956 | 95.000 | 95.000 |
| S100P_HLA-DPA1 | 0.804 | 95.000 | 0.620 | 0.947 | 95.000 | 95.000 |
| S100P_IL7R | 0.837 | 95.053 | 0.666 | 0.939 | 94.949 | 95.960 |
| SH3BGRL_GLOD4 | 0.788 | 95.058 | 0.596 | 0.950 | 95.000 | 95.960 |
| SLC11A2_ID3 | 0.814 | 94.998 | 0.625 | 0.944 | 94.898 | 95.000 |
| SLC12A9_CTDSP2 | 0.821 | 95.038 | 0.686 | 0.929 | 95.000 | 95.000 |
| SLC12A9_CTDSP2 | 0.815 | 95.038 | 0.669 | 0.949 | 95.000 | 95.000 |

(continued)

| Derived Biomarker | AUC | NPV | AUC (upper) | AUC (lower) | NPV (lower) | NPV (upper) |
|---|---|---|---|---|---|---|
| SLC25A37_FBXO7 | 0.808 | 95.048 | 0.614 | 0.944 | 95.000 | 95.048 |
| SLC25A37_FBXO7 | 0.804 | 95.048 | 0.665 | 0.950 | 95.000 | 95.048 |
| SLC25A37_FBXO7 | 0.802 | 95.048 | 0.612 | 0.956 | 95.000 | 95.048 |
| SLC2A3_ADAM19 | 0.795 | 95.010 | 0.599 | 0.937 | 95.000 | 95.000 |
| SLC2A3_MFSD10 | 0.790 | 95.099 | 0.613 | 0.929 | 94.949 | 95.960 |
| SLC39A8_CCND2 | 0.867 | 95.069 | 0.726 | 0.969 | 94.949 | 95.960 |
| SLC39A8_IL7R | 0.848 | 95.051 | 0.705 | 0.963 | 94.949 | 95.920 |
| SLC39A8_LFNG | 0.798 | 95.058 | 0.637 | 0.933 | 95.000 | 95.960 |
| SLC39A8_WDR33 | 0.819 | 95.019 | 0.660 | 0.958 | 95.000 | 95.000 |
| SNAPC1_IL7R | 0.816 | 95.000 | 0.644 | 0.967 | 95.000 | 95.000 |
| SORT1_CNBP | 0.849 | 94.997 | 0.703 | 0.954 | 94.949 | 95.000 |
| SORT1_INPP1 | 0.837 | 95.077 | 0.685 | 0.954 | 95.000 | 95.960 |
| SORT1_NAGK | 0.807 | 95.077 | 0.683 | 0.916 | 95.000 | 95.960 |
| SORT1_OSBPL9 | 0.847 | 95.038 | 0.617 | 0.981 | 95.000 | 95.000 |
| SORT1_PDCD5 | 0.815 | 95.010 | 0.614 | 0.977 | 95.000 | 95.000 |
| SORT1_PDCD5 | 0.841 | 95.010 | 0.610 | 0.966 | 95.000 | 95.000 |
| SORT1_PPP1R11 | 0.812 | 95.038 | 0.641 | 0.943 | 94.898 | 95.878 |
| SORT1_SASH3 | 0.781 | 95.009 | 0.613 | 0.935 | 94.949 | 95.000 |
| SORT1_TINF2 | 0.798 | 95.096 | 0.599 | 0.929 | 94.949 | 95.960 |
| ST3GAL6_KLRF1 | 0.820 | 95.077 | 0.661 | 0.960 | 95.000 | 95.960 |
| TAX1BP1_NUPL2 | 0.802 | 95.000 | 0.628 | 0.929 | 95.000 | 100.000 |
| TIMP1_EMP3 | 0.801 | 95.049 | 0.624 | 0.931 | 94.898 | 95.918 |
| TIMP1_IL7R | 0.839 | 95.000 | 0.703 | 0.944 | 95.000 | 100.000 |
| TLR5_CPVL | 0.789 | 95.086 | 0.626 | 0.918 | 95.000 | 95.960 |
| TLR5_CTSH | 0.664 | 95.019 | 0.448 | 0.835 | 94.949 | 95.000 |
| TLR5_DIAPH2 | 0.553 | 95.035 | 0.328 | 0.761 | 94.444 | 95.831 |
| TLR5_ENO1 | 0.662 | 95.002 | 0.457 | 0.861 | 94.949 | 95.000 |
| TLR5_FGL2 | 0.792 | 95.163 | 0.644 | 0.933 | 95.000 | 95.960 |
| TLR5_FTL | 0.707 | 94.996 | 0.533 | 0.872 | 94.898 | 95.000 |
| TLR5_FUT8 | 0.862 | 95.048 | 0.701 | 0.962 | 95.000 | 95.048 |
| TLR5_GBP2 | 0.663 | 95.185 | 0.425 | 0.870 | 94.949 | 95.960 |
| TLR5_HIST1H2BM | 0.722 | 94.990 | 0.469 | 0.923 | 94.949 | 95.000 |
| TLR5_HIST1H3C | 0.637 | 95.242 | 0.409 | 0.820 | 94.565 | 96.705 |
| TLR5_HIST1H4L | 0.711 | 95.029 | 0.484 | 0.900 | 95.000 | 95.000 |
| TLR5_HLA-DPA1 | 0.848 | 95.067 | 0.663 | 0.966 | 95.000 | 95.960 |
| TLR5_IFIT1 | 0.759 | 95.038 | 0.543 | 0.933 | 95.000 | 95.000 |
| TLR5_IFNGR2 | 0.613 | 94.983 | 0.427 | 0.768 | 94.898 | 95.000 |

(continued)

| Derived Biomarker | AUC | NPV | AUC (upper) | AUC (lower) | NPV (lower) | NPV (upper) |
|---|---|---|---|---|---|---|
| TLR5_ITGB1 | 0.779 | 95.010 | 0.546 | 0.933 | 95.000 | 95.000 |
| TLR5_ITGB1 | 0.786 | 95.010 | 0.583 | 0.943 | 95.000 | 95.000 |
| TLR5_MX1 | 0.756 | 95.307 | 0.524 | 0.935 | 95.000 | 95.960 |
| TLR5_NMI | 0.681 | 95.028 | 0.418 | 0.878 | 94.681 | 95.833 |
| TLR5_PLAC8 | 0.640 | 94.769 | 0.455 | 0.836 | 80.000 | 100.000 |
| TLR5_RDX | 0.699 | 95.077 | 0.456 | 0.901 | 95.000 | 95.960 |
| TLR5_SDHC | 0.641 | 94.983 | 0.391 | 0.844 | 94.898 | 95.000 |
| TLR5_SLAMF7 | 0.706 | 95.144 | 0.482 | 0.879 | 95.000 | 95.960 |
| TLR5_ST3GAL5 | 0.821 | 95.115 | 0.669 | 0.960 | 95.000 | 95.960 |
| TLR5_TMEM106C | 0.752 | 95.002 | 0.576 | 0.939 | 94.949 | 95.000 |
| TLR5_TPP2 | 0.812 | 95.010 | 0.631 | 0.951 | 95.000 | 95.000 |
| TLR5_VAV1 | 0.677 | 95.284 | 0.446 | 0.868 | 94.949 | 95.960 |
| TMEM106C_IL7R | 0.666 | 95.004 | 0.492 | 0.872 | 94.949 | 95.000 |
| TMEM80_IMP3 | 0.795 | 95.086 | 0.625 | 0.946 | 95.000 | 95.960 |
| TPP2_IL7R | 0.659 | 95.000 | 0.460 | 0.870 | 95.000 | 95.000 |
| USP3_RNF34 | 0.812 | 94.999 | 0.655 | 0.935 | 94.949 | 95.000 |
| VAV1_IL7R | 0.798 | 95.010 | 0.644 | 0.937 | 95.000 | 95.000 |
| YPEL5_ARL2BP | 0.793 | 95.006 | 0.621 | 0.942 | 94.949 | 95.000 |
| ZBTB17 ID3 | 0.831 | 95.067 | 0.627 | 0.960 | 95.000 | 95.960 |

Table 8: Table of calculated negative predictive values (NPV) for the final triage signature (DIAPH2 / SERTAD2; PARL / PAFAH2; SORT1 / OSBPL9) at sepsis prevalences of 4, 6, 8 and 10%. Based on the scientific literature, the prevalence of sepsis in the ER is approximately 4%. For these calculations the sensitivity and specificity were set at 0.9535 and 0.7303 respectively based on the ROC curve for the final triage signature (see Figure lb).

| Sepsis Prevalence | Sensitivity | Specificity | NPV |
|---|---|---|---|
| 4% | 0.9535 | 0.7303 | 0.99735 |
| 6% | 0.9535 | 0.7303 | 0.99595 |
| 8% | 0.9535 | 0.7303 | 0.99449 |
| 10% | 0.9535 | 0.7303 | 0.99297 |

Table 9: List of numerators and denominators that appear more than once in the top 200 derived biomarkers.

| Numerator Symbol | Times Appearing | Denominator Symbol | Times Appearing |
|---|---|---|---|
| TLR5 | 25 | IL7R | 27 |
| MMP8 | 17 | CCND2 | 13 |
| DIAPH2 | 9 | HLA-DPA1 | 7 |
| SORT1 | 8 | GIMAP4 | 6 |
| LTA4H | 7 | MX1 | 6 |

(continued)

| Numerator Symbol | Times Appearing | Denominator Symbol | Times Appearing |
|---|---|---|---|
| MEGF9 | 5 | IMP3 | 4 |
| CD44 | 4 | PAFAH2 | 4 |
| FCER1G | 4 | ST3GAL5 | 4 |
| FURIN | 4 | FUT8 | 3 |
| PDGFC | 4 | ITGB1 | 3 |
| SLC39A8 | 4 | SDHC | 3 |
| GAPDH | 3 | TMEM106C | 3 |
| GBP2 | 3 | WDR33 | 3 |
| IFNGR2 | 3 | ADD1 | 2 |
| MAPK14 | 3 | BTG2 | 2 |
| MNT | 3 | CD44 | 2 |
| S100P | 3 | CDC14A | 2 |
| CDC14A | 2 | CPVL | 2 |
| CHPT1 | 2 | ENO1 | 2 |
| CLEC4E | 2 | FBXO7 | 2 |
| CLU | 2 | FGL2 | 2 |
| CTSA | 2 | FTL | 2 |
| FTL | 2 | GLOD4 | 2 |
| GAS7 | 2 | HMGN4 | 2 |
| GSTO1 | 2 | ID3 | 2 |
| HIST1H2BM | 2 | KLF2 | 2 |
| LAMP1 | 2 | MAP4K2 | 2 |
| MCTP1 | 2 | NUPL2 | 2 |
| MICAL1 | 2 | PLAC8 | 2 |
| MYL9 | 2 | RANBP10 | 2 |
| PROS1 | 2 | RPS8 | 2 |
| TIMP1 | 2 | STK38 | 2 |
| | | TPP2 | 2 |
| | | VAV1 | 2 |

Table 10: SEQ ID numbers, HUGO gene symbol and Ensembl ID of individual biomarkers.

| SEQ ID# | Symbol | Ensembl |
|---|---|---|
| 1 | ADAM19 | ENST00000257527 |
| 2 | ADD1 | ENST00000398129 |
| 3 | ADGRE1 | ENST00000312053 |
| 4 | AIF1 | ENST00000440907 |
| 5 | AKAP7 | ENST00000342266 |
| 6 | AKT1 | ENST00000555528 |
| 7 | AKTIP | ENST00000394657 |
| 8 | ALDOA | ENST00000338110 |
| 9 | AMD1 | ENST00000368885 |
| 10 | ARL2BP | ENST00000219204 |
| 11 | ATG9A | ENST00000396761 |
| 12 | ATP13A3 | ENST00000256031 |
| 13 | ATP6V0A1 | ENST00000393829 |
| 14 | ATP8B4 | ENST00000284509 |
| 15 | BRD7 | ENST00000394688 |
| 16 | BTG2 | ENST00000290551 |
| 17 | C21orf59 | ENST00000290155 |
| 18 | C6orf48 | ENST00000414434 |
| 19 | CCND2 | ENST00000261254 |
| 20 | CD44 | ENST00000428726 |
| 21 | CD59 | ENST00000395850 |
| 22 | CDC14A | ENST00000336454 |
| 23 | CERK | ENST00000216264 |
| 24 | CHPT1 | ENST00000229266 |
| 25 | CLEC4E | ENST00000299663 |
| 26 | CLU | ENST00000316403 |
| 27 | CNBP | ENST00000422453 |
| 28 | COMMD4 | ENST00000267935 |
| 29 | COQ10B | ENST00000263960 |
| 30 | COX5B | ENST00000258424 |
| 31 | CPVL | ENST00000396276 |
| 32 | CTDSP2 | ENST00000398073 |
| 33 | CTSA | ENST00000372484 |
| 34 | CTSC | ENST00000227266 |
| 35 | CTSH | ENST00000220166 |
| 36 | CYBB | ENST00000378588 |
| 37 | CYP20A1 | ENST00000356079 |
| 38 | DERA | ENST00000428559 |
| 39 | DHX16 | ENST00000451456 |
| 40 | DIAPH2 | ENST00000324765 |
| 41 | DLST | ENST00000334220 |
| 42 | EIF4A2 | ENST00000323963 |
| 43 | EIF4E2 | ENST00000258416 |
| 44 | EMP3 | ENST00000270221 |
| 45 | ENO1 | ENST00000234590 |
| 46 | FBXO7 | ENST00000266087 |
| 47 | FCER1G | ENST00000289902 |
| 48 | FGL2 | ENST00000248598 |
| 49 | FLVCR2 | ENST00000238667 |
| 50 | FTL | ENST00000331825 |
| 51 | FURIN | ENST00000268171 |
| 52 | FUT8 | ENST00000557164 |
| 53 | FXR1 | ENST00000357559 |
| 54 | GAPDH | ENST00000229239 |
| 55 | GAS7 | ENST00000580865 |
| 56 | GBP2 | ENST00000370466 |
| 57 | GIMAP4 | ENST00000255945 |
| 58 | GLOD4 | ENST00000301329 |
| 59 | GNS | ENST00000258145 |
| 60 | GRAP2 | ENST00000344138 |
| 61 | GSTO1 | ENST00000369713 |
| 62 | HEBP1 | ENST00000014930 |
| 63 | HIST1H2BM | ENST00000621112 |
| 64 | HIST1H3C | ENST00000612966 |
| 65 | HIST1H4L | ENST00000618305 |
| 66 | HLA-DPA1 | ENST00000383224 |
| 67 | HMG20B | ENST00000333651 |
| 68 | HMGN4 | ENST00000377575 |
| 69 | HOXB6 | ENST00000225648 |
| 70 | HSPA4 | ENST00000304858 |
| 71 | ID3 | ENST00000374561 |
| 72 | IFIT1 | ENST00000371804 |
| 73 | IFNGR2 | ENST00000290219 |
| 74 | IL7R | ENST00000303115 |
| 75 | IMP3 | ENST00000403490 |
| 76 | IMPDH1 | ENST00000338791 |
| 77 | INPP1 | ENST00000322522 |
| 78 | ISG20 | ENST00000306072 |

| SEQ ID# | Symbol | Ensembl |
|---|---|---|
| 79 | ITGAX | ENST00000268296 |
| 80 | ITGB1 | ENST00000302278 |
| 81 | KATNA1 | ENST00000367411 |
| 82 | KLF2 | ENST00000248071 |
| 83 | KLRF1 | ENST00000617889 |
| 84 | LAMP1 | ENST00000332556 |
| 85 | LFNG | ENST00000402045 |
| 86 | LHFPL2 | ENST00000380345 |
| 87 | LILRB3 | ENST00000617251 |
| 88 | LTA4H | ENST00000228740 |
| 89 | LTF | ENST00000231751 |
| 90 | MAP4K2 | ENST00000294066 |
| 91 | MAPK14 | ENST00000229795 |
| 92 | MAPK8IP3 | ENST00000250894 |
| 93 | MCTP1 | ENST00000515393 |
| 94 | MEGF9 | ENST00000373930 |
| 95 | METTL9 | ENST00000358154 |
| 96 | MFSD10 | ENST00000329687 |
| 97 | MICAL1 | ENST00000358807 |
| 98 | MMP8 | ENST00000236826 |
| 99 | MNT | ENST00000174618 |
| 100 | MRPS18B | ENST00000412451 |
| 101 | MUT | ENST00000274813 |
| 102 | MX1 | ENST00000398598 |
| 103 | MYL9 | ENST00000279022 |
| 104 | MYOM2 | ENST00000616680 |
| 105 | NAGK | ENST00000613852 |

| SEQ ID# | Symbol | Ensembl |
|---|---|---|
| 106 | NMI | ENST00000243346 |
| 107 | NUPL2 | ENST00000258742 |
| 108 | OBFC1 | ENST00000224950 |
| 109 | OSBPL9 | ENST00000428468 |
| 110 | PAFAH2 | ENST00000374282 |
| 111 | PARL | ENST00000317096 |
| 112 | PDCD5 | ENST00000590247 |
| 113 | PDGFC | ENST00000502773 |
| 114 | PHB | ENST00000300408 |
| 115 | PHF3 | ENST00000393387 |
| 116 | PLAC8 | ENST00000311507 |
| 117 | PLEKHG3 | ENST00000247226 |
| 118 | PLEKHM2 | ENST00000375799 |
| 119 | POLR2C | ENST00000219252 |
| 120 | PPP1CA | ENST00000376745 |
| 121 | PPP1CB | ENST00000395366 |
| 122 | PPP1R11 | ENST00000431424 |
| 123 | PROS1 | ENST00000394236 |
| 124 | PRPF40A | ENST00000410080 |
| 125 | PRRG4 | ENST00000257836 |
| 126 | PSMB4 | ENST00000290541 |
| 127 | PSTPIP2 | ENST00000409746 |
| 128 | PTPN2 | ENST00000309660 |
| 129 | PUS3 | ENST00000227474 |
| 130 | RAB11FIP1 | ENST00000287263 |
| 131 | RAB11FIP3 | ENST00000611004 |
| 132 | RAB9A | ENST00000464506 |

| SEQ ID# | Symbol | Ensembl |
|---|---|---|
| 133 | RANBP10 | ENST00000317506 |
| 134 | RASGRP2 | ENST00000354024 |
| 135 | RASGRP3 | ENST00000407811 |
| 136 | RASSF7 | ENST00000622100 |
| 137 | RDX | ENST00000343115 |
| 138 | RNASE6 | ENST00000304677 |
| 139 | RNF34 | ENST00000361234 |
| 140 | RPA2 | ENST00000373912 |
| 141 | RPS6KB2 | ENST00000312629 |
| 142 | RPS8 | ENST00000396651 |
| 143 | S100A12 | ENST00000368737 |
| 144 | S100P | ENST00000296370 |
| 145 | SASH3 | ENST00000356892 |
| 146 | SBF1 | ENST00000380817 |
| 147 | SDF2L1 | ENST00000248958 |
| 148 | SDHC | ENST00000367975 |
| 149 | SERTAD2 | ENST00000313349 |
| 150 | SH3BGRL | ENST00000373212 |
| 151 | SH3GLB2 | ENST00000372564 |
| 152 | SLAMF7 | ENST00000368043 |
| 153 | SLC11A2 | ENST00000262052 |
| 154 | SLC12A9 | ENST00000354161 |
| 155 | SLC25A37 | ENST00000519973 |
| 156 | SLC2A3 | ENST00000075120 |
| 157 | SLC39A8 | ENST00000394833 |
| 158 | SLC9A3R1 | ENST00000262613 |
| 159 | SNAPC1 | ENST00000216294 |

| SEQ ID# | Symbol | Ensembl |
|---|---|---|
| 160 | SORT1 | ENST00000256637 |
| 161 | SSBP2 | ENST00000320672 |
| 162 | ST3GAL5 | ENST00000377332 |
| 163 | ST3GAL6 | ENST00000394162 |
| 164 | STK38 | ENST00000229812 |
| 165 | SYNE2 | ENST00000344113 |
| 166 | TAX1BP1 | ENST00000396319 |

| SEQ ID# | Symbol | Ensembl |
|---|---|---|
| 167 | TIMP1 | ENST00000218388 |
| 168 | TINF2 | ENST00000399423 |
| 169 | TLR5 | ENST00000366881 |
| 170 | TMEM106C | ENST00000552561 |
| 171 | TMEM80 | ENST00000397510 |
| 172 | TOB1 | ENST00000499247 |
| 173 | TPP2 | ENST00000376065 |

| SEQ ID# | Symbol | Ensembl |
|---|---|---|
| 174 | TRAF3IP2 | ENST00000340026 |
| 175 | USP3 | ENST00000380324 |
| 176 | VAV1 | ENST00000602142 |
| 177 | WDR33 | ENST00000322313 |
| 178 | YPEL5 | ENST00000261353 |
| 179 | ZBTB17 | ENST00000375743 |

Table 11: SEQ ID numbers, HUGO gene symbol and Ensembl ID of individual biomarkers.

| SEQ ID# | Symbol | Genbank | SEQ ID# | Symbol | Genbank | SEQ ID# | Symbol | Genbank |
|---|---|---|---|---|---|---|---|---|
| 180 | ADAM19 | NP_150377 | 206 | CNBP | NP_003409 | 232 | FXR1 | NP_005078 |
| 181 | ADD1 | NP_001110 | 207 | COMMD4 | NP_060298 | 233 | GAPDH | NP_002037 |
| 182 | ADGRE1 | NP_001965 | 208 | COQ10B | NP_079423 | 234 | GAS7 | NP_003635 |
| 183 | AIF1 | NP_001614 | 209 | COX5B | NP_001853 | 235 | GBP2 | NP_004111 |
| 184 | AKAP7 | NP_004833 | 210 | CPVL | NP_061902 | 236 | GIMAP4 | NP_060796 |
| 185 | AKT1 | NP_005154 | 211 | CTDSP2 | NP_005721 | 237 | GLOD4 | NP_057164 |
| 186 | AKTIP | NP_071921 | 212 | CTSA | NP_000299 | 238 | GNS | NP_002067 |
| 187 | ALDOA | NP_000025 | 213 | CTSC | NP_001805 | 239 | GRAP2 | NP_004801 |
| 188 | AMD1 | NP_001625 | 214 | CTSH | NP_004381 | 240 | GSTO1 | NP_004823 |
| 189 | ARL2BP | NP_036238 | 215 | CYBB | NP_000388 | 241 | HEBP1 | NP_057071 |
| 190 | ATG9A | NP_076990 | 216 | CYP20A1 | NP_803882 | 242 | HIST1H2BM | NP_003512 |
| 191 | ATP13A3 | NP_078800 | 217 | DERA | NP_057038 | 243 | HIST1H3C | NP_003522 |
| 192 | ATP6V0A1 | NP_005168 | 218 | DHX16 | NP_003578 | 244 | HIST1H4L | NP_003537 |
| 193 | ATP8B4 | NP_079113 | 219 | DIAPH2 | NP_006720 | 245 | HLA-DPA1 | NP_291032 |
| 194 | BRD7 | NP_037395 | 220 | DLST | NP_001924 | 246 | HMG20B | NP_006330 |
| 195 | BTG2 | NP_006754 | 221 | EIF4A2 | NP_001958 | 247 | HMGN4 | NP_006344 |
| 196 | C21orf59 | NP_067077 | 222 | EIF4E2 | NP_004837 | 248 | HOXB6 | NP_061825 |
| 197 | C6orf48 | NP_001035527 | 223 | EMP3 | NP_001416 | 249 | HSPA4 | NP_002145 |
| 198 | CCND2 | NP_001750 | 224 | ENO1 | NP_001419 | 250 | ID3 | NP_002158 |
| 199 | CD44 | NP_000601 | 225 | FBXO7 | NP_036311 | 251 | IFIT1 | NP_001539 |
| 200 | CD59 | NP_000602 | 226 | FCER1G | NP_004097 | 252 | IFNGR2 | NP_005525 |
| 201 | CDC14A | NP_003663 | 227 | FGL2 | NP_006673 | 253 | IL7R | NP_002176 |
| 202 | CERK | NP_073603 | 228 | FLVCR2 | NP_060261 | 254 | IMP3 | NP_060755 |
| 203 | CHPT1 | NP_064629 | 229 | FTL | NP_000137 | 255 | IMPDH1 | NP_000874 |
| 204 | CLEC4E | NP_055173 | 230 | FURIN | NP_002560 | 256 | INPP1 | NP_002185 |
| 205 | CLU | NP_001822 | 231 | FUT8 | NP_004471 | 257 | ISG20 | NP_002192 |

| SEQ ID# | Symbol | Genbank |
|---|---|---|
| 258 | ITGAX | NP_000878 |
| 259 | ITGB1 | NP_002202 |
| 260 | KATNA1 | NP_008975 |
| 261 | KLF2 | NP_057354 |
| 262 | KLRF1 | NP_057607 |
| 263 | LAMP1 | NP_005552 |
| 264 | LFNG | NP_002295 |
| 265 | LHFPL2 | NP_005770 |
| 266 | LILRB3 | NP_006855 |
| 267 | LTA4H | NP_000886 |
| 268 | LTF | NP_002334 |
| 269 | MAP4K2 | NP_004570 |
| 270 | MAPK14 | NP_001306 |
| 271 | MAPK8IP3 | NP_055948 |
| 272 | MCTP1 | NP_078993 |
| 273 | MEGF9 | NP_001073966 |
| 274 | METTL9 | NP_057109 |
| 275 | MFSD10 | NP_011111 |
| 276 | MICAL1 | NP_073602 |
| 277 | MMP8 | NP_002415 |
| 278 | MNT | NP_064706 |
| 279 | MRPS18B | NP_054765 |
| 280 | MUT | NP_000246 |
| 281 | MX1 | NP_002453 |
| 282 | MYL9 | NP_006088 |
| 283 | MYOM2 | NP_003961 |
| 284 | NAGK | NP_060037 |
| 285 | NMI | NP_004679 |
| 286 | NUPL2 | NP_031368 |
| 287 | OBFC1 | NP_079204 |
| 288 | OSBPL9 | NP_078862 |
| 289 | PAFAH2 | NP_000428 |
| 290 | PARL | NP_061092 |
| 291 | PDCD5 | NP_004699 |
| 292 | PDGFC | NP_057289 |
| 293 | PHB | NP_002625 |
| 294 | PHF3 | NP_055968 |
| 295 | PLAC8 | NP_057703 |
| 296 | PLEKHG3 | NP_056364 |
| 297 | PLEKHM2 | NP_055979 |
| 298 | POLR2C | NP_116558 |
| 299 | PPP1CA | NP_002699 |
| 300 | PPP1CB | NP_002700 |
| 301 | PPP1R11 | NP_068778 |
| 302 | PROS1 | NP_000304 |
| 303 | PRPF40A | NP_060362 |
| 304 | PRRG4 | NP_076986 |
| 305 | PSMB4 | NP_002787 |
| 306 | PSTPIP2 | NP_077748 |
| 307 | PTPN2 | NP_002819 |
| 308 | PUS3 | NP_112597 |
| 309 | RAB11FIP1 | NP_079427 |
| 310 | RAB11FIP3 | NP_055515 |
| 311 | RAB9A | NP_004242 |
| 312 | RANBP10 | NP_065901 |
| 313 | RASGRP2 | NP_722541 |
| 314 | RASGRP3 | NP_056191 |
| 315 | RASSF7 | NP_003466 |
| 316 | RDX | NP_002897 |
| 317 | RNASE6 | NP_005606 |
| 318 | RNF34 | NP_079402 |
| 319 | RPA2 | NP_002937 |
| 320 | RPS6KB2 | NP_003943 |
| 321 | RPS8 | NP_001003 |
| 322 | S100A12 | NP_005612 |
| 323 | S100P | NP_005971 |
| 324 | SASH3 | NP_061863 |
| 325 | SBF1 | NP_002963 |
| 326 | SDF2L1 | NP_071327 |
| 327 | SDHC | NP_002992 |
| 328 | SERTAD2 | NP_055570 |
| 329 | SH3BGRL | NP_003013 |
| 330 | SH3GLB2 | NP_064530 |
| 331 | SLAMF7 | NP_067004 |
| 332 | SLC11A2 | NP_000608 |
| 333 | SLC12A9 | NP_064631 |
| 334 | SLC25A37 | NP_057696 |
| 335 | SLC2A3 | NP_008862 |
| 336 | SLC39A8 | NP_071437 |
| 337 | SLC9A3R1 | NP_004243 |
| 338 | SNAPC1 | NP_003073 |

| SEQ ID# | Symbol | Genbank |
|---|---|---|
| 353 | TRAF3IP2 | NP_671733 |
| 354 | USP3 | NP_006528 |
| 355 | VAV1 | NP_005419 |
| 356 | WDR33 | NP_060853 |
| 357 | YPEL5 | NP_057145 |
| 358 | ZBTB17 | NP_003434 |

| SEQ ID# | Symbol | Genbank |
|---|---|---|
| 346 | TIMP1 | NP_003245 |
| 347 | TINF2 | NP_036593 |
| 348 | TLR5 | NP_003259 |
| 349 | TMEM106C | NP_076961 |
| 350 | TMEM80 | NP_777600 |
| 351 | TOB1 | NP_005740 |
| 352 | TPP2 | NP_003282 |

| SEQ ID# | Symbol | Genbank |
|---|---|---|
| 339 | SORT1 | NP_002950 |
| 340 | SSBP2 | NP_036578 |
| 341 | ST3GAL5 | NP_003887 |
| 342 | ST3GAL6 | NP_006091 |
| 343 | STK38 | NP_009202 |
| 344 | SYNE2 | NP_055995 |
| 345 | TAX1BP1 | NP_006015 |

The invention is further described by way of the following items:

1. A method for determining an indicator used in assessing a likelihood of a subject presenting to emergency having an absence of BaSIRS, the method comprising, consisting or consisting essentially of: (1) determining biomarker values that are measured or derived for at least two (e.g., 2, 3, 4, 5, 6, 7, 8, 9, 10, or more) corresponding rule out (RO) BaSIRS biomarkers in a sample taken from the subject and that is at least partially indicative of the levels of the RO BaSIRS biomarkers in the sample; and (2) determining the indicator using the biomarker values.

2. A method for ruling out the likelihood of BaSIRS (*i.e.,* for diagnosing the absence of BaSIRS), or not, for a subject presenting to emergency having an absence of BaSIRS, the method comprising, consisting or consisting essentially of: (1) determining biomarker values that are measured or derived for at least two (e.g., 2, 3, 4, 5, 6, 7, 8, 9, 10, or more) corresponding RO BaSIRS biomarkers in a sample taken from the subject and that is at least partially indicative of the levels of the RO BaSIRS biomarkers in the sample; (2) determining the indicator using the biomarker values; and (3) ruling out the likelihood of BaSIRS for the subject or not, based on the indicator.

3. The method of item 1 or item 2, wherein the subject has at least one clinical sign of systemic inflammatory response syndrome (SIRS).

4. The method of any one of items 1 to 3, wherein at the least two RO BaSIRS biomarkers are not biomarkers of at least one other SIRS condition (e.g., 1, 2, 3, 4 or 5 other SIRS conditions) selected from the group consisting of: autoimmune disease associated SIRS (ADaSIRS), cancer associated SIRS (CANaSIRS), trauma associated SIRS (TRAUMaSIRS), anaphylaxis associated SIRS (ANAPHYLaSIRS), schizophrenia associated SIRS (SCHIZaSIRS) and virus associated SIRS (VaSIRS).

5. The method of any one of items 1 to 3, wherein at the least two RO BaSIRS biomarkers are not biomarkers of any of the following SIRS conditions: ADaSIRS, CANaSIRS, TRAUMaSIRS, ANAPHYLaSIRS, SCHIZaSIRS and VaSIRS.

6. The method of any one of items 1 to 5, wherein the sample is a biological sample.

7. The method of item 6, wherein the biological sample is a blood sample.

8. The method of item 7, wherein the blood sample is a peripheral blood sample.

9. The method of any one of items 6 to 8, wherein the biological sample comprises leukocytes.

10. The method of any one of items 1 to 9, wherein the at least two RO BaSIRS biomarkers are expression products of a gene selected from the group consisting of: ADAM19, ADD1, ADGRE1, AIF1, AKAP7, AKT1, AKTIP, ALDOA, AMD1, ARL2BP, ATG9A, ATP13A3, ATP6VOA1, ATP8B4, BRD7, BTG2, C21orf59, C6orf48, CCND2, CD44, CD59, CDC14A, CERK, CHPT1, CLEC4E, CLU, CNBP, COMMD4, COQ10B, COX5B, CPVL, CTDSP2, CTSA, CTSC, CTSH, CYBB, CYP20A1, DERA, DHX16, DIAPH2, DLST, EIF4A2, EIF4E2, EMP3, ENO1, FBXO7, FCER1G, FGL2, FLVCR2, FTL, FURIN, FUT8, FXR1, GAPDH, GAS7, GBP2, GIMAP4, GLOD4, GNS, GRAP2, GSTO1, HEBP1, HIST1H2BM, HIST1H3C, HIST1H4L, HLA-DPA1, HMG20B, HMGN4, HOXB6, HSPA4, ID3, IFIT1, IFNGR2, IL7R, IMP3, IMPDH1, INPP1, ISG20, ITGAX, ITGB1, KATNA1, KLF2, KLRF1, LAMP1, LFNG, LHFPL2, LILRB3, LTA4H, LTF, MAP4K2, MAPK14, MAPK8IP3, MCTP1, MEGF9, METTL9, MFSD10, MICAL1, MMP8, MNT, MRPS18B, MUT, MX1, MYL9, MYOM2, NAGK, NMI, NUPL2, OBFC1, OSBPL9, PAFAH2, PARL, PDCD5, PDGFC, PHB, PHF3, PLAC8, PLEKHG3, PLEKHM2, POLR2C, PPP1CA, PPP1CB, PPP1R11, PROS1, PRPF40A, PRRG4, PSMB4, PSTPIP2, PTPN2, PUS3, RAB11FIP1, RAB11FIP3, RAB9A, RANBP10, RASGRP2, RASGRP3, RASSF7, RDX, RNASE6, RNF34, RPA2, RPS6KB2, RPS8, S100A12, S100P, SASH3, SBF1, SDF2L1, SDHC, SERTAD2, SH3BGRL, SH3GLB2, SLAMF7, SLC11A2, SLC12A9, SLC25A37, SLC2A3, SLC39A8, SLC9A3R1, SNAPC1, SORT1, SSBP2, ST3GAL5, ST3GAL6, STK38, SYNE2, TAX1BP1, TIMP1, TINF2, TLR5, TMEM106C, TMEM80, TOB1, TPP2, TRAF3IP2, USP3, VAV1, WDR33, YPEL5, ZBTB17.

11. The method of item 10, wherein an individual RO BaSIRS biomarker is selected from the group consisting of: (a) a polynucleotide expression product comprising a nucleotide sequence that shares at least 70% (or at least 71% to at least 99% and all integer percentages in between) sequence identity with the sequence set forth in any one of SEQ ID NO: 1-179, or a complement thereof; (b) a polynucleotide expression product comprising a nucleotide sequence that encodes a polypeptide comprising the amino acid sequence set forth in any one of SEQ ID NO: 180-358; (c) a polynucleotide expression product comprising a nucleotide sequence that encodes a polypeptide that shares at least 70% (or at least 71% to at least 99% and all integer percentages in between) sequence similarity or identity with at least a portion of the sequence set forth in SEQ ID NO: 180-358; (d) a polynucleotide expression product comprising a nucleotide sequence that hybridizes to the sequence of (a), (b), (c) or a complement thereof, under medium or high stringency conditions; (e) a polypeptide expression product comprising the amino acid sequence set forth in any one of SEQ ID NO: 180-358; and (f) a polypeptide expression product comprising an amino acid sequence that shares at least 70% (or at least 71% to at least 99% and all integer percentages in between) sequence similarity or identity with the sequence set forth in any one of SEQ ID NO: 180-358.

12. The method of item 10 or item 11, wherein an individual RO BaSIRS biomarker comprises, consists, or consists essentially of: a nucleotide sequence selected from the group consisting of SEQ ID NOs: 1-179.

13. The method of item 10 or item 11, wherein an individual RO BaSIRS biomarker comprises, consists or consists essentially of an amino acid sequence selected from the group consisting of SEQ ID NOs: 180-358.

14. The method of any one of items 1 to 13, wherein at least one pair of RO BaSIRS biomarkers is used to determine the indicator.

15. The method of item 14, wherein one biomarker of a biomarker pair is selected from Group A RO BaSIRS biomarkers and the other is selected from Group B RO BaSIRS biomarkers, wherein an individual Group A RO BaSIRS biomarker is an expression product of a gene selected from the group consisting of: *DIAPH2, CYBB, SLC39A8, PRPF40A, MUT, NMI, PUS3, MNT, SLC11A2, FXR1, SNAPC1, PRRG4, SLAMF7, MAPK8IP3, GBP2, PPP1CB, TMEM80, HIST1H2BM, NAGK, HIST1H4L* and wherein an individual Group B RO BaSIRS biomarker is an expression product of a gene selected from the group consisting of: *SERTAD2, PHF3, BRD7, TOB1, MAP4K2, WDR33, BTG2, AMD1, RNASE6, RAB11FIP1, ADD1, HMG20B.*

16. The method of item 14 or item 15, wherein one biomarker of a biomarker pair is selected from Group C RO BaSIRS biomarkers and the other is selected from Group D RO BaSIRS biomarkers, wherein an individual Group C RO BaSIRS biomarker is an expression product of a gene selected from the group consisting of: *PARL, AIF1, PTPN2, COX5B, PSMB4, EIF4E2, RDX, DERA, CTSH, HSPA4, VAV1, PPP1CA, CPVL, PDCD5,* and wherein an individual Group D RO BaSIRS biomarker is an expression product of a gene selected from the group consisting of: *PAFAH2, IMP3, GLOD4, IL7R, ID3, KLRF1, SBF1, CCND2, LFNG, MRPS18B, HLA-DPA1, SLC9A3R1, HMGN4, C6orf48, ARL2BP, CDC14A, RPA2, ST3GAL5, EIF4A2, CERK, RASSF7, PHB, TRAF3IP2, KLF2, RAB11FIP3, C21orf59, SSBP2, GIMAP4, CYP20A1, RASGRP2, AKT1, HCP5, TPP2, SYNE2, FUT8, NUPL2, MYOM2, RPS8, RNF34, DLST, CTDSP2, EMP3, PLEKHG3, DHX16, RASGRP3, COMMD4, ISG20, POLR2C, SH3GLB2, SASH3, GRAP2, RPS6KB2, FGL2, AKAP7, SDF2L1, FBXO7, MX1, IFIT1, TMEM106C, RANBP10.*

17. The method of any one of items 14 to 16, wherein one biomarker of a biomarker pair is selected from Group E RO BaSIRS biomarkers and the other is selected from Group F RO BaSIRS biomarkers, wherein an individual Group E RO BaSIRS biomarker is an expression product of a gene selected from the group consisting of: *SORT1, GAS7, FLVCR2, TLR5, FCER1G, SLC2A3, S100A12, PSTPIP2, GNS, METTL9, MMP8, MAPK14, CD59, CLEC4E, MICAL1, MCTP1, GAPDH, IMPDH1, ATP8B4, EMR1, SLC12A9, S100P, IFNGR2, PDGFC, CTSA, ALDOA, ITGAX, GSTO1, LHFPL2, LTF, SDHC, TIMP1, LTA4H, USP3, MEGF9, FURIN, ATP6V0A1, PROS1, ATG9A, PLAC8, LAMP1, COQ10B, ST3GAL6, CTSC, ENO1, OBFC1, TAX1BP1, MYL9, HIST1H3C, ZBTB17, CHPT1, SLC25A37, PLEKHM2, LILRB3, YPEL5, FTL, SH3BGRL, HOXB6, PPP1R11, CLU, HEBP1,* and wherein an individual Group F RO BaSIRS biomarker is an expression product of a gene selected from the group consisting of: *OSBPL9, CD44, AKTIP, ATP13A3, ADAM19, KATNA1, STK38, TINF2, RAB9A, INPP1, CNBP, ITGB1, MFSD10.*

18. The method of any one of items 15, wherein biomarker values are measured or derived for a Group A RO BaSIRS biomarker and for a Group B RO BaSIRS biomarker, and the indicator is determined by combining the biomarker values.

19. The method of any one of items 16, wherein biomarker values are measured or derived for a Group C RO BaSIRS biomarker and for a Group D RO BaSIRS biomarker, and the indicator is determined by combining the biomarker values.

20. The method of any one of items 17, wherein biomarker values are measured or derived for a Group E RO BaSIRS biomarker and for a Group F RO BaSIRS biomarker, and the indicator is determined by combining the biomarker values.

21. The method of item 15, wherein biomarker values are measured or derived for a Group A RO BaSIRS biomarker, for a Group B RO BaSIRS biomarker, for a Group C RO BaSIRS biomarker and for a Group D RO BaSIRS biomarker, and the indicator is determined by combining the biomarker values.

22. The method of item 16, wherein biomarker values are measured or derived for a Group A RO BaSIRS biomarker, for a Group B RO BaSIRS biomarker, for a Group C RO BaSIRS biomarker, for a Group D RO BaSIRS biomarker, for a Group E RO BaSIRS biomarker, for a Group F RO BaSIRS biomarker, and the indicator is determined by combining the biomarker values.

23. The method of any one of items 14 to 22, wherein the method further comprises combining the biomarker values using a combining function, wherein the combining function is at least one of: an additive model; a linear model; a support vector machine; a neural network model; a random forest model; a regression model; a genetic algorithm; an annealing algorithm; a weighted sum; a nearest neighbor model; and a probabilistic model.

24. The method of any one of items 14 to 23, wherein the method comprises: (a) determining a pair of biomarker values, each biomarker value being a value measured or derived for at least one corresponding RO BaSIRS biomarker; (b) determining a derived biomarker value using the pair of biomarker values, the derived biomarker value being indicative of a ratio of concentrations of the pair of RO BaSIRS biomarkers; and determining the indicator using the derived marker value.

25. The method of item 24, wherein biomarker values are measured or derived for a Group A RO BaSIRS biomarker and for a Group B RO BaSIRS biomarker to obtain the pair of biomarker values and the derived biomarker value is determined using the pair of biomarker values.

26. The method of item 24, wherein biomarker values are measured or derived for a Group C RO BaSIRS biomarker and for a Group D RO BaSIRS biomarker to obtain the pair of biomarker values and the derived biomarker value is determined using the pair of biomarker values.

27. The method of item 24, wherein biomarker values are measured or derived for a Group E RO BaSIRS biomarker and for a Group F RO BaSIRS biomarker to obtain the pair of biomarker values and the derived biomarker value is determined using the pair of biomarker values.

28. The method of any one of items 14 to 27, wherein the method comprises: (a) determining a first derived biomarker value using a first pair of biomarker values, the first derived biomarker value being indicative of a ratio of concentrations of first and second RO BaSIRS biomarkers; (b) determining a second derived biomarker value using a second pair of biomarker values, the second derived biomarker value being indicative of a ratio of concentrations of third and fourth RO BaSIRS biomarkers; (c) determining a third derived biomarker value using a third pair of biomarker values, the third derived biomarker value being indicative of a ratio of concentrations of fifth and sixth RO BaSIRS biomarkers; and (d) determining the indicator by combining the first, second and third derived biomarker values.

29. The method of item 28, wherein the first RO BaSIRS biomarker is selected from Group A RO BaSIRS biomarkers, the second RO BaSIRS biomarker is selected from Group B RO BaSIRS biomarkers, the third RO BaSIRS biomarker is selected from Group C RO BaSIRS biomarkers, the fourth RO BaSIRS biomarker is selected from Group D RO BaSIRS biomarkers, the fifth RO BaSIRS biomarker is selected from Group E RO BaSIRS biomarkers, and the sixth RO BaSIRS biomarker is selected from Group F RO BaSIRS biomarkers.

30. The method of item 28 or item 29, wherein the method comprises combining the biomarker values using a combining function, wherein the combining function is at least one of: an additive model; a linear model; a support vector machine; a neural network model; a random forest model; a regression model; a genetic algorithm; an annealing algorithm; a weighted sum; a nearest neighbor model; and a probabilistic model.

31. The method of any one of items 14 to 30, wherein an individual pair of RO BaSIRS biomarkers has a mutual correlation in respect of ruling out BaSIRS that lies within a mutual correlation range, the mutual correlation range being between $\pm 0.9$ (or between $\pm 0.8$, $\pm 0.7$, $\pm 0.6$, $\pm 0.5$, $\pm 0.4$, $\pm 0.3$, $\pm 0.2$ or $\pm 0.1$) and the indicator has a performance value greater than or equal to a performance threshold representing the ability of the indicator to diagnose the absence of BaSIRS, wherein the performance threshold is indicative of an explained variance of at least 0.3.

32. The method of any one of items 14 to 31, wherein an individual RO BaSIRS biomarker has a condition correlation with the absence of RO BaSIRS that lies outside a condition correlation range, wherein the condition correlation range is between $\pm 0.3$.

33. The method of any one of items 14 to 31, wherein an individual RO BaSIRS biomarker has a condition correlation with the absence of BaSIRS that lies outside a condition correlation range, wherein the condition correlation range is at least one of $\pm 0.9$, $\pm 0.8$, $\pm 0.7$, $\pm 0.6$, $\pm 0.5$ or $\pm 0.4$.

34. The method of any one of items 14 to 33, wherein the performance threshold is indicative of an explained variance of at least one of 0.4, 0.5, 0.6, 0.7, 0.8 and 0.9.

35. The method of any one of items 14 to 34, wherein the Group A RO BaSIRS biomarker is an expression product of *DIAPH2,* the Group B RO BaSIRS biomarker is an expression product of *SERTAD2,* the Group C RO BaSIRS biomarker is an expression product of *PARL,* the Group D RO BaSIRS biomarker is an expression product of *PAFAH2,* the Group E RO BaSIRS biomarker is an expression product of *SORT1,* and the Group F RO BaSIRS biomarker is an expression product of *OSBPL9.*

36. An apparatus for determining an indicator used in assessing a likelihood of a subject having an absence of BaSIRS, the apparatus comprising at least one electronic processing device that:

> a) determines a pair of biomarker values, each biomarker value being a value measured or derived for at least one corresponding RO BaSIRS biomarker, as broadly described above and elsewhere herein, of a sample taken from the subject and being at least partially indicative of a concentration of the RO BaSIRS biomarker in the sample;
> b) determines a derived biomarker value using the pair of biomarker values, the derived biomarker value being indicative of a ratio of concentrations of the pair of RO BaSIRS biomarkers; and
> c) determines the indicator using the derived biomarker value.

37. A composition for determining an indicator used in assessing a likelihood of a subject having an absence of BaSIRS, the composition comprising, consisting, or consisting essentially of, at least one pair of cDNAs and at least one oligonucleotide primer or probe that hybridizes to an individual one of the cDNAs, wherein the at least one pair of cDNAs is selected from pairs of cDNA including a first pair, a second pair and a third pair of cDNAs, wherein the first pair comprises a Group A RO BaSIRS biomarker cDNA and a Group B RO BaSIRS biomarker cDNA, and wherein the second pair comprises a Group C RO BaSIRS biomarker cDNA and a Group D RO BaSIRS biomarker cDNA, and wherein the third pair comprises a Group E RO BaSIRS biomarker cDNA and a Group F RO BaSIRS biomarker cDNA, wherein an individual Group A RO BaSIRS biomarker is an expression product of a gene selected from the group consisting of: *DIAPH2, CYBB, SLC39A8, PRPF40A, MUT, NMI, PUS3, MNT, SLC11A2, FXR1, SNAPC1, PRRG4, SLAMF7, MAPK8IP3, GBP2, PPP1CB, TMEM80, HIST1H2BM, NAGK, HIST1H4L,* wherein an individual Group B RO BaSIRS biomarker is an expression product of a gene selected from the group consisting of: *SERTAD2, PHF3, BRD7, TOB1, MAP4K2, WDR33, BTG2, AMD1, RNASE6, RAB11FIP1, ADD1, HMG20B,* wherein an individual Group C RO BaSIRS biomarker is an expression product of a gene selected from the group consisting of: *PARL, AIF1, PTPN2, COX5B, PSMB4, EIF4E2, RDX, DERA, CTSH, HSPA4, VAV1, PPP1CA, CPVL, PDCD5,* wherein an individual Group D RO BaSIRS biomarker is an expression product of a gene selected from the group consisting of: *PAFAH2, IMP3, GLOD4, IL7R, ID3, KLRF1, SBF1, CCND2, LFNG, MRPS18B, HLA-DPA1, SLC9A3R1, HMGN4, C6orf48, ARL2BP, CDC14A, RPA2, ST3GAL5, EIF4A2, CERK, RASSF7, PHB, TRAF3IP2, KLF2, RAB11FIP3, C21orf59, SSBP2, GIMAP4, CYP20A1, RASGRP2, AKT1, HCP5, TPP2, SYNE2, FUT8, NUPL2, MYOM2, RPS8, RNF34, DLST, CTDSP2, EMP3, PLEKHG3, DHX16, RASGRP3, COMMD4, ISG20, POLR2C, SH3GLB2, SASH3, GRAP2, RPS6KB2, FGL2, AKAP7, SDF2L1, FBX07, MX1, IFIT1, TMEM106C, RANBP10,* wherein an individual Group E RO BaSIRS biomarker is an expression product of a gene selected from the group consisting of: *SORT1, GAS7, FLVCR2, TLR5, FCER1G, SLC2A3, S100A12, PSTPIP2, GNS, METTL9, MMP8, MAPK14, CD59, CLEC4E, MICAL1, MCTP1, GAPDH, IMPDH1, ATP8B4, EMR1, SLC12A9, S100P, IFNGR2, PDGFC, CTSA, ALDOA, ITGAX, GSTO1, LHFPL2, LTF, SDHC, TIMP1, LTA4H, USP3, MEGF9, FURIN, ATP6V0A1, PROS1, ATG9A, PLAC8, LAMP1, COQ10B, ST3GAL6, CTSC, ENO1, OBFC1, TAX1BP1, MYL9, HIST1H3C, ZBTB17, CHPT1, SLC25A37, PLEKHM2, LILRB3, YPEL5, FTL, SH3BGRL, HOXB6, PPP1R11, CLU, HEBP1,* and wherein an individual Group F RO BaSIRS biomarker is an expression product of a gene selected from the group consisting of: *OSBPL9, CD44, AKTIP, ATP13A3, ADAM19, KATNA1, STK38, TINF2, RAB9A, INPP1, CNBP, ITGB1, MFSD10.*

38. The composition of item 37, comprising a population of cDNAs corresponding to mRNA derived from a cell or cell population.

39. The composition of item 38, wherein the cell is a cell of the immune system.

40. The composition of item 38, wherein the cell is a leukocyte.

41. The composition of any one of items 38 to 40, wherein the cell population is blood.

42. The composition of any one of items 38 to 40, wherein the cell population is peripheral blood.

43. The composition of any one of items 38 to 42, wherein the at least one oligonucleotide primer or probe is hybridized to an individual one of the cDNAs.

44. The composition of any one of items 38 to 43, further comprising a labeled reagent for detecting the cDNA.

45. The composition of item 44, wherein the labeled reagent is a labeled said at least one oligonucleotide primer or probe.

46. The composition of item 44, wherein the labeled reagent is a labeled said cDNA.

47. The composition of any one of items 38 to 46, wherein the at least one oligonucleotide primer or probe is in a form other than a high density array.

48. A kit for determining an indicator which is indicative of the likelihood of the absence of BaSIRS, and on which the likelihood of BaSIRS is ruled out or not, the kit comprising, consisting or consisting essentially of at least one pair of reagents selected from reagent pairs including a first pair of reagents, a second pair of reagents and a third pair of reagents, wherein the first pair of reagents comprises (i) a reagent that allows quantification of a Group A RO BaSIRS biomarker; and (ii) a reagent that allows quantification of a Group B RO BaSIRS biomarker, wherein the second pair of reagents comprises: (iii) a reagent that allows quantification of a Group C RO BaSIRS biomarker; and (iv) a reagent that allows quantification of a Group D RO BaSIRS biomarker, and wherein the third pair of reagents comprises: (v) a reagent that allows quantification of a Group E RO BaSIRS biomarker; and (vi) a reagent that allows quantification of a Group F RO BaSIRS biomarker, wherein an individual Group A RO BaSIRS biomarker is an expression product of a gene selected from the group consisting of: *DIAPH2, CYBB, SLC39A8, PRPF40A, MUT, NMI, PUS3, MNT, SLC11A2, FXR1, SNAPC1, PRRG4, SLAMF7, MAPK8IP3, GBP2, PPP1CB, TMEM80, HIST1H2BM, NAGK, HIST1H4L,* wherein an individual Group B RO BaSIRS biomarker is an expression product of a gene selected from the group consisting of: *SERTAD2, PHF3, BRD7, TOB1, MAP4K2, WDR33, BTG2, AMD1, RNASE6, RAB11FIP1, ADD1, HMG20B,* wherein an individual Group C RO BaSIRS biomarker is an expression product of a gene selected from the group consisting of: *PARL, AIF1, PTPN2, COX5B, PSMB4, EIF4E2, RDX, DERA, CTSH, HSPA4, VAV1, PPP1CA, CPVL, PDCD5,* wherein an individual Group D RO BaSIRS biomarker is an expression product of a gene selected from the group consisting of: *PAFAH2, IMP3, GLOD4, IL7R, ID3, KLRF1, SBF1, CCND2, LFNG, MRPS18B, HLA-DPA1, SLC9A3R1, HMGN4, C6orf48, ARL2BP, CDC14A, RPA2, ST3GAL5, EIF4A2, CERK, RASSF7, PHB, TRAF3IP2, KLF2, RAB11FIP3, C21orf59, SSBP2, GIMAP4, CYP20A1, RASGRP2, AKT1, HCP5, TPP2, SYNE2, FUT8, NUPL2, MYOM2, RPS8, RNF34, DLST, CTDSP2, EMP3, PLEKHG3, DHX16,*

*RASGRP3, COMMD4, ISG20, POLR2C, SH3GLB2, SASH3, GRAP2, RPS6KB2, FGL2, AKAP7, SDF2L1, FBX07, MX1, IFIT1, TMEM106C, RANBP10,* wherein an individual Group E RO BaSIRS biomarker is an expression product of a gene selected from the group consisting of: *SORT1, GAS7, FLVCR2, TLR5, FCER1G, SLC2A3, S100A12, PSTPIP2, GNS, METTL9, MMP8, MAPK14, CD59, CLEC4E, MICAL1, MCTP1, GAPDH, IMPDH1, ATP8B4, EMR1, SLC12A9, S100P, IFNGR2, PDGFC, CTSA, ALDOA, ITGAX, GSTO1, LHFPL2, LTF, SDHC, TIMP1, LTA4H, USP3, MEGF9, FURIN, ATP6V0A1, PROS1, ATG9A, PLAC8, LAMP1, COQ10B, ST3GAL6, CTSC, ENO1, OBFC1, TAX1BP1, MYL9, HIST1H3C, ZBTB17, CHPT1, SLC25A37, PLEKHM2, LILRB3, YPEL5, FTL, SH3BGRL, HOXB6, PPP1R11, CLU, HEBP1,* and wherein an individual Group F RO BaSIRS biomarker is an expression product of a gene selected from the group consisting of: *OSBPL9, CD44, AKTIP, ATP13A3, ADAM19, KATNA1, STK38, TINF2, RAB9A, INPP1, CNBP, ITGB1, MFSD10.*

49. A method for managing a subject with at least one clinical sign of SIRS, the method comprising, consisting or consisting essentially of: not exposing the subject to a treatment regimen for specifically treating BaSIRS based on an indicator obtained from an indicator-determining method, wherein the indicator is indicative of the absence of BaSIRS in the subject, and of ruling out the likelihood of the presence of BaSIRS in the subject, and wherein the indicator-determining method is an indicator-determining method according to any one of items 1 to 35.

50. The method of item 49, wherein when the indicator is indicative of the absence of BaSIRS in the subject, the method further comprises exposing the subject to a non-BaSIRS treatment.

51. The method of item 50, wherein the non-BaSIRS treatment is a treatment for a SIRS other than BaSIRS (e.g., a treatment for ADaSIRS, CANaSIRS, TRAUMaSIRS, ANAPHYLaSIRS, SCHIZaSIRS and VaSIRS).

52. The method of item 49, wherein when the indicator is indicative of the absence of BaSIRS in the subject, the methods further comprises not exposing the subject to a treatment.

53. The method of any one of items 49 to 52, further comprising taking a sample from the subject and determining an indicator indicative of the likelihood of the absence of BaSIRS using the indicator-determining method.

54. The method of any one of items 49 to 53, further comprising sending a sample taken from the subject to a laboratory at which the indicator is determined according to the indicator-determining method.

55. The method of item 54, further comprising receiving the indicator from the laboratory.

**Claims**

1. A method for determining an indicator used in assessing a likelihood of a subject presenting to emergency having an absence of bacterial associated systemic inflammatory response syndrome (BaSIRS), the method comprising:

   (1) determining biomarker values that are measured or derived for at least two corresponding rule out (RO) BaSIRS biomarkers in a sample taken from the subject and that are at least partially indicative of the levels of the RO BaSIRS biomarkers in the sample, wherein the at least two RO BaSIRS biomarkers are expression products of GBP2 and a gene selected from the group consisting of: ADAM19, ADD1, ADGRE1, AIF1, AKAP7, AKT1, AKTIP, ALDOA, AMD1, ARL2BP, ATG9A, ATP13A3, ATP6V0A1, ATP8B4, BRD7, BTG2, C21orf59, C6orf48, CCND2, CD44, CD59, CDC14A, CERK, CHPT1, CLEC4E, CLU, CNBP, COMMD4, COQ10B, COX5B, CPVL, CTDSP2, CTSA, CTSC, CTSH, CYBB, CYP20A1, DERA, DHX16, DIAPH2, DLST, EIF4A2, EIF4E2, EMP3, ENO1, FBXO7, FCER1G, FGL2, FLVCR2, FTL, FURIN, FUT8, FXR1, GAPDH, GAS7, GIMAP4, GLOD4, GNS, GRAP2, GSTO1, HEBP1, HIST1H2BM, HIST1H3C, HIST1H4L, HLA-DPA1, HMG20B, HMGN4, HOXB6, HSPA4, ID3, IFIT1, IFNGR2, IL7R, IMP3, IMPDH1, INPP1, ISG20, ITGAX, ITGB1, KATNA1, KLF2, KLRF1, LAMP1, LFNG, LHFPL2, LILRB3, LTA4H, LTF, MAP4K2, MAPK14, MAPK8IP3, MCTP1, MEGF9, METTL9, MFSD10, MICAL1, MMP8, MNT, MRPS18B, MUT, MX1, MYL9, MYOM2, NAGK, NMI, NUPL2, OBFC1, OSBPL9, PAFAH2, PARL, PDCD5, PDGFC, PHB, PHF3, PLAC8, PLEKHG3, PLEKHM2, POLR2C, PPP1CA, PPP1CB, PPP1R11, PROS1, PRPF40A, PRRG4, PSMB4, PSTPIP2, PTPN2, PUS3, RAB11FIP1, RAB11FIP3, RAB9A, RANBP10, RASGRP2, RASGRP3, RASSF7, RDX, RNASE6, RNF34, RPA2, RPS6KB2, RPS8, S100A12, S100P, SASH3, SBF1, SDF2L1, SDHC, SERTAD2, SH3BGRL, SH3GLB2, SLAMF7, SLC11A2, SLC12A9, SLC25A37, SLC2A3, SLC39A8, SLC9A3R1, SNAPC1, SORT1, SSBP2, ST3GAL5, ST3GAL6, STK38, SYNE2, TAX1BP1, TIMP1, TINF2, TLR5, TMEM106C, TMEM80, TOB1, TPP2, TRAF3IP2, USP3, VAV1, WDR33, YPEL5, ZBTB17; and
   (2) determining the indicator using the biomarker values,
   wherein at least one pair of RO BaSIRS biomarkers is used to determine the indicator, wherein one biomarker of the biomarker pair is an expression product of GBP2 and the other is an expression product of a gene selected from the group consisting of GIMAP4, HCP5, MX1 and TLR5.

2. A method for ruling out the likelihood of bacterial associated systemic inflammatory response syndrome (BaSIRS), or not, for a subject presenting to emergency having an absence of BaSIRS, the method comprising, consisting or consisting essentially of:

> (1) determining biomarker values that are measured or derived for at least two corresponding RO BaSIRS biomarkers in a sample taken from the subject and that are at least partially indicative of the levels of the RO BaSIRS biomarkers in the sample, wherein the at least two RO BaSIRS biomarkers are expression products of GBP2 and a gene selected from the group consisting of: ADAM19, ADD1, ADGRE1, AIF1, AKAP7, AKT1, AKTIP, ALDOA, AMD1, ARL2BP, ATG9A, ATP13A3, ATP6V0A1, ATP8B4, BRD7, BTG2, C21orf59, C6orf48, CCND2, CD44, CD59, CDC14A, CERK, CHPT1, CLEC4E, CLU, CNBP, COMMD4, COQ10B, COX5B, CPVL, CTDSP2, CTSA, CTSC, CTSH, CYBB, CYP20A1, DERA, DHX16, DIAPH2 DLST, EIF4A2, EIF4E2, EMP3, ENO1, FBXO7, FCER1G, FGL2, FLVCR2, FTL, FURIN, FUT8, FXR1, GAPDH, GAS7, GBP2, GIMAP4, GLOD4, GNS, GRAP2, GSTO1, HEBP1, HIST1H2BM, HIST1H3C, HIST1H4L, HLA-DPA1, HMG20B, HMGN4, HOXB6, HSPA4, ID3, IFIT1, IFNGR2, IL7R, IMP3, IMPDH1, INPP1, ISG20, ITGAX, ITGB1, KATNA1, KLF2, KLRF1, LAMP1, LFNG, LHFPL2, LILRB3, LTA4H, LTF, MAP4K2, MAPK14, MAPK8IP3, MCTP1, MEGF9, METTL9, MFSD10, MICAL1, MMP8, MNT, MRPS18B, MUT, MX1, MYL9, MYOM2, NAGK, NMI, NUPL2, OBFC1, OSBPL9, PAFAH2, PARL, PDCD5, PDGFC, PHB, PHF3, PLAC8, PLEKHG3, PLEKHM2, POLR2C, PPP1CA, PPP1CB, PPP1R11, PROS1, PRPF40A, PRRG4, PSMB4, PSTPIP2, PTPN2, PUS3, RAB11FIP1, RAB11FIP3, RAB9A, RANBP10, RASGRP2, RASGRP3, RASSF7, RDX, RNASE6, RNF34, RPA2, RPS6KB2, RPS8, S100A12, S100P, SASH3, SBF1, SDF2L1, SDHC, SERTAD2, SH3BGRL, SH3GLB2, SLAMF7, SLC11A2, SLC12A9, SLC25A37, SLC2A3, SLC39A8, SLC9A3R1, SNAPC1, SORT1, SSBP2, ST3GAL5, ST3GAL6, STK38, SYNE2, TAX1BP1, TIMP1, TINF2, TLR5, TMEM106C, TMEM80, TOB1, TPP2, TRAF3IP2, USP3, VAV1, WDR33, YPEL5, ZBTB17;
> (2) determining the indicator using the biomarker values,
> wherein at least one pair of RO BaSIRS biomarkers is used to determine the indicator, wherein one biomarker of the biomarker pair is an expression product of GBP2 and the other is an expression product of a gene selected from the group consisting of GIMAP4, HCP5, MX1 and TLR5; and
> (3) ruling out the likelihood of BaSIRS for the subject or not, based on the indicator.

3. The method of claim 1 or claim 2, wherein one biomarker of the biomarker pair is an expression product of GBP2 and the other is an expression product of GIMAP4 or of TLR5.

4. The method of any one of claims 1 to 3, wherein the subject has at least one clinical sign of systemic inflammatory response syndrome (SIRS) and wherein the sample is a peripheral blood sample.

5. The method of any one of claims 1 to 4, wherein the method comprises:

> (a) determining a pair of biomarker values, each biomarker value being a value measured or derived for at least one corresponding RO BaSIRS biomarker;
> (b) determining a derived biomarker value using the pair of biomarker values, the derived biomarker value being indicative of a ratio of concentrations of the pair of RO BaSIRS biomarkers; and determining the indicator using the derived biomarker value.

6. The method of claim 5, wherein the method comprises: (a) determining a first derived biomarker value using a first pair of biomarker values, the first derived biomarker value being indicative of a ratio of concentrations of first and second RO BaSIRS biomarkers; (b) determining a second derived biomarker value using a second pair of biomarker values, the second derived biomarker value being indicative of a ratio of concentrations of third and fourth RO BaSIRS biomarkers; (c) determining a third derived biomarker value using a third pair of biomarker values, the third derived biomarker value being indicative of a ratio of concentrations of fifth and sixth RO BaSIRS biomarkers; and (d) determining the indicator by combining the first, second and third derived biomarker values.

7. The method of claim 6, wherein the method comprises combining the biomarker values using a combining function, preferably wherein the combining function is at least one of: an additive model; a linear model; a support vector machine; a neural network model; a random forest model; a regression model; a genetic algorithm; an annealing algorithm; a weighted sum; a nearest neighbor model; and a probabilistic model.

8. The method of claim 6 or claim 7, wherein the pairs of biomarkers are expression products of gene pairs selected from the group consisting of: AIF1:HMGN4, ALDOA:MAP4K2, ATG9A:RAB11FIP3, ATP13A3:IL7R, ATP6V0A1:RASSF7, ATP8B4:CCND2, CD44:GIMAP4, CD44:HLA-DPA1, CD44:IL7R, CD44:RPA2, CD59:GIMAP4, CDC14A:CCND2, CDC14A:IL7R, CHPT1:FBXO7, CHPT1:RANBP10, CLEC4E:MX1, CLEC4E:SYNE2, CLU:CCND2, CLU:IL7R, COQ10B:TRAF3IP2, COX5B:PHB, CPVL:IL7R, CTSA:DLST, CTSA:HMG20B, CTSC:CCND2, CTSH:IL7R, CYBB:BRD7, DERA:HMGN4, DIAPH2:CCND2, DIAPH2:HLA-DPA1, DIAPH2:IL7R, DIAPH2:PHF3, DIAPH2:RAB9A, DIAPH2:RNASE6, DIAPH2:SERTAD2, DIAPH2:ST3GAL5, DIAPH2:STK38, EIF4E2:C21orf59, EMR1:AKT1, ENO1:IL7R, FCER1G:CD44, FCER1G:CDC14A, FCER1G:MX1, FCER1G:SDHC, FLVCR2:KATNA1, FTL:CCND2, FTL:IL7R, FURIN:ADD1, FURIN:BTG2, FURIN:RANBP10, FURIN:SH3GLB2, FUT8:IL7R, FXR1:EIF4A2, GAPDH:COMMD4, GAPDH:PPP1CA, GAPDH:RPS6KB2, GAS7:ADD1, GAS7:RAB11FIP1, GBP2:GIMAP4, GBP2:HCP5, GBP2:MX1, GNS:PLEKHG3, GSTO1:RASGRP3, GSTO1:SDF2L1, HEBP1:SSBP2, HIST1H2BM:CCND2, HIST1H2BM:IL7R, HIST1H3C:IL7R, HOXB6:PAFAH2, HSPA4:IMP3, IFNGR2:CCND2, IFNGR2:HLA-DPA1, IFNGR2:IL7R, IMPDH1:BTG2, ITGAX:RASGRP2, ITGB1:IL7R, LAMP1:HLA-DPA1, LAMP1:IL7R, LHFPL2:ISG20, LILRB3:IL7R, LTA4H:CCND2, LTA4H:CERK, LTA4H:CPVL, LTA4H:RPS8, LTA4H:ST3GAL5, LTA4H:TMEM106C, LTA4H:WDR33, LTF:MAP4K2, MAPK14:GIMAP4, MAPK14:IL7R, MAPK14:MX1, MAPK8IP3:IMP3, MCTP1:AMD1, MCTP1:TOB1, MEGF9:CCND2, MEGF9:CDC14A, MEGF9:GIMAP4, MEGF9:HLA-DPA1, MEGF9:IL7R, METTL9:AKTIP, MICAL1:DHX16, MICAL1:STK38, MMP8:CCND2, MMP8:CD44, MMP8:CTSC, MMP8:ENO1, MMP8:FGL2, MMP8:FTL, MMP8:FUT8, MMP8:IL7R, MMP8:ITGB1, MMP8:LAMP1, MMP8:PLAC8, MMP8:RPS8, MMP8:SDHC, MMP8:ST3GAL5, MMP8:TMEM106C, MMP8:TPP2, MMP8:VAV1, MNT:KLF2, MNT:SLC9A3R1, MUT:NUPL2, MYL9:GRAP2, MYL9:KLF2, NMI:MX1, OBFC1:C6orf48, PARL:PAFAH2, PDGFC:CCND2, PDGFC:FUT8, PDGFC:IL7R, PDGFC:ITGB1, PLEKHM2:SBF1, PPP1CB:PAFAH2, PROS1:MYOM2, PROS1:WDR33, PRPF40A:MRPS18B, PRRG4:GLOD4, PSMB4:IMP3, PSTPIP2:AKAP7, PTPN2:CYP20A1, PUS3:PAFAH2, S100A12:POLR2C, S100P:GIMAP4, S100P:HLA-DPA1, S100P:IL7R, SH3BGRL:GLOD4, SLC11A2:ID3, SLC12A9:CTDSP2, SLC25A37:FBXO7, SLC2A3:ADAM19, SLC2A3:MFSD10, SLC39A8:CCND2, SLC39A8:IL7R, SLC39A8:LFNG, SLC39A8:WDR33, SNAPC1:IL7R, SORT1:CNBP, SORT1:INPP1, SORT1:NAGK, SORT1:OSBPL9, SORT1:PDCD5, SORT1:PPP1R11, SORT1:SASH3, SORT1:TINF2, ST3GAL6:KLRF1, TAX1BP1:NUPL2, TIMP1:EMP3, TIMP1:IL7R, TLR5:CPVL, TLR5:CTSH, TLR5:DIAPH2, TLR5:ENO1, TLR5:FGL2, TLR5:FTL, TLR5:FUT8, TLR5:GBP2, TLR5:HIST1H2BM, TLR5:HIST1H3C, TLR5:HIST1H4L, TLR5:HLA-DPA1, TLR5:IFIT1, TLR5:IFNGR2, TLR5:ITGB1, TLR5:MX1, TLR5:NMI, TLR5:PLAC8, TLR5:RDX, TLR5:SDHC, TLR5:SLAMF7, TLR5:ST3GAL5, TLR5:TMEM106C, TLR5:TPP2, TLR5:VAV1, TMEM106C:IL7R, TMEM80:IMP3, TPP2:IL7R, USP3:RNF34, VAV1:IL7R, YPEL5:ARL2BP and ZBTB17:ID3.

9. The method of any one of claims 1 to 8, wherein the at least two RO BaSIRS biomarkers are quantified using polymerase chain reaction, preferably wherein the polymerase chain reaction is a real-time polymerase chain reaction.

10. The method of any one of claims 1 to 9, wherein a respective biomarker value is in the form of an amplification amount, preferably wherein the amplification amount is a cycle time, a number of cycles, a cycle threshold or an amplification time.

11. An apparatus for determining an indicator used in assessing a likelihood of a subject having an absence of bacterial associated systemic inflammatory response syndrome (BaSIRS), the apparatus comprising at least one electronic processing device that:

a) determines for a pair of RO BaSIRS biomarkers a pair of biomarker values, each biomarker value being a value measured or derived for at least one corresponding RO BaSIRS biomarker in a sample taken from the subject and being at least partially indicative of a concentration of the RO BaSIRS biomarker in the sample, wherein the RO BaSIRS biomarkers are expression products of GBP2 and a gene selected from the group consisting of: GIMAP4, HCP5, MX1 and TLR5;
b) determines a derived biomarker value using the pair of biomarker values, the derived biomarker value being indicative of a ratio of concentrations of the pair of RO BaSIRS biomarkers; and
c) determines the indicator using the derived biomarker value.

12. The apparatus of claim 11, wherein the RO BaSIRS biomarkers are expression products of GBP2 and GIMAP4 or wherein the RO BaSIRS biomarkers are expression products of GBP2 and TLR5; and wherein the at least one electronic processing device uses pairs of RO BaSIRS biomarkers to determine the indicator.

13. The apparatus of claim 12, wherein the at least one electronic processing device (a) determines a first derived biomarker value using a first pair of biomarker values, the first derived biomarker value being indicative of a ratio of concentrations of first and second RO BaSIRS biomarkers; (b) determines a second derived biomarker value using a second pair of biomarker values, the second derived biomarker value being indicative of a ratio of concentrations of third and fourth RO BaSIRS biomarkers; (c) determines a third derived biomarker value using a third pair of biomarker values, the third derived biomarker value being indicative of a ratio of concentrations of fifth and sixth RO BaSIRS biomarkers; and (d) determines the indicator by combining the first, second and third derived biomarker values.

14. The apparatus of claim 13, wherein the at least one electronic processing device combines the biomarker values using a combining function, preferably wherein the combining function is at least one of: an additive model; a linear model; a support vector machine; a neural network model; a random forest model; a regression model; a genetic algorithm; an annealing algorithm; a weighted sum; a nearest neighbor model; and a probabilistic model.

15. The apparatus of any one of claims 13 to 14, wherein the pairs of biomarkers are expression products of gene pairs selected from the group consisting of: AIF1:HMGN4, ALDOA:MAP4K2, ATG9A:RAB11FIP3, ATP13A3:IL7R, ATP6V0A1:RASSF7, ATP8B4:CCND2, CD44:GIMAP4, CD44:HLA-DPA1, CD44:IL7R, CD44:RPA2, CD59:GIMAP4, CDC14A:CCND2, CDC14A:IL7R, CHPT1:FBXO7, CHPT1:RANBP10, CLEC4E:MX1, CLEC4E:SYNE2, CLU:CCND2, CLU:IL7R, COQ10B:TRAF3IP2, COX5B:PHB, CPVL:IL7R, CTSA:DLST, CTSA:HMG20B, CTSC:CCND2, CTSH:IL7R, CYBB:BRD7, DERA:HMGN4, DIAPH2:CCND2, DIAPH2:HLA-DPA1, DIAPH2:IL7R, DIAPH2:PHF3, DIAPH2:RAB9A, DIAPH2:RNASE6, DIAPH2:SERTAD2, DIAPH2:ST3GAL5, DIAPH2:STK38, EIF4E2:C21orf59, EMR1:AKT1, ENO1:IL7R, FCER1G:CD44, FCER1G:CDC14A, FCER1G:MX1, FCER1G:SDHC, FLVCR2:KATNA1, FTL:CCND2, FTL:IL7R, FURIN:ADD1, FURIN:BTG2, FURIN:RANBP10, FURIN:SH3GLB2, FUT8:IL7R, FXR1:EIF4A2, GAPDH:COMMD4, GAPDH:PPP1CA, GAPDH:RPS6KB2, GAS7:ADD1, GAS7:RAB11FIP1, GBP2:GIMAP4, GBP2:HCP5, GBP2:MX1, GNS:PLEKHG3, GSTO1:RASGRP3, GSTO1:SDF2L1, HEBP1:SSBP2, HIST1H2BM:CCND2, HIST1H2BM:IL7R, HIST1H3C:IL7R, HOXB6:PAFAH2, HSPA4:IMP3, IFNGR2:CCND2, IFNGR2:HLA-DPA1, IFNGR2:IL7R, IMPDH1:BTG2, ITGAX:RASGRP2, ITGB1:IL7R, LAMP1:HLA-DPA1, LAMP1:IL7R, LHFPL2:ISG20, LILRB3:IL7R, LTA4H:CCND2, LTA4H:CERK, LTA4H:CPVL, LTA4H:RPS8, LTA4H:ST3GAL5, LTA4H:TMEM106C, LTA4H:WDR33, LTF:MAP4K2, MAPK14:GIMAP4, MAPK14:IL7R, MAPK14:MX1, MAPK8IP3:IMP3, MCTP1:AMD1, MCTP1:TOB1, MEGF9:CCND2, MEGF9:CDC14A, MEGF9:GIMAP4, MEGF9:HLA-DPA1, MEGF9:IL7R, METTL9:AKTIP, MICAL1:DHX16, MICAL1:STK38, MMP8:CCND2, MMP8:CD44, MMP8:CTSC, MMP8:ENO1, MMP8:FGL2, MMP8:FTL, MMP8:FUT8, MMP8:IL7R, MMP8:ITGB1, MMP8:LAMP1, MMP8:PLAC8, MMP8:RPS8, MMP8:SDHC, MMP8:ST3GAL5, MMP8:TMEM106C, MMP8:TPP2, MMP8:VAV1, MNT:KLF2, MNT:SLC9A3R1, MUT:NUPL2, MYL9:GRAP2, MYL9:KLF2, NMI:MX1, OBFC1:C6orf48, PARL:PAFAH2, PDGFC:CCND2, PDGFC:FUT8, PDGFC:IL7R, PDGFC:ITGB1, PLEKHM2:SBF1, PPP1CB:PAFAH2, PROS1:MYOM2, PROS1:WDR33, PRPF40A:MRPS18B, PRRG4:GLOD4, PSMB4:IMP3, PSTPIP2:AKAP7, PTPN2:CYP20A1, PUS3:PAFAH2, S100A12:POLR2C, S100P:GIMAP4, S100P:HLA-DPA1, S100P:IL7R, SH3BGRL:GLOD4, SLC11A2:ID3, SLC12A9:CTDSP2, SLC25A37:FBXO7, SLC2A3:ADAM19, SLC2A3:MFSD10, SLC39A8:CCND2, SLC39A8:IL7R, SLC39A8:LFNG, SLC39A8:WDR33, SNAPC1:IL7R, SORT1:CNBP, SORT1:INPP1, SORT1:NAGK, SORT1:OSBPL9, SORT1:PDCD5, SORT1:PPP1R11, SORT1:SASH3, SORT1:TINF2, ST3GAL6:KLRF1, TAX1BP1:NUPL2, TIMP1:EMP3, TIMP1:IL7R, TLR5:CPVL, TLR5:CTSH, TLR5:DIAPH2, TLR5:ENO1, TLR5:FGL2, TLR5:FTL, TLR5:FUT8, TLR5:GBP2, TLR5:HIST1H2BM, TLR5:HIST1H3C, TLR5:HIST1H4L, TLR5:HLA-DPA1, TLR5:IFIT1, TLR5:IFNGR2, TLR5:ITGB1, TLR5:MX1, TLR5:NMI, TLR5:PLAC8, TLR5:RDX, TLR5:SDHC, TLR5:SLAMF7, TLR5:ST3GAL5, TLR5:TMEM106C, TLR5:TPP2, TLR5:VAV1, TMEM106C:IL7R, TMEM80:IMP3, TPP2:IL7R, USP3:RNF34, VAV1:IL7R, YPEL5:ARL2BP and ZBTB17:ID3.

**FIGURE 1a**

**Final Signature**

FIGURE 1b

Triage signature performance across datasets

FIGURE 2

FIGURE 3

FIGURE 4

FIGURE 5a

FIGURE 5b

FIGURE 5c

**PATIENT REPORT**

✕ Immune

SeptiCyte® Triage

| Patient Name | Medical Report # | Blood Collection Date | Sample ID |
|---|---|---|---|
| Jane Doe | 1000000 | 28-Jul-2014 | IXP100000 |

**5.9**$
PATIENT'
SCORE'

0'          4.5'      6'        10'

Band 3$

SEPTICYTE® SCORE RANGE'

**PROBABILITY OF SEPSIS:**

**> 80%**

73 – 86% (80% Confidence Interval)

| Score Interpretation Band | | Prevalence SIRS | Prevalence Sepsis | Sepsis Likelihood Ratio |
|---|---|---|---|---|
| **Band 1** (Patient Score 0 – 3.1) | Sepsis is <10% likely | 92% | 8% | 0.05 |
| **Band 2** (Patient Score 3 .1 – 4.5) | Sepsis is >40% likely | 56% | 44% | 0.46 |
| **Band 3** (Patient Score 4.5 – 6.0) | Sepsis is >80% likely | 15% | 85% | 6 |
| **Band 4** (Patient Score 6 .0 – 10) | Sepsis is >90% likely | 6% | 94% | 9 |

**TEST DESCRIPTION:**
The SeptiCyte® Triage test is an aid in excluding a diagnosis of sepsis in patients with SIRS presenting to emergency departments. Increasing values of the SeptiCyte® Score is associated with an increased likelihood that systemic inflammation is attributable to infection (i.e. sepsis) relative to other causes. The SeptiCyte® Score is a quantitative blood test that combines the results of six RNA transcripts (DIAPH2, SERTAD2, PARL, PAFAH2, SORT1, OSBPL9) as a single numerical Patient Score.

**TEST RESULT INFORMATION:**
The SeptiCyte® Triage test should only be used in patients with clinical signs of SIRS. Individual patient values should be interpreted only in the context of all other clinical and laboratory information; The SeptiCyte® Score should not be used as the sole basis for excluding sepsis in a patient with clinical signs of SIRS.

**LIMITATIONS:**
The reported probability of sepsis for the The SeptiCyte® Triage test were determined in a sample of XX European and US patients aged X – X assessed for SIRS or sepsis with a background prevalence of sepsis of XX%. Patients were classified by retrospective expert physician assessment after admission to a hospital.

ACTUAL PATIENT SCORE IN RELATION TO HISTORICAL DATA

Probability of Sepsis

0    3,1# 4,5# 6                    10#

| BAND 1 | 2 | 3 | 4 |

SeptiCyte® Score

**PHYSICIAN COMMENTS:**

✕ Immune

425 PONTIUS AVE N, SUITE 430
SEATTLE, WA 98104
P: 206.273.7975
www.immunexpress.com

# FIGURE 6

Europäisches
Patentamt

European
Patent Office

Office européen
des brevets

# EUROPEAN SEARCH REPORT

Application Number

EP 21 20 0635

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/203534 A1 (HOSSAIN HAMID [DE] ET AL) 13 August 2009 (2009-08-13)<br>* abstract; claim 23; table 1; sequences 2346, 5128, 1472, 4126, 4345 *<br>* page 6 - page 53 * | 1-15 | INV.<br>C12Q1/68<br>C12Q1/6883<br>G01N33/68 |
| A | LEO MCHUGH ET AL: "A Molecular Host Response Assay to Discriminate Between Sepsis and Infection- Negative Systemic Inflammation in Critically Ill Patients: Discovery and Validation in Independent Cohorts",<br>PLOS MEDICINE,<br>vol. 12, no. 12,<br>8 December 2015 (2015-12-08), page e1001916, XP055336105,<br>DOI: 10.1371/journal.pmed.1001916<br>* abstract; table 3 *<br>* page 2, paragraph top *<br>* page 25, last paragraph * | 1-15 | |
| A | JIE MA ET AL: "Lysosome and Cytoskeleton Pathways Are Robustly Enriched in the Blood of Septic Patients: A Meta-Analysis of Transcriptomic Data",<br>MEDIATORS OF INFLAMMATION.,<br>vol. 2015, 1 January 2015 (2015-01-01), pages 1-15, XP055395638,<br>GB<br>ISSN: 0962-9351, DOI: 10.1155/2015/984825<br>* abstract; table 3 *<br>* page 8 * | 1-15 | **TECHNICAL FIELDS SEARCHED (IPC)**<br><br>C12Q |

The present search report has been drawn up for all claims

| Place of search | Date of completion of the search | Examiner |
|---|---|---|
| Munich | 24 March 2022 | Celler, Jakub |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 0635

This annex lists the patent family members relating to the patent documents cited in  the above-mentioned European search report.
The members are as contained in the European Patent Office EDP file on
The European Patent Office is in no way liable for these particulars which are merely given for the purpose of information.

24-03-2022

| Patent document cited in search report | | Publication date | Patent family member(s) | | Publication date |
|---|---|---|---|---|---|
| US 2009203534 | A1 | 13-08-2009 | DE 102005050933 | A1 | 26-04-2007 |
| | | | EP 2004846 | A2 | 24-12-2008 |
| | | | US 2009203534 | A1 | 13-08-2009 |
| | | | WO 2007045197 | A2 | 26-04-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- AU 2015905392
- WO 2015117204 A
- US 4683195 A
- US 4683202 A
- US 4800159 A
- WO 9007641 A
- US 20070190540
- US 5143854 A, Pirrung
- US 5925525 A, Fodor

- US 20020055186
- US 20030003599
- WO 03062444 A
- WO 03077851 A
- WO 0259601 A
- WO 0239120 A
- WO 0179849 A
- WO 9939210 A
- AU 2014900363

### Non-patent literature cited in the description

- **NISKA, R. ; BHUIYA, F. ; XU, J.** National hospital ambulatory medical care survey: 2007 emergency department summary. *Natl Health Stat Report,* 2010, vol. 26 (26), 1-31
- Definitions for sepsis and organ failure and guidelines for the use of innovative therapies in sepsis. **BONE, R. ; BALK, R. ; CERRA, F. ; DELLINGER, R. ; FEIN, A. ; KNAUS, W. et al.** Chest. The ACCP/SCCM Consensus Conference Committee. American College of Chest Physicians/Society of Critical Care Medicine, 1992, vol. 101, 1644-1655
- **BROWN, T. ; GHELANI-ALLEN, A. ; YEUNG, D. ; NGUYEN, H. B.** Comparative effectiveness of physician diagnosis and guideline definitions in identifying sepsis patients in the emergency department. *Journal of Critical Care,* 2014
- **GLICKMAN, S. W. ; CAIRNS, C. B. ; OTERO, R. M. ; WOODS, C. W. ; TSALIK, E. L. ; LANGLEY, R. J. et al.** Disease Progression in Hemodynamically Stable Patients Presenting to the Emergency Department With Sepsis. *Academic Emergency Medicine,* 2010, vol. 17 (4), 383-390
- **DELLINGER, R. P. et al.** Surviving Sepsis Campaign: international guidelines for management of severe sepsis and septic shock: 2008. *Crit. Care Med.,* 2008, vol. 36, 296-327
- **BRAYKOV, N. P. ; MORGAN, D. J. ; SCHWEIZER, M. L. ; USLAN, D. Z. ; KELESIDIS, T. ; WEISENBERG, S. A. et al.** Assessment of empirical antibiotic therapy optimisation in six hospitals: an observational cohort study. *The Lancet Infectious Diseases,* 2014, vol. 14 (12), 1220-1227

- **LALKHEN, A. G. ; MCCLUSKEY, A.** Clinical tests: sensitivity and specificity. *Continuing Education in Anaesthesia, Critical Care & Pain,* 2008, vol. 8 (6), 221-223
- **REZENDE, E. ; SILVA JUNIOR, J. M. ; ISOLA, A. M. ; CAMPOS, E. V. ; AMENDOLA, C. P. ; ALMEIDA, S. L.** Epidemiology of severe sepsis in the emergency department and difficulties in the initial assistance. *Clinics,* 2008, vol. 63 (4), 457-464
- **SINGHAL, S. ; ALLEN, M. W. ; MCANNALLY, J.-R. ; SMITH, K. S. ; DONNELLY, J. P. ; WANG, H. E.** National estimates of emergency department visits for pediatric severe sepsis in the United States. *PeerJ,* 2013, vol. 1 (1), e79-12
- **FISCHER, J. E. et al.** Quantifying uncertainty: physicians' estimates of infection in critically ill neonates and children. *Clin. Infect. Dis.,* 2004, vol. 38, 1383-1390
- **THIERRY CALANDRA ; JONATHAN COHEN.** The International Sepsis Forum Consensus Conference on Definitions of Infection in the Intensive Care Unit. *Critical Care Medicine,* July 2005, vol. 33 (7), 1538-1548
- **R PHILLIP DELLINGER et al.** *Surviving Sepsis Campaign: International Guidelines for Management of Severe Sepsis and Septic Shock: 2008.,* 2008, vol. 36, 296-327
- **MÜLLER, B. ; SCHUETZ, P. ; TRAMPUZ, A.** Circulating biomarkers as surrogates for bloodstream infections. *International Journal of Antimicrobial Agents,* 2007, vol. 30, 16-23
- **JEAN-LOUIS VINCENT et al.** Sepsis in European Intensive Care Units: Results of the SOAP Study. *Critical Care Medicine,* February 2006, vol. 34 (2), 344-353

- **BRIGITTE LAMY et al.** What Is the Relevance of Obtaining Multiple Blood Samples for Culture? A Comprehensive Model to Optimize the Strategy for Diagnosing Bacteremia. *Clinical Infectious Diseases: an Official Publication of the Infectious Diseases Society of America,* 01 October 2002, vol. 35 (7), 842-850
- **M D ARONSON ; D H BOR.** Blood Cultures. *Annals of Internal Medicine,* February 1987, vol. 106 (2), 246-253
- **BATES, D. W. ; GOLDMAN, L. ; LEE, T. H.** Contaminant blood cultures and resource utilization. The true consequences of false-positive results. *JAMA,* 1991, vol. 265, 365-369
- **BAUER M ; REINHART K.** Molecular diagnostics of sepsis - Where are we today?. *International Journal of Medical Microbiology,* 2010, vol. 300, 411-413
- **LJUNGSTRÖM, L. ; ENROTH, H. ; CLAESSON, B. E. ; OVEMYR, I. ; KARLSSON, J. ; FRÖBERG, B. et al.** Clinical evaluation of commercial nucleic acid amplification tests in patients with suspected sepsis. *BMC Infectious Diseases,* 2015, vol. 15 (1), 199
- **AVOLIO, M. ; DIAMANTE, P. ; MODOLO, M. L. ; DE ROSA, R. ; STANO, P. ; CAMPORESE, A.** Direct Molecular Detection of Pathogens in Blood as Specific Rule-In Diagnostic Biomarker in Patients With Presumed Sepsis. *Shock,* 2014, vol. 42 (2), 86-92
- **MICHAEL BAUER ; KONRAD REINHART.** Molecular Diagnostics of Sepsis - Where Are We Today?. *International Journal of Medical Microbiology,* 01 August 2010, vol. 300 (6), 411-413
- **JOHN C MARSHALL ; KONRAD REINHART.** Biomarkers of Sepsis. *Critical Care Medicine,* July 2009, vol. 37 (7), 2290-2298
- **REINHART, K. ; BAUER, M. ; RIEDEMANN, N. C. ; HARTOG, C. S.** New Approaches to Sepsis: Molecular Diagnostics and Biomarkers. *Clinical Microbiology Reviews,* 2012, vol. 25, 609-634
- **WACKER, C. ; PRKNO, A. ; BRUNKHORST, F. M. ; SCHLATTMANN, P.** Procalcitonin as a diagnostic marker for sepsis: a systematic review and meta-analysis. *The Lancet Infectious Diseases,* 2013, vol. 13 (5), 426-435
- **HOENIGL, M. ; RAGGAM, R. B. ; WAGNER, J. ; PRUELLER, F. ; GRISOLD, A. J. ; LEITNER, E. et al.** Procalcitonin fails to predict bacteremia in SIRS patients: a cohort study. *International Journal of Clinical Practice,* 2014, vol. 68 (10), 1278-1281
- **GIBOT, S. ; BENE, M. C. ; NOEL, R. ; MASSIN, F. ; GUY, J. ; CRAVOISY, A. et al.** Combination biomarkers to diagnose sepsis in the critically ill patient. *American Journal of Respiratory and Critical Care Medicine,* 2012, vol. 186 (1), 65-71
- **FRIEDERICHS, J. ; HUTTER, M. ; HIERHOLZER, C. ; NOVOTNY, A. ; FRIESS, H. ; BUHREN, V. ; HUNGERER, S.** Procalcitonin ratio as a predictor of successful surgical treatment of severe necrotizing soft tissue infections. *American Journal of Surgery,* 2013, vol. 206 (3), 368-373
- **GILLE-JOHNSON, P. ; HANSSON, K. E. ; GARDLUND, B.** Severe sepsis and systemic inflammatory response syndrome in emergency department patients with suspected severe infection. *Scandinavian Journal of Infectious Diseases,* 2013, vol. 45 (3), 186-193
- **CRAIG, J. C. ; WILLIAMS, G. J. ; JONES, M. ; CODARINI, M. ; MACASKILL, P. ; HAYEN, A. et al.** The accuracy of clinical symptoms and signs for the diagnosis of serious bacterial infection in young febrile children: prospective cohort study of 15,781 febrile illnesses. *BMJ (Clinical Research Ed.),* 2010, vol. 340, c1594
- **NEEDLEMAN ; WÜNSCH.** *J. Mol. Biol.,* 1970, vol. 48, 444-453
- **E. MEYERS ; W. MILLER.** *Cabios,* 1989, vol. 4, 11-17
- **ALTSCHUL et al.** *J Mol Biol.,* 1990, vol. 215, 403-10
- **ALTSCHUL et al.** *Nucleic Acids Res,* 1997, vol. 25, 3389-3402
- **AUSUBEL.** CURRENT PROTOCOLS IN MOLECULAR BIOLOGY. John Wiley & Sons Inc, 1994-1998
- **SAMBROOK et al.** MOLECULAR CLONING. A LABORATORY MANUAL. Cold Spring Harbor Press, 1989
- **INNIS et al.** PCR Protocols. Academic Press, Inc, 1990
- **HIGUCHI et al.** *Biotechnology,* 1992, vol. 10, 413-417
- **HACIA et al.** *Nature Genetics,* 1996, vol. 14, 441-447
- **SHOEMAKER et al.** *Nature Genetics,* 1996, vol. 14, 450-456
- **PEASE et al.** *Proc. Natl. Acad. Sci. U.S.A.,* 1994, vol. 91, 5022-5026
- **FODOR et al.** *Science,* 1991, vol. 251, 767-773
- NUCLEIC ACID HYBRIDIZATION, A PRACTICAL APPROACH. IRL press, 1985
- **HARRIS.** *Science,* 2008, vol. 320, 106-109
- **MARGULIES et al.** *Nature,* 2005, vol. 437, 376-380
- **SONI ; MELLER.** *Clin Chem,* 2007, vol. 53, 1996-2001
- **GE.** *Nucleic Acids Res,* 2000, vol. 28 (2), e3
- **LOPEZ et al.** *J. Chromatogram. B,* 2003, vol. 787, 19-27
- **CAHILL.** *Trends in Biotechnology,* 2000, vol. 7, 47-51

- **HEALY.** *Ann. Pharmacother.,* 2002, vol. 36 (4), 648-54
- **BRINDLEY.** *CJEM,* 2005, vol. 7 (4), 227
- **JENKINS.** *J Hosp Med,* 2006, vol. 1 (5), 285-295
- **XIAO, W. ; MINDRINOS, M. N. ; SEOK, J. ; CUSCHIERI, J. ; CUENCA, A. G. ; GAO, H. et al.** A genomic storm in critically injured humans. *Journal of Experimental Medicine,* 2011, vol. 208 (13), 2581-2590
- **NISKA R ; BHUIYA F ; XU J.** National hospital ambulatory medical care survey: 2007 emergency department summary. *Natl Health Stat Report,* 2010, vol. 26, 1-31
- **VAN LAAR, P. J. ; COHEN, J.** A prospective study of fever in the accident and emergency department. *Clinical Microbiology and Infection : the Official Publication of the European Society of Clinical Microbiology and Infectious Diseases,* 2003, vol. 9 (8), 878-880
- **MANNING, L. V. ; TOUQUET, R.** The relevance of pyrexia in adults as a presenting symptom in the accident and emergency department. *Archives of Emergency Medicine,* 1988, vol. 5 (2), 86-90
- **LIMPER M ; EEFTINCK SCHATTENKERK D ; DE KRUIF MD ; VAN WISSEN M ; BRANDJES DPM et al.** One-year epidemiology of fever at the Emergency Department. *Neth J Med,* 2011, vol. 69, 124-128
- **DE NAUROIS, J. ; NOVITZKY-BASSO, I. ; GILL, M. J. ; MARTI, F. M. ; CULLEN, M. H. ; ROILA, F.** On behalf of the ESMO Guidelines Working Group. *Management of febrile neutropenia: ESMO Clinical Practice Guidelines. Annals of Oncology,* 2010, vol. 21 (5), v252-v256
- **KASISKE, B. L. ; VAZQUEZ, M. A. ; HARMON, W. E. ; BROWN, R. S. ; DANOVITCH, G. M. ; GASTON, R. S. et al.** Recommendations for the outpatient surveillance of renal transplant recipients. American Society of Transplantation. *Journal of the American Society of Nephrology,* October 2000, vol. 11, S1-S86
- **FISCHER, J. E. ; HARBARTH, S. ; AGTHE, A. G. ; BENN, A. ; RINGER, S. A. ; GOLDMANN, D. A. ; FANCONI, S.** Quantifying uncertainty: physicians' estimates of infection in critically ill neonates and children. *Clinical Infectious Diseases : an Official Publication of the Infectious Diseases Society of America,* 2004, vol. 38 (10), 1383-1390